# EUROPEAN PATENT APPLICATION

(11) **EP 4 234 582 A1**
(43) Date of publication of application: **30.08.2023**
(21) Application number: 22382174.5
(22) Date of filing: 28.02.2022
(51) Int. Cl.: C07K 16/30, A61K 39/00, A61P 35/02, C07K 14/705, C07K 14/725

(54) **HUMANIZED CD1A TARGETING MOIETY FOR THE TREATMENT OF CD1A-POSITIVE CANCER**

(71) Applicant: Onechain Immunotherapeutics SL, 08021 Barcelona (ES); Fundació Institut de Recerca Contra la Leucèmia Josep Carreras, 08916 Barcelona (ES); Institució Catalana de Recerca I Estudis Avançats, 08010 Barcelona (ES); Fundació Institut d'Investigació en Ciències de la Salut Germans Trias i Pujol, 08916 Badalona, Barcelona (ES)
(72) Inventor: DÍAZ CORTÉS, Víctor Manuel, 08028 Barcelona (ES); MENÉNDEZ BUJÁN, Pablo, 08916 Badalona (ES); SÁNCHEZ MARTÍNEZ, Diego, 08916 Badalona (ES); GARCÍA PÉREZ, Laura, 08028 Barcelona (ES)
(74) Representative: Hoffmann Eitle

(57) **Abstract**

Relapsed/refractory T-cell acute lymphoblastic leukemia (T-ALL) has a dismal outcome, and no effective targeted immunotherapies for T-ALL exist. CD1a is exclusively expressed in cortical T-ALLs, a major subset of T-ALL. The expression of CD1a is restricted to cortical thymocytes and neither CD34+ progenitors nor T-cells express CD1a during ontogeny, confining the risk of on-target/off-tumor toxicity. The present invention provides CD1a-targeting moieties comprising a CD1a-which may be placed into T cells. The resultant CARTs are suitable for the treatment of cortical T-ALLs.

## Description

### Technical Field

The present invention provides therapeutics for the treatment of CD1a-positive cancers such as T-cell acute lymphoblastic leukemia. In particular, the present invention provides an humanized CD1a targeting moiety.

### Background art

T-cell lineage acute lymphoblastic leukemia (T-ALL) is a malignant disorder resulting from leukemic transformation of thymic T-cell precursors [1]. T-ALL is phenotypically and genetically heterogeneous, and is commonly associated with genetic alterations/mutations in transcription factors involved in hematopoietic stem/progenitor cell (HSPC) homeostasis and in master regulators of T-cell development [2]. T-ALL comprises 10-15% and 20-25% of all acute leukemias diagnosed in children and adults, respectively [3, 4] with a median diagnostic age of 9 years [5]. Intensive chemotherapy regimens have led to the improved survival of patients with T-ALL. However, the event-free (EFS) and overall (OS) survival remains <70%, and relapsed/refractory (R/R) T-ALL has a particularly poor outcome. There are currently no potential curative options beyond hematopoietic cell transplantation and conventional chemotherapy, which is linked to large trade-offs in toxicities [4, 6], reinforcing the need for novel targeted therapies.

Immunotherapy has generated unprecedented expectations in cancer treatment and relies on the immune system as a powerful weapon against cancer. In recent years, adoptive cellular immunotherapy based on chimeric antigen receptors (CARs) has shown great potential. CAR therapy redirects genetically modified T-cells to specifically recognize and eliminate specific antigen-expressing tumor cells in a major histocompatibility complex-independent fashion [7, 8]. The success of CAR T-cells (CARTs) re-directed against CD19 or CD22 is now indisputable for B-cell malignancies (mainly B-ALL) [9, 10]. But strategies targeting T-cell malignancies using CARTs remain challenging because of the shared expression of target antigens between CARTs and T-lineage tumoral cells. In this regard, CARTs against pan T-cell antigens have two major drawbacks: i) CARTs self-targeting/fratricide and, ii) T-cell aplasia, leading to life-threating immunodeficiency [11-13].

Recent elegant studies demonstrated that T-cells transduced with either CD7, CD3, CD5 or TCR CARs, the most expressed pan-T-cell antigens, efficiently eliminate T-ALL blasts *in vitro* and are able to control the disease *in vivo* [11-15]. Yet, approaches still far from the clinic, such as CRISPR/Cas9 genome editing or protein expression blockers, were required for disruption of the target antigen in T-cells prior to CAR transduction, to avoid extensive self-antigen driven fratricide [11-15].

Thus, there remains a need for a therapy that can successfully treat T-ALL. The present invention aims to provide a therapy for treating CD1a-positive T-ALL.

### Figures

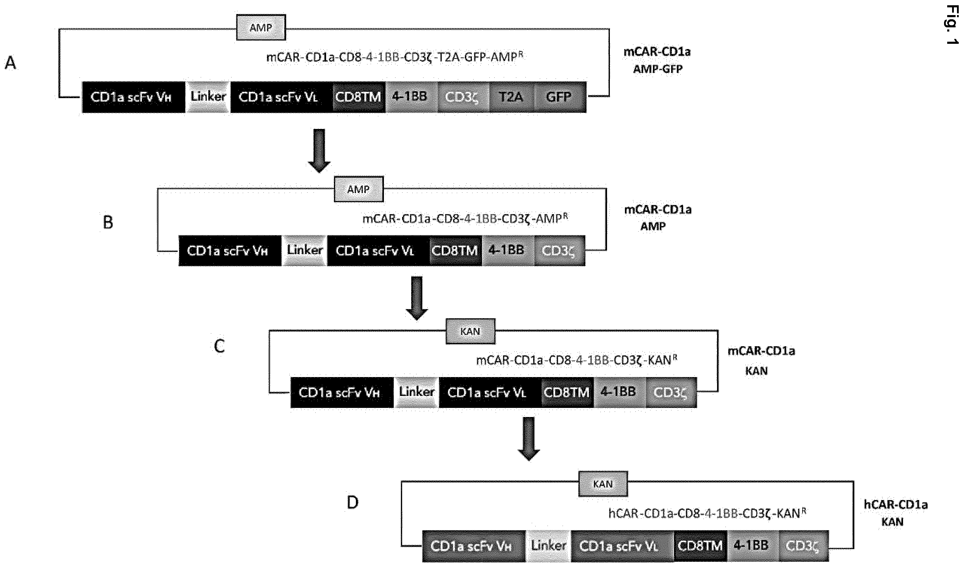

**Figure 1****.** Vector modifications from the murine to the humanized version of CAR-CD1a (mCAR-CD1a and hCAR-CD1a, respectively).

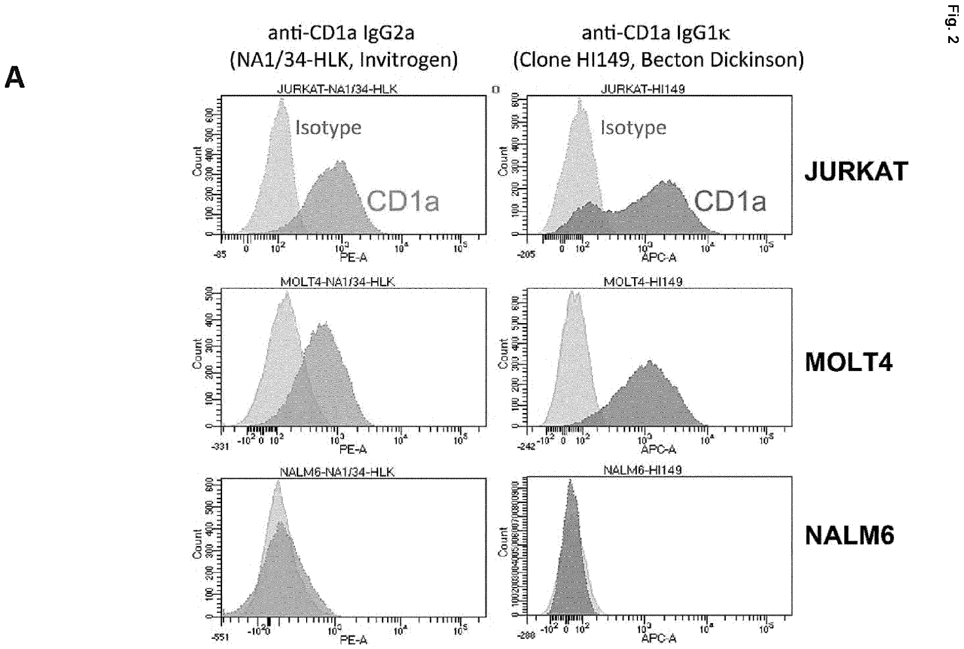

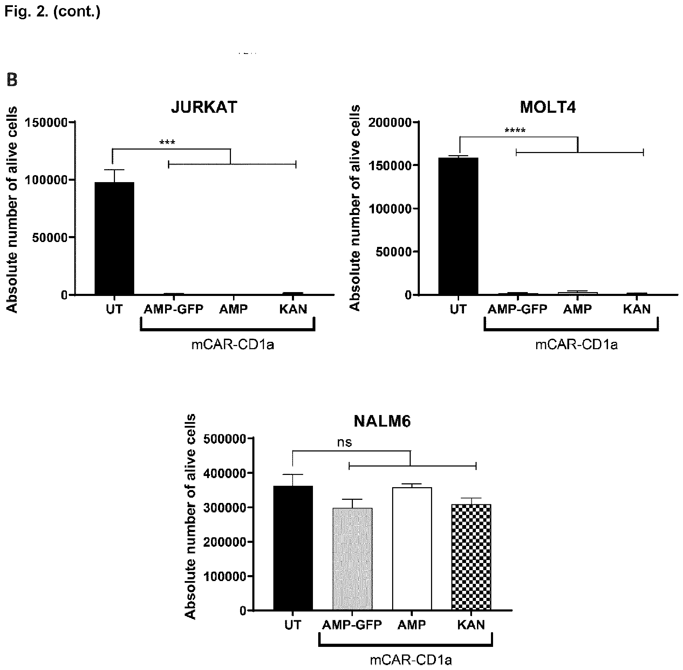

**Figure 2****.** (A) FACS histogram analysis of CD1a expression with two different antibodies with cells positive for CD1a (Jurkat and Molt4) compared to NALM6 (CD1a negative cells). Isotype, IgG control antibody. (B) Cytotoxicity of CD1a CARTs and UT T-cells against coT-ALL and B-ALL cell lines. Absolute counts of alive eFluor-positive target cells measured according to FACS in 72-hour cytotoxicity assays at a 1:1 E:T ratio. ***p<0.001; ****p<0,0001. T-test. One-tailed. AMP-GFP, mCAR-CD1a-AMP-GFP; AMP, mCAR-CD1a-AMP; KAN, mCAR-CD1a; n.s., not significant; UT, untransduced PBMCs.

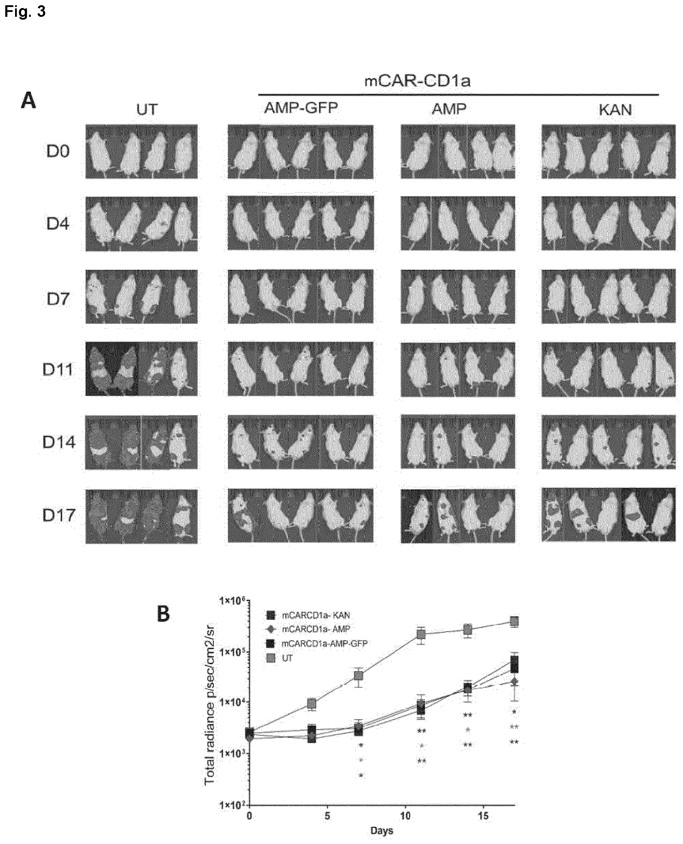

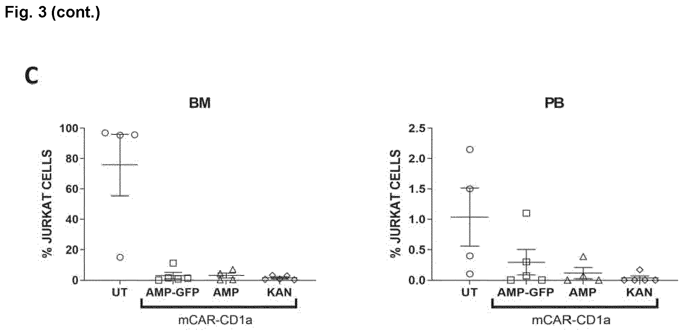

**Figure 3****.** Activity of CD1a CARTs *in vivo.* (A) IVIS imaging of tumor burden monitored by BLI at the indicated time days (D, days). (B) Total radiance quantification at the indicated time points shows all mCAR-CD1a versions efficiently abolish progression of coT-ALL cells in mice xenografts. (C) Circulating Jurkat in PB and in BM after animals were euthanized. *p<0.05, **p<0.01, ***p<0.001. T-test. One-tailed. AMP-GFP, mCAR-CD1a-AMP-GFP; AMP, mCAR-CD1a-AMP; KAN, mCAR-CD1a.

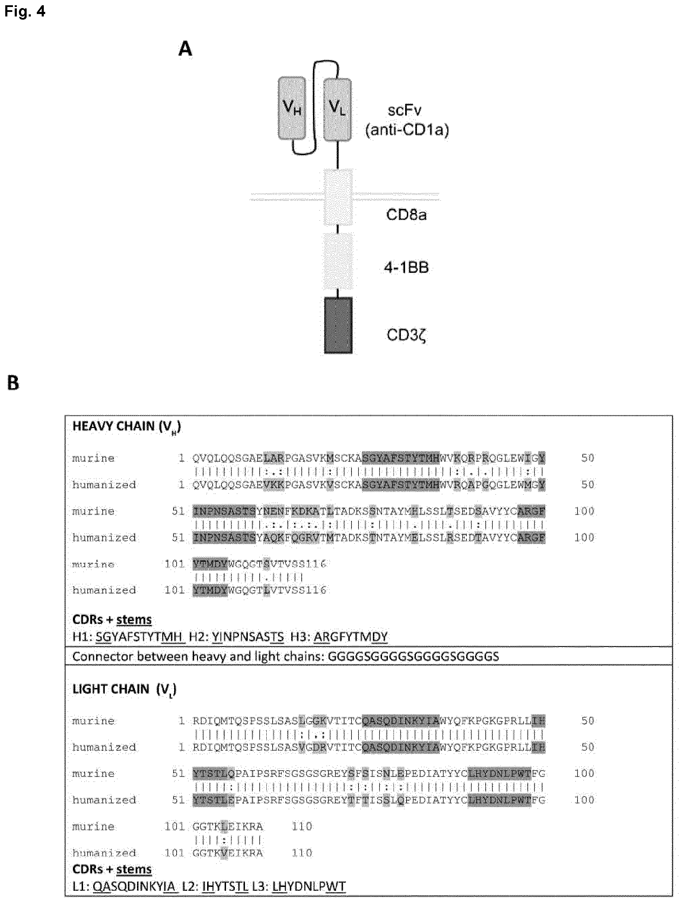

**Figure 4.** A) Structure of the chimeric antigen receptor (CAR) of OC_1. (B) Sequence alignments between murine and humanized (h2) scFvs for the heavy (VH) (upper) and light (VL) (lower) chains. Connector sequence between heavy and light chains is also shown (middle). CDRs and stems sequences are indicated. Changed aminoacids and individual CDR and stem sequences (underlined) have been highlighted.

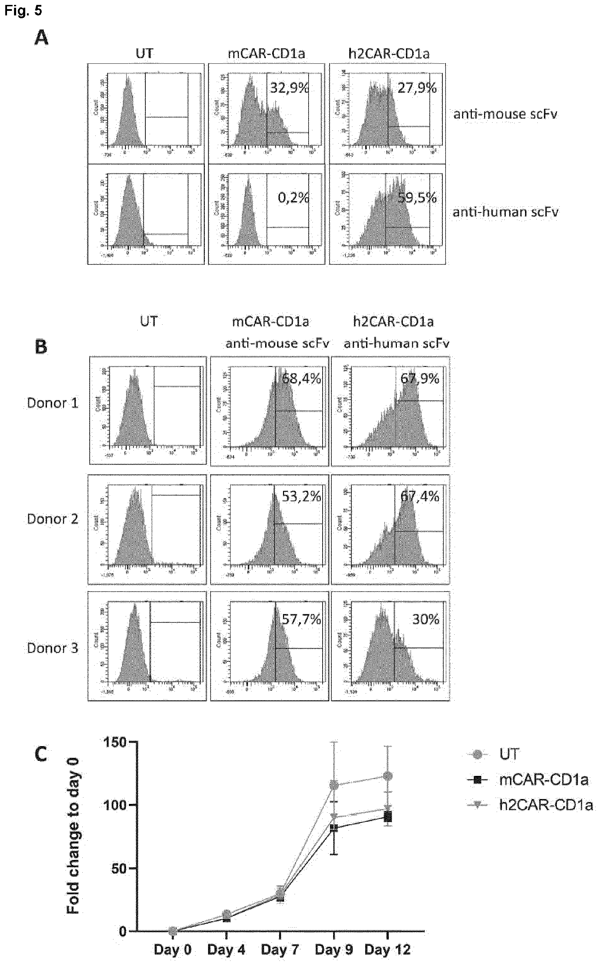

**Figure 5****.** FACS histogram analysis of transduced PBMCs with mCAR-CD1a and h2CAR-CD1a. A) Comparable detection with anti(a)-mouse and anti(a)-human scFvs from the same transduced donor; B) Transduction of 3 different donors with lentiviral particles encoding for mCAR-CD1a and h2CAR-CD1a. Percentages show membrane expression of scFvs respect to untransduced cells (UT); C) Representative experiment determining similar expansion of transduced PBMCs along time with the different CAR-CD1a and UT cells. UT, unstranduced PBMCs.

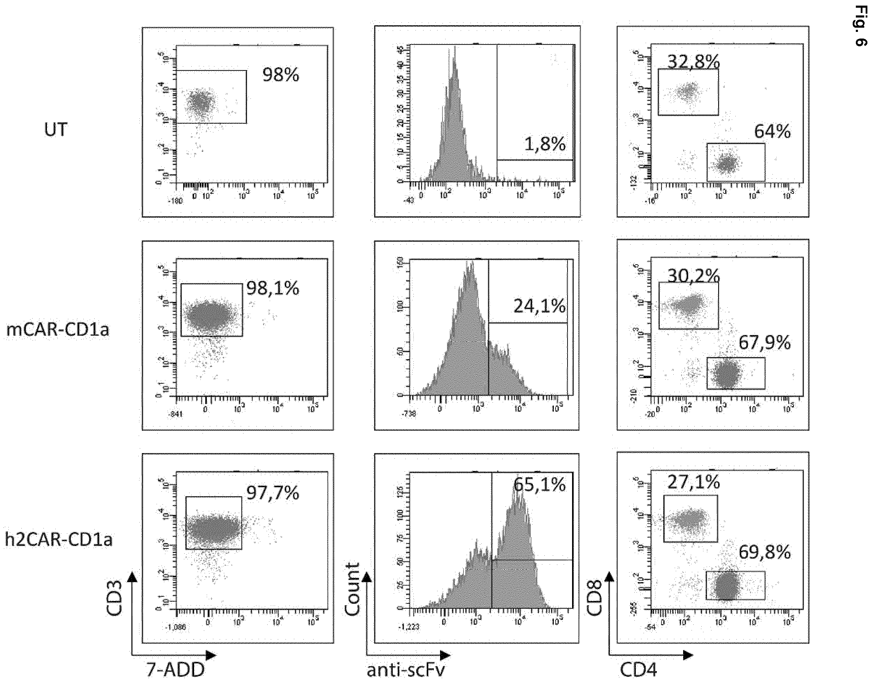

**Figure 6****.** FACS analysis of transduced PBMCs with mCAR-CD1a and h2CAR-CD1a. Percentages show membrane expression of scFvs, CD4+ and CD8+ cells. UT, untransduced PBMCs. 7-ADD, 7-Aminoactinomycin D, negative (live) cells are shown.

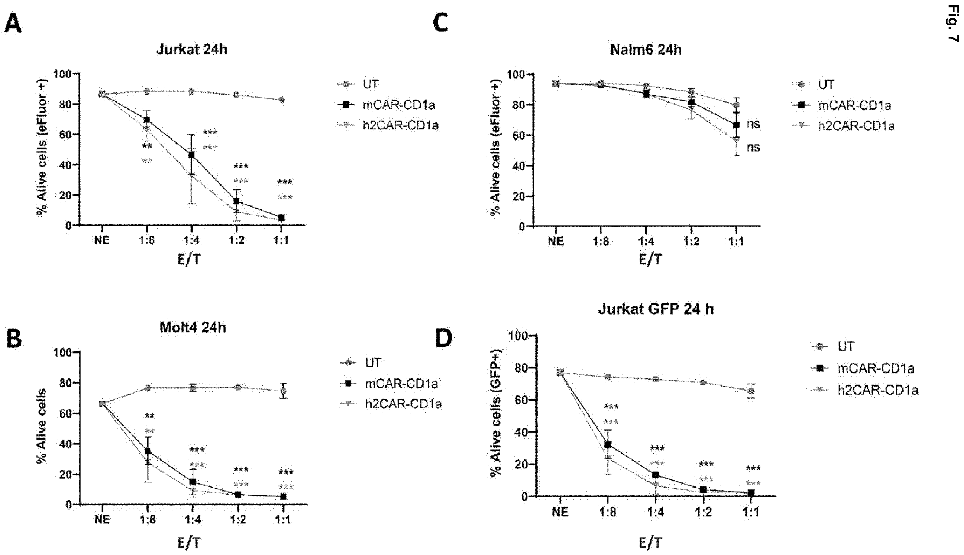

**Figure 7****.** Cytotoxicity assays with indicated cell lines at different effector/target ratios (E/T). Levels of viable cells were quantified by FACS analysis of eFluor positive cells (A, B and C) or GFP positive cells (D) at 24h of co-culture. **p<0.01, ***p<0.001 respect to UT. T-test. One-tailed. UT, untransduced PBMCs. NE, non-effector cells. ns, not significant.

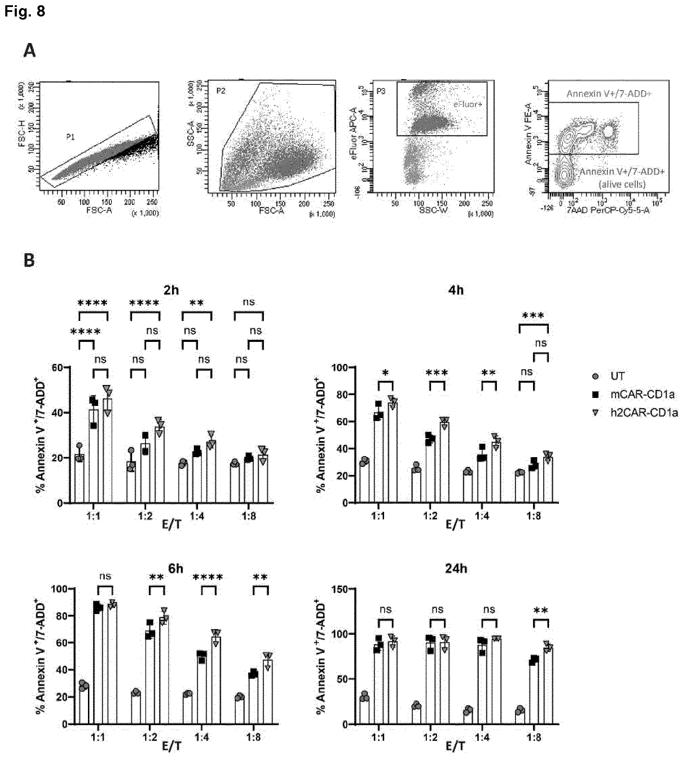

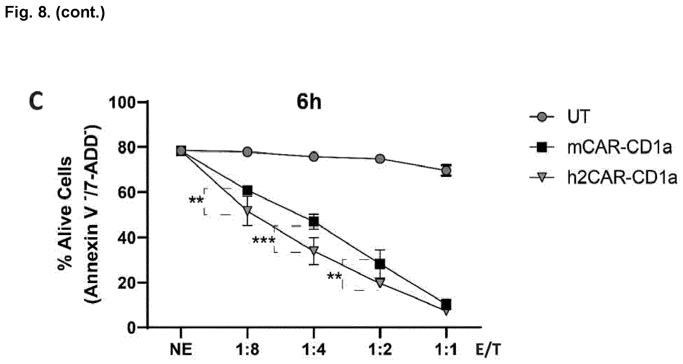

**Figure 8****.** Cytotoxicity assays with eFluor labelled Jurkat cells determined by the Annexin V method. Cell death was quantified by FACS analysis of Annexin V positive and 7-ADD positive cells at 2h, 4h, 6h and 24 h of co-culture with indicated CD1a-CAR-T or UT cells. A) Representative FACS showing the gating strategy (e-Fluor+/Annexin V+/7-ADD+). B) Quantification of cell death by using CAR-Ts from three different donors. UT, untransduced PBMCs. NE, non-effector cells. C) Levels of viable cells were quantified by FACS analysis of eFluor+/Anexin V-/7-ADD- cells, as indicated in panel A, at 6h of co-culture. ****p<0,0001; ***p<0,001; **p<0,01; ns, not significant. Two-way ANOVA test (Tukey correction for multiple comparisons).

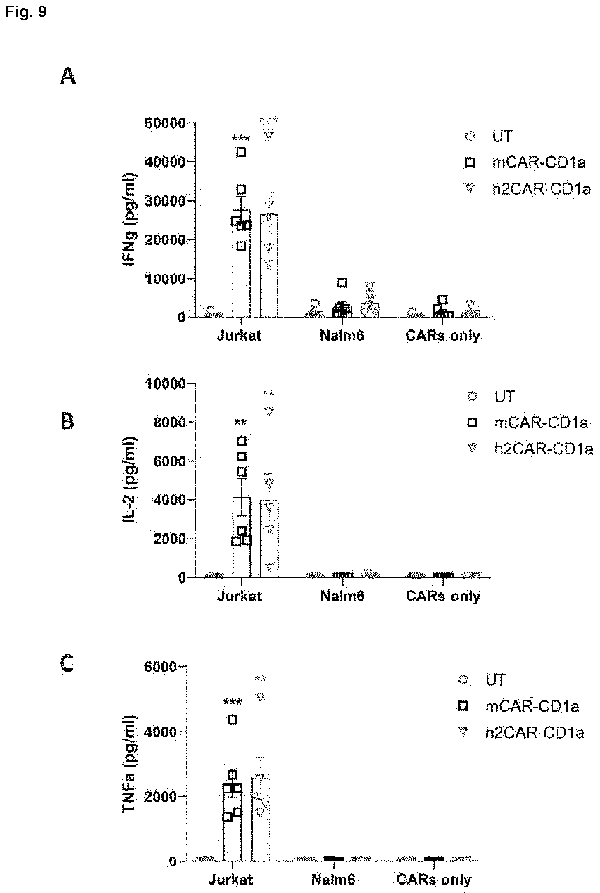

**Figure 9****.** Levels of pro-inflammatory cytokines determined by ELISA (A, B and C) in cells positive for CD1a (Jurkat) compared to NALM6 (CD1a negative cells) co-cultured for 24h with indicated CD1a-CAR-T or UT cells. Data were pooled from two independent experiments with 3 different donors **p<0.01, ***p<0.001, respect to UT cells. T-test. One-tailed. ELISA, enzyme-linked immunosorbent assay; UT, untransduced PBMCs.

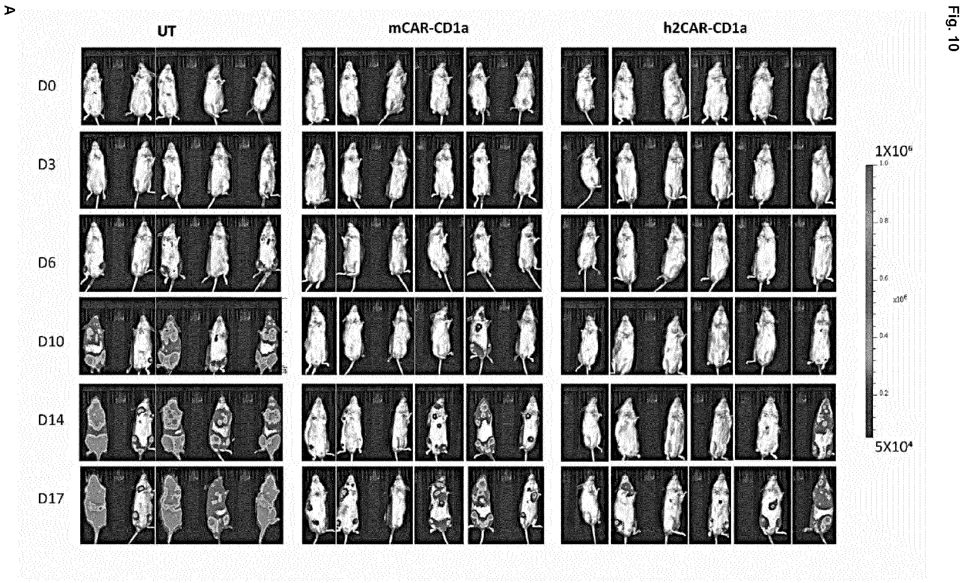

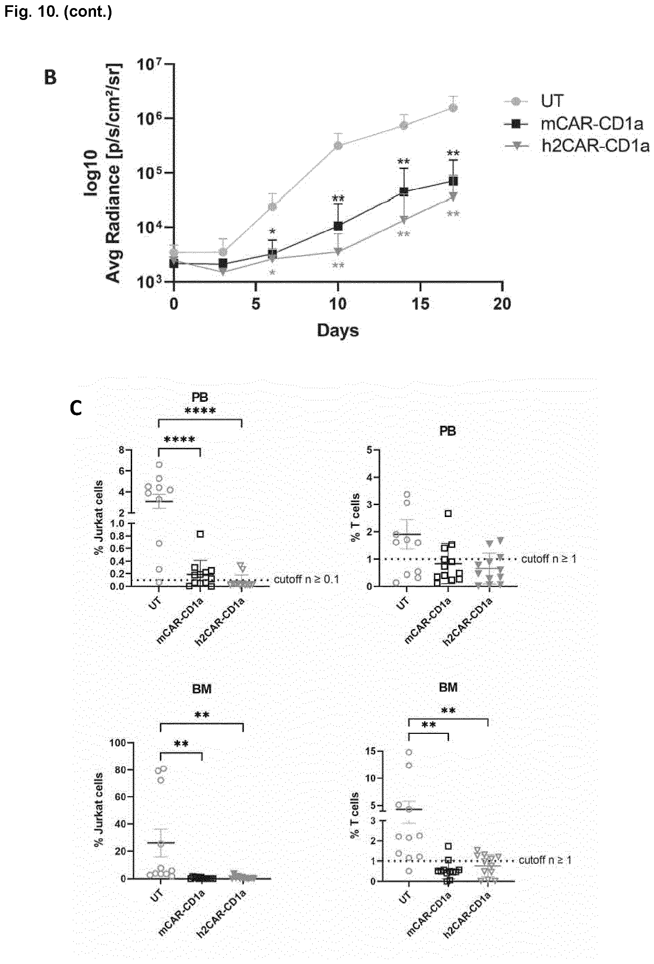

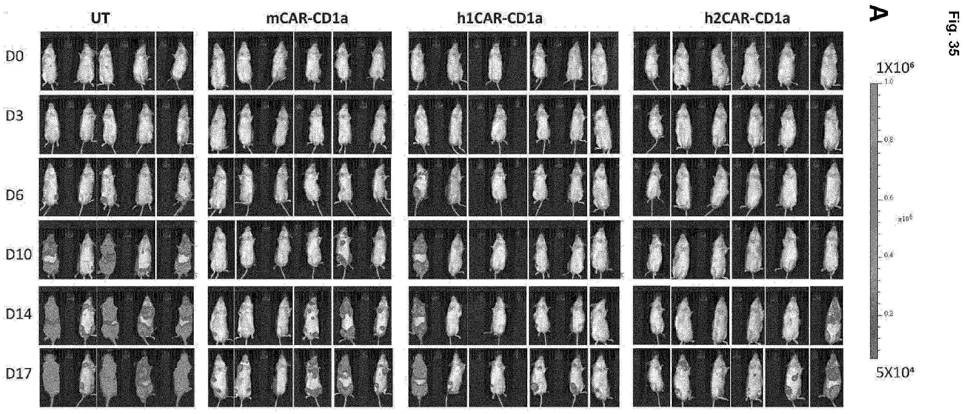

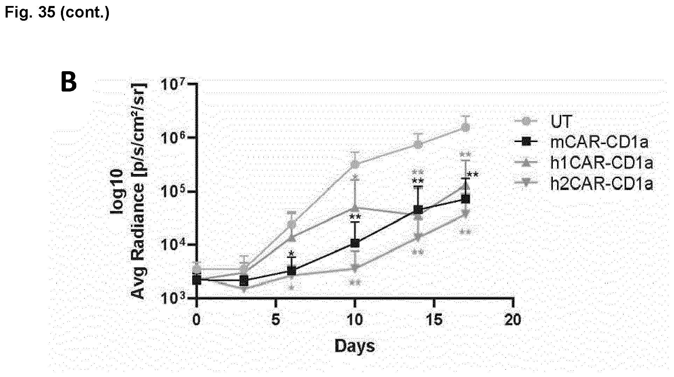

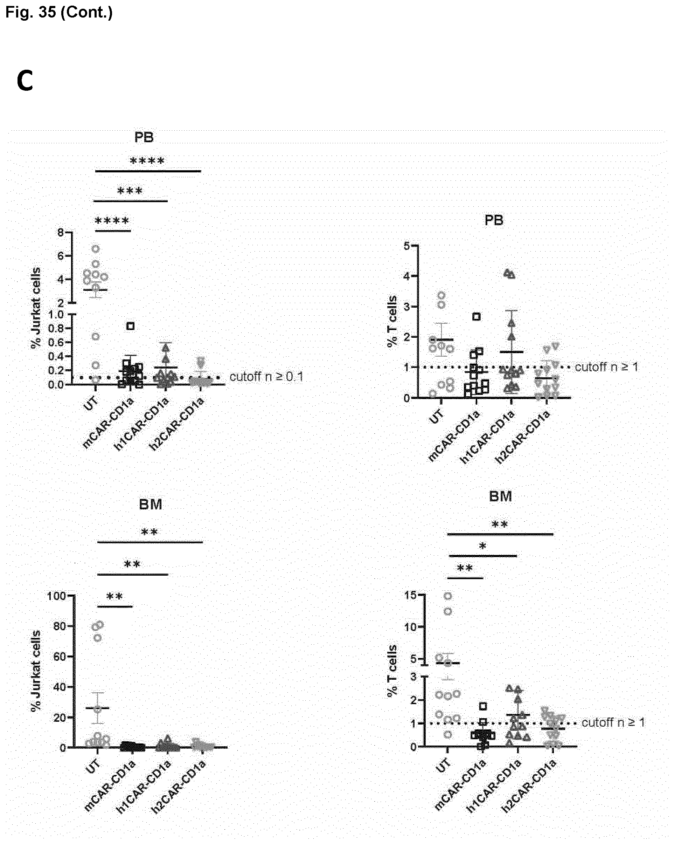

**Figure 10****.** In vivo activity of mCAR-CD1a and h2CAR-CD1a against Jurkat cells. NSG mice were IV injected with 3 ×10⁶ Luc/GFP-expressing Jurkat cells followed 3 days after by a single IV injection of 5×10⁶ mock (UT) or CD1a CAR-Ts. A) IVIS imaging of tumor burden monitored by BLI at the indicated time days (D). Representative BLI images from one experiment are shown. Color bar indicates intensity levels. B) Average radiance quantification (p/sec/cm2/sr) at the indicated days shows all CAR-CD1a versions efficiently abolish progression of coT-ALL cells in mice xenografts (n=6 mice/group). Statistical analysis: Unpaired two-sample t test related to UT. C) Circulating Jurkat cells in PB and in BM after animals were euthanized. Statistical analysis: ANOVA test (Tukey correction for multiple comparisons) related to UT. BLI, bioluminescence; BM, bone marrow; PB, peripheral blood; UT, untransduced PBMCs. *p<0.05, **p<0.01, ***p<0.001, ****p<0.0001. Complete data with h1 is also shown in Fig. 35. Arbitrary cutoffs were indicated.

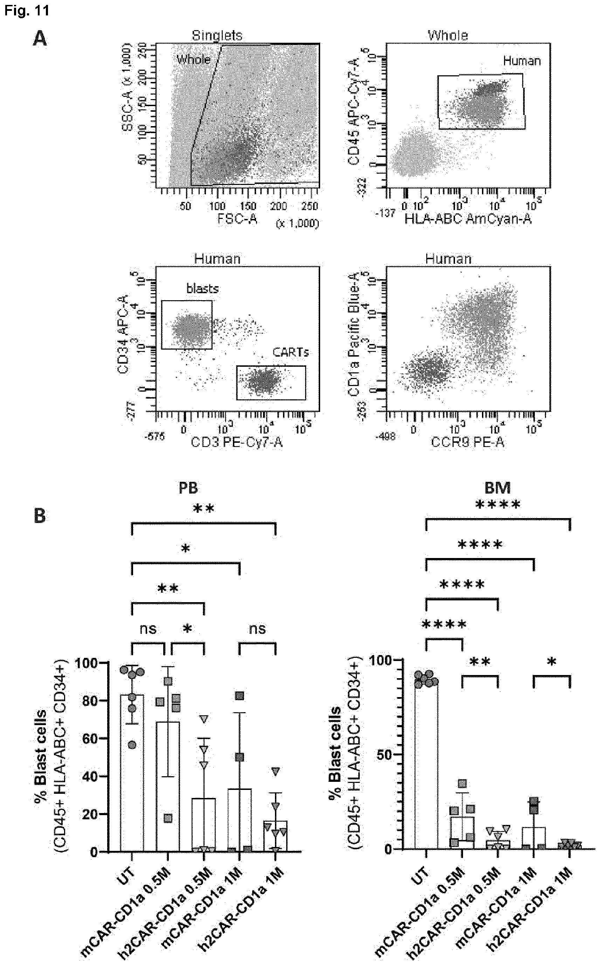

**Figure 11****.** *In vivo* activity of mCAR-CD1a and h2CAR-CD1a against coT-ALL blasts in a PDX setting. NSG mice (n=6/group) were IV injected with 0,5×10⁶ primary coT-ALL cells followed 3 days after by a single IV injection of 0,5 or 1×10⁶ mock (UT) or CD1a-CAR-Ts. Tumor burden was monitored according to FACS by bleeding and BM aspirate after 6 weeks. A) Representative FACS showing gating strategy to quantify blast and CAR-T cells. CD1a+ and CCR9 markers were used to confirm T-ALL blast CD45+/HLA-ABC+/CD34+ cell populations. B) and C) T-ALL blast cells in PB and in BM, respectively, after animals were euthanized after 6 weeks. Statistical analysis: One-way ANOVA corrected for multiple comparisons. BM, bone marrow; PB, peripheral blood; UT, untransduced PBMCs. *p<0.05, **p<0.01, ***p<0.001, ****p<0.0001; ns, not significant.

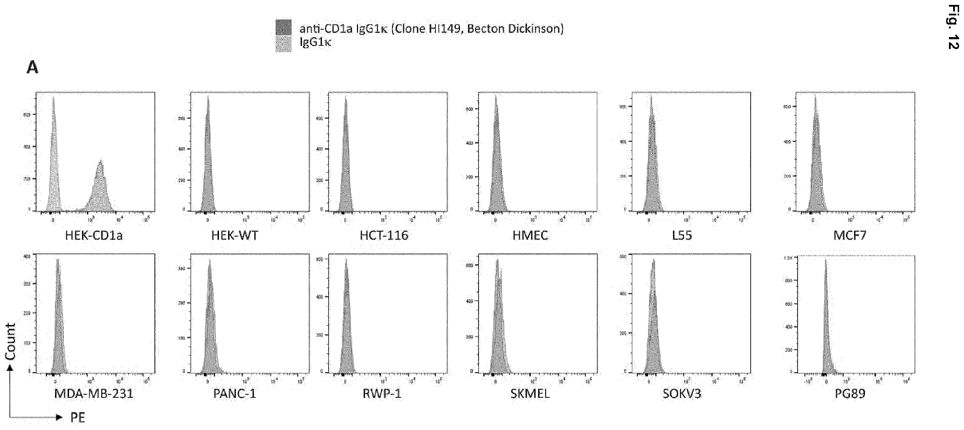

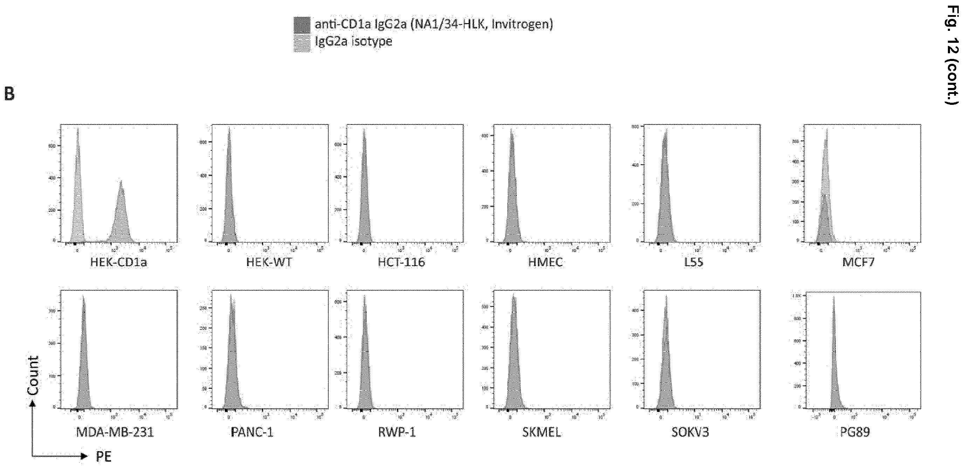

**Figure 12****.** FACS histogram analysis of CD1a expression with indicated antibodies in several adherent cells lines of different origins (A and B). HEK-CD1a are HEK-WT infected with a CD1a lentivirus.

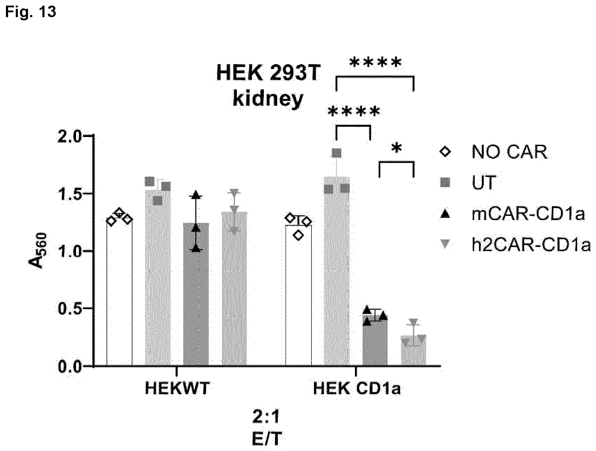

**Figure 13****.** Cell viability monitored by MTT incorporation at 72 hours with HEK293T cells WT and stably expressing CD1a. UT, untransduced PBMCs; WT, wild-type. ****p<0.001, *p<0.05. Two-way ANOVA corrected for multiple comparisons.

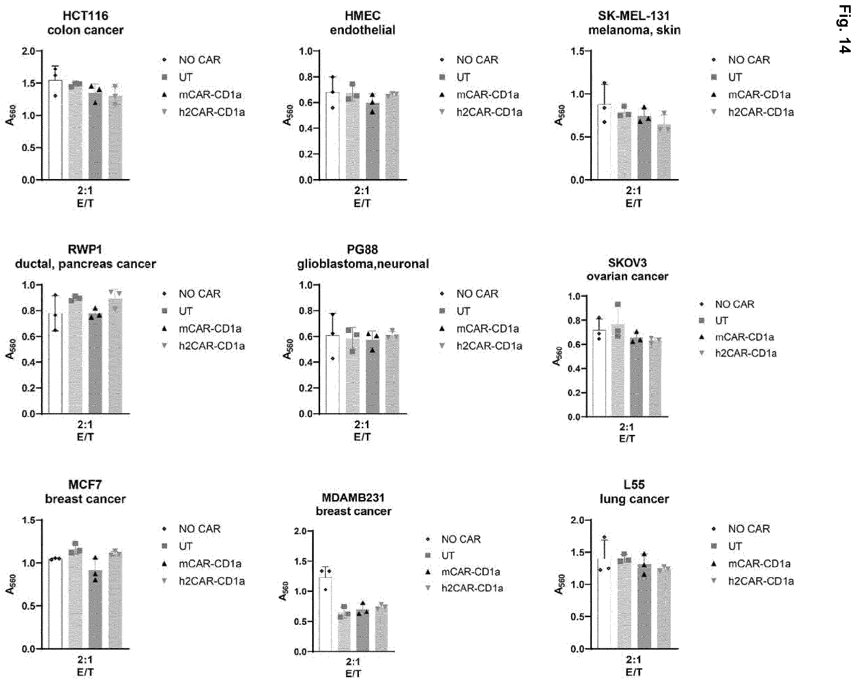

**Figure 14****.** Cell viability monitored by MTT incorporation at 72 hours with indicated cell lines. UT, untransduced PBMCs.

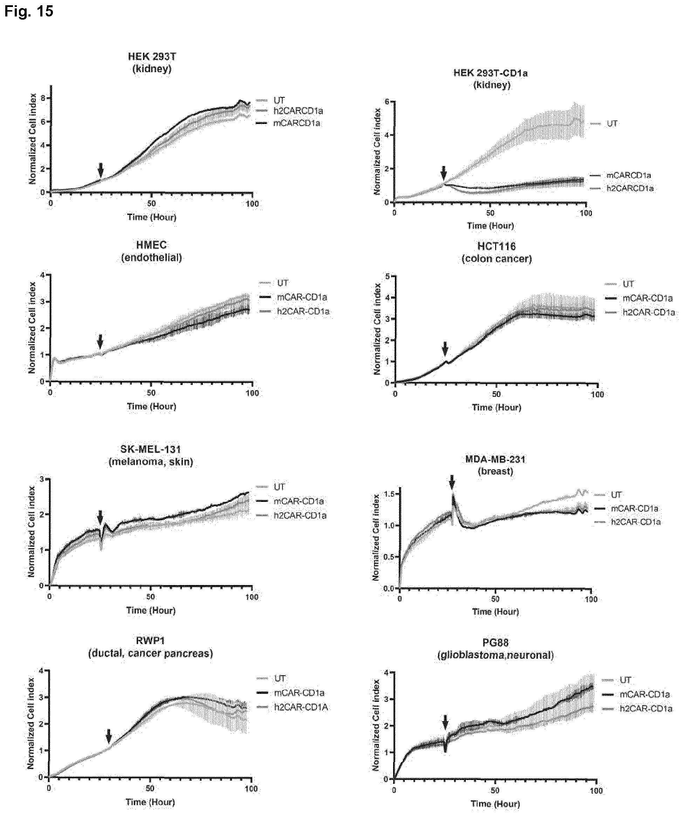

**Figure 15****.** Cell proliferation quantified as normalized cell index and monitored by the XCELLigence system in a real-time dependent fashion. UT, untransduced PBMCs. HEK293T-CD1a were used as a positive control of cell death in the presence of mCAR-CD1a and h2CAR-CD1a (right upper panel).

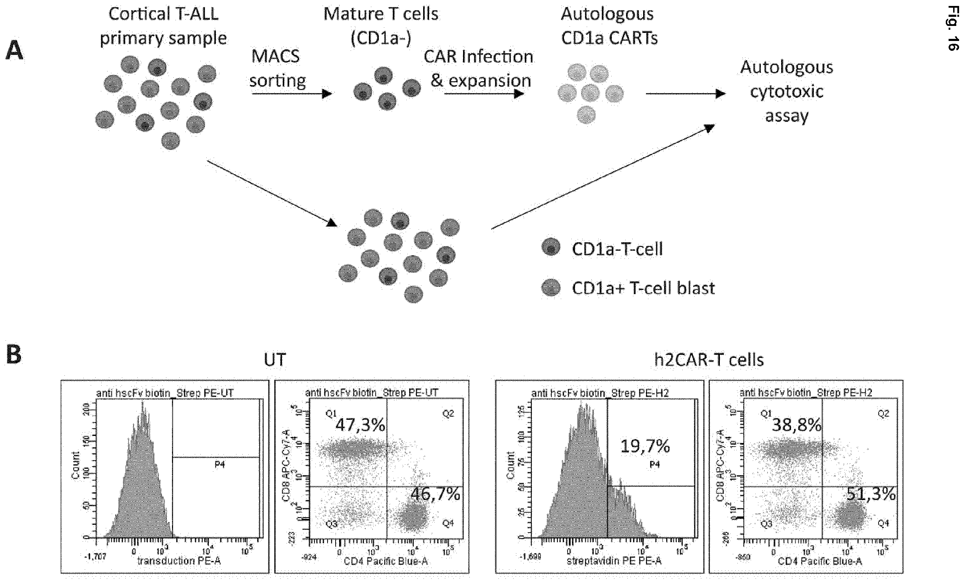

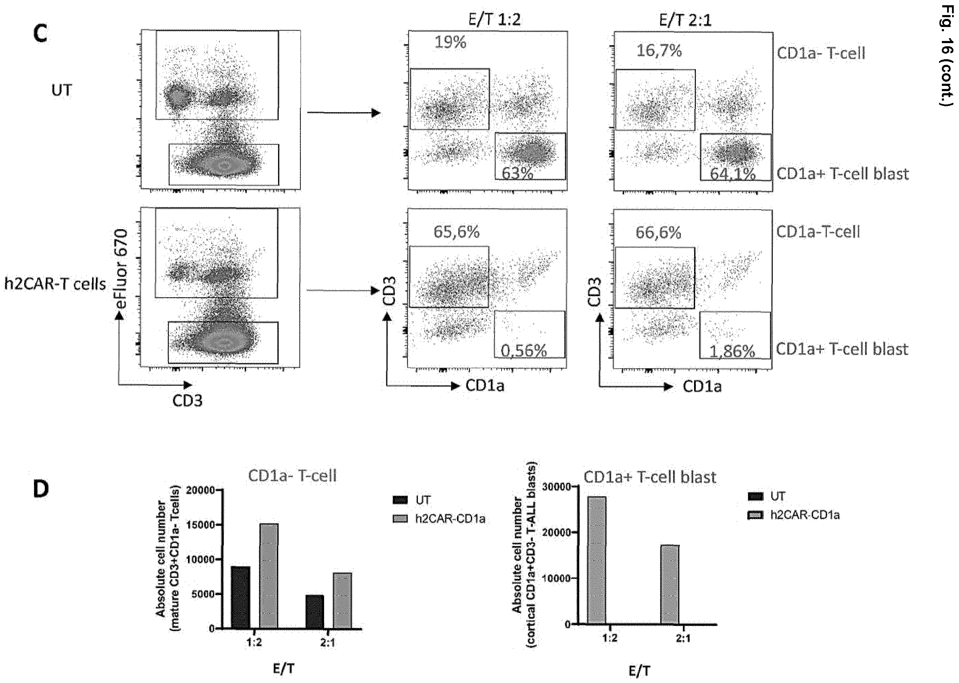

**Figure 16****.** h2CD1a-CAR-Ts derived from patients with coT-ALL specifically lyse autologous CD1a+ T-ALL blasts. (A) Scheme depicting the experimental design for the autologous cytotoxic assay. Mature (normal) CD3+CD1a- T cells were MACS-purified from the PB of a patient with coT-ALL, infected with h2CAR-CD1a, expanded, and exposed to autologous total PBMCs. (B) Representative FACS plots showing transduction efficiency and percentage of CD4+ and CD8+ from transduced mature T cells (CD3+CD1a-). (C) Cytotoxic 48-hour assay at 1:2 and 2:1 E:T ratios. Panels show representative FACS analysis of eFluor labelled target cells co-cultured with UT or h2CD1a-CAR-T cells (eFluor-/CD3+). Right panels show the quantification of blast T-cells (CD3-/CD1a+) and normal T-cells (CD3+/CD1a). D) Panels show absolute cell number quantification of alive mature CD3+CD1a- T cells and cortical CD3-CD1a+ T-ALL blasts.

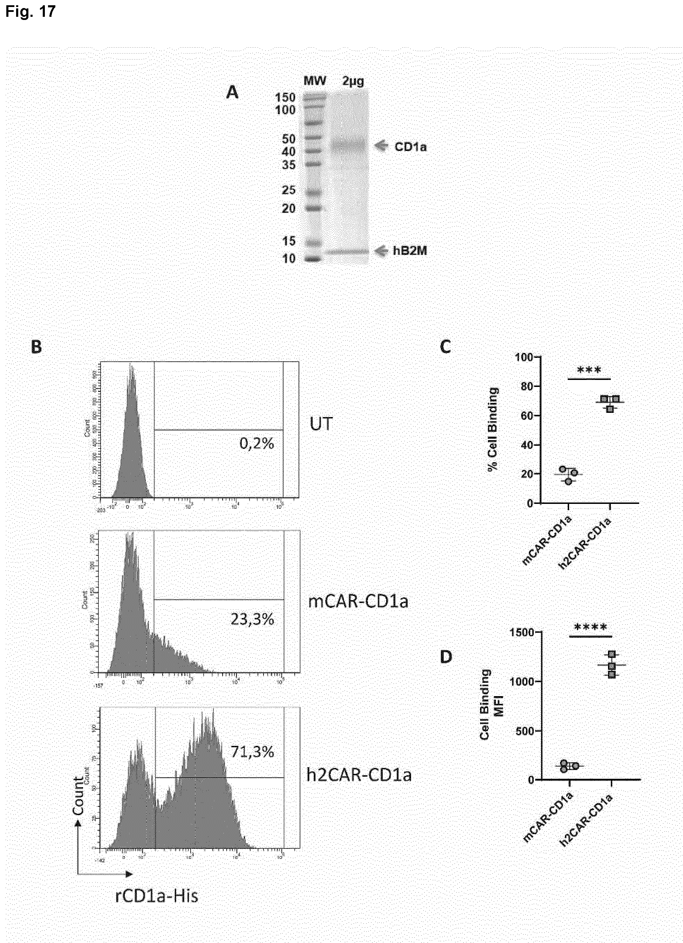

**Figure 17****.** Binding or recombinant protein CD1a (rCD1a) to h2CD1a and mCD1a-CAR-T cells. A) Coomassie stained gel of purified recombinant CD1a produced as a dimer with hb2-microglobulin. MW, standard molecular weight markers. B) FACS analysis of h2CD1a-CAR-Ts and mCD1a-CAR-Ts that were incubated with 25 nM rCD1a in the same conditions. Percentages of rCD1a positive bound cells are indicated. C) Binding quantification of rCD1a to CAR-Ts from three different PBMC donors infected with h2CD1a-CAR or mCD1a-CAR, expressed as % of rCD1a bound to cells (1×10⁵ cells/condition; 25 nM rCD1a). D) Binding quantification calculated as median fluorescence intensity (geometric) MFI. ***p<0.001; ****p<0,0001. Two-tailed unpaired T test.

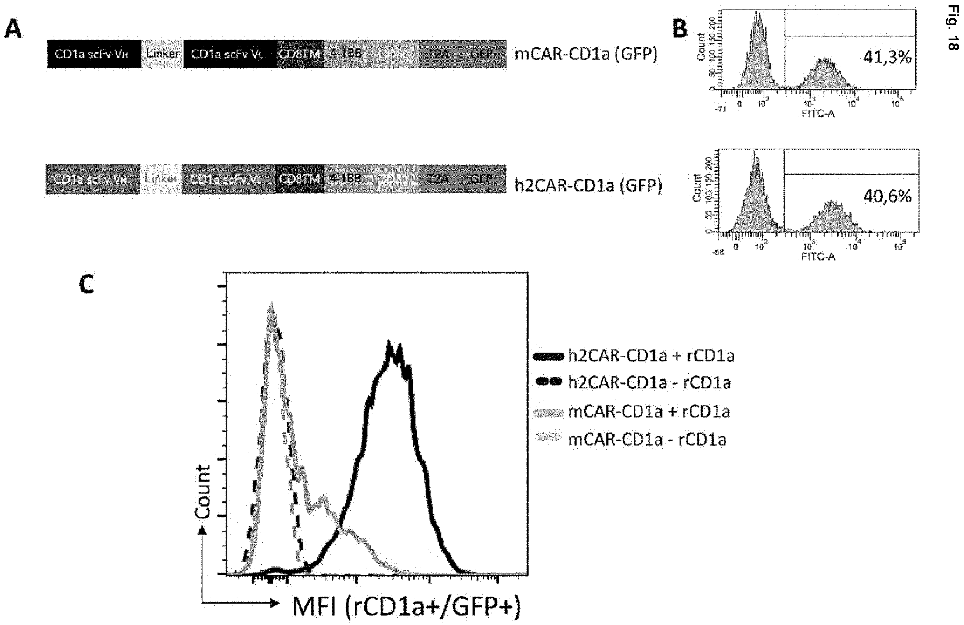

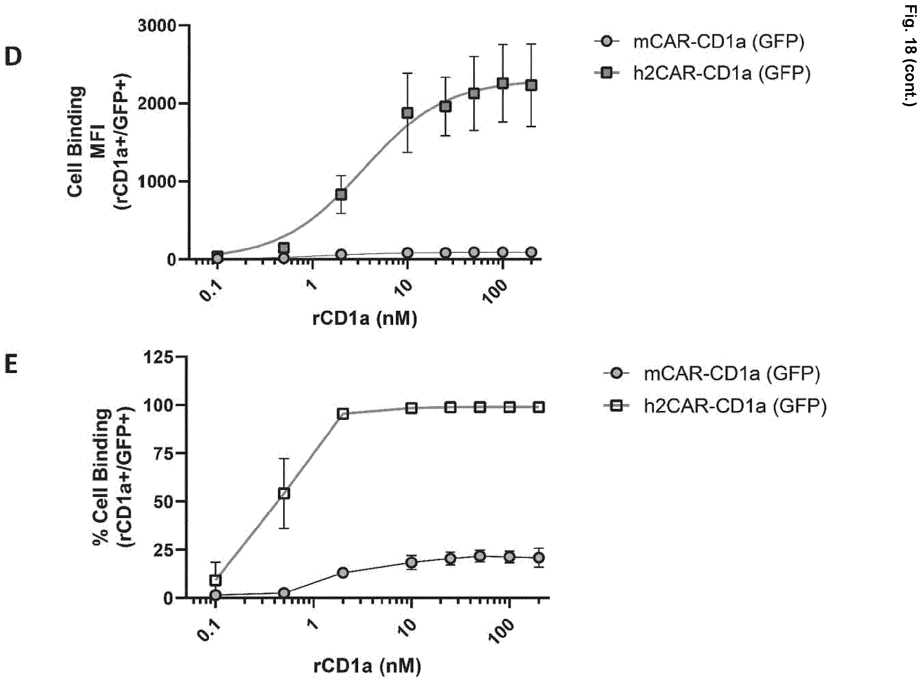

**Figure 18****.** Binding or recombinant protein CD1a (rCD1a) to h2CD1a and mCD1a-CAR-T cells co-expressing GFP for data normalization. A) Murine and humanized CAR-CD1a co-expressing the CAR and GFP. B) Representative FACS histogram showing similar GFP expression of mCD1a and h2CD1a-CAR-Ts. C) FACS histogram overlay showing rCD1a (25 nM) binding to indicated CAR-Ts (solid line) compared to control cells without rCD1a (dashed lines). Median fluorescence intensity (geometric) MFI of rCD1a+ cells in the GFP+ cell population. D) Dose-dependent specific binding quantification of rCD1a to GFP+ CAR-Ts from three different PBMC donors infected with h2CD1a-CAR (GFP) or mCD1a-CAR (GFP), expressed as median fluorescence intensity (geometric) MFI (1×10⁵ cells/condition). Curve was adjusted to one-site binding with a Kd=3,41 nM and Bmax=2304 nM for h2CD1a-CAR-T cells. E) Binding quantification calculated as % of rCD1a bound to GFP + cells (1×10⁵ cells/condition).

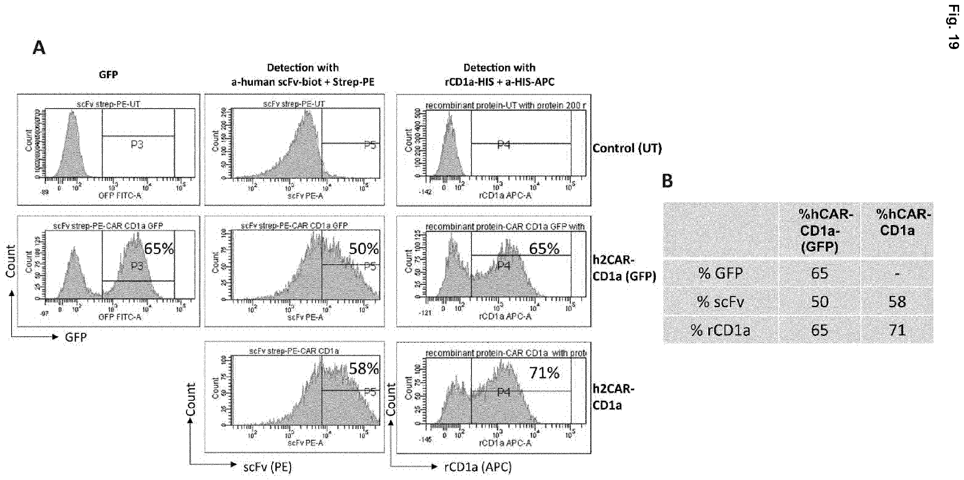

**Figure 19****.** Comparative binding analysis of PBMCs transduced with h2CAR-CD1a (GFP) and with GFP as a control, showing the detection with GFP, anti-human-scFv and rCD1a binding. Similar analysis with h2CAR-CD1a (without GFP) comparing CAR detection with anti-human-scFv to rCD1a binding. Quantification showing a better detection with rCD1a than with anti-scFv is indicated. Table inset shows quantifications.

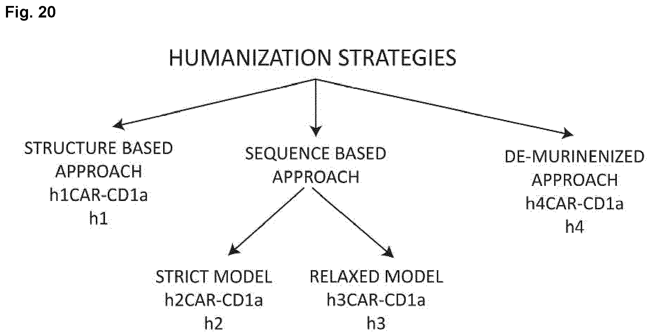

**Figure 20****.** Summary scheme of humanization strategies followed to obtain the different humanization constructs h1CAR-CD1a (h1), h2CARCD1a (h2), h3CAR-CD1a (h3) and h4CAR-CD1a (h4).

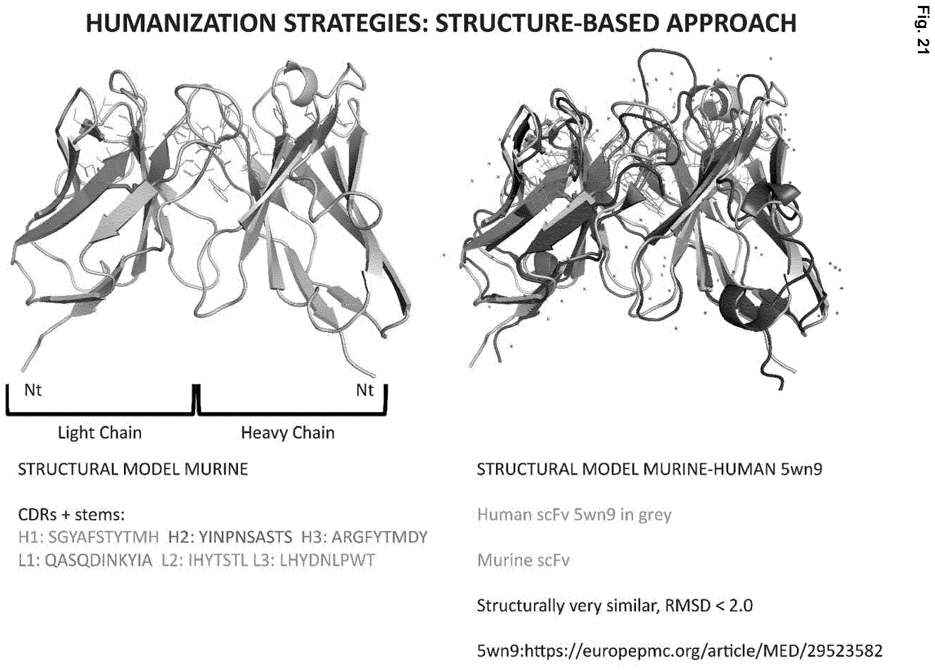

**Figure 21****.** Structure-based humanization. In silico prediction of the anti-CD1a murine scFv 3D structure (left) and structural overlapping between structures of murine and proposed human scFv 5wn9 (right). Calculated root-mean-square deviation (RMSD) (RMSD <2 is indicative of highly similar structures.

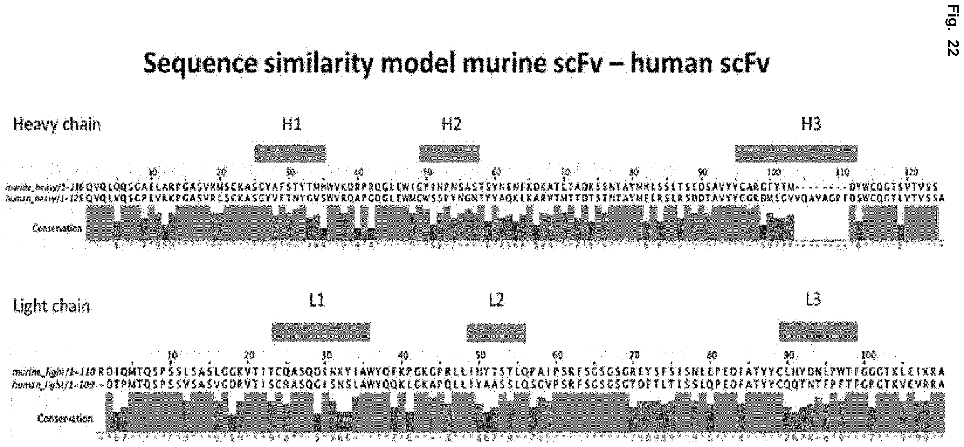

**Figure 22****.** Structure-based humanization. Sequence alignment between murine anti-CD1a scFv and the human 5wn9. CDRs and stems are indicated for the heavy (H1 to H3) and light chains (L1 to L3).

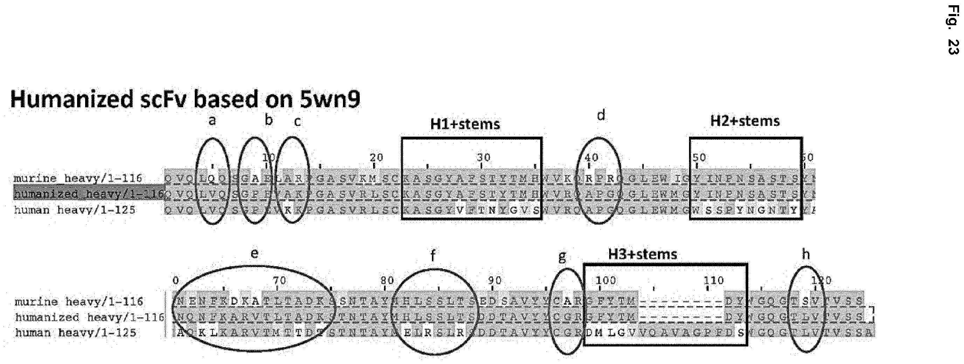

**Figure 23****.** Structure-based humanization. Comparison between the murine and the human 5wn9 heavy chains and proposed humanized sequence (excluding CDRs and stems). Boxes and squares indicate the aminoacids changes introduced (see Examples section for a detailed explanation).

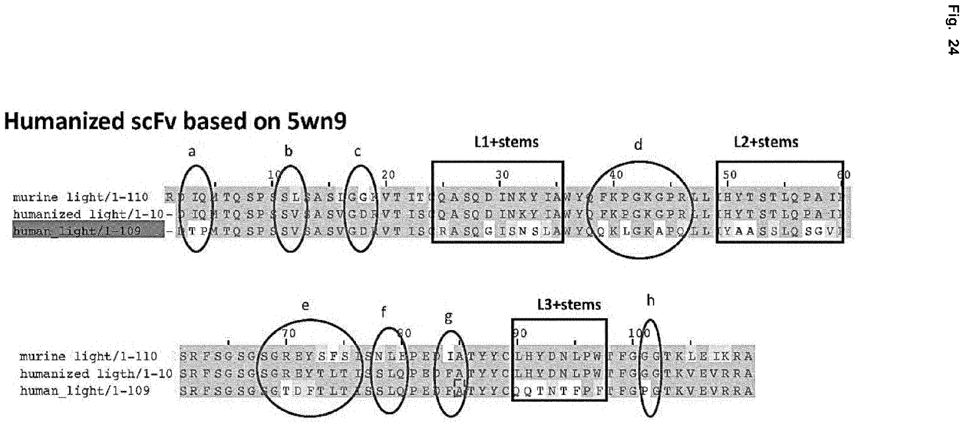

**Figure 24****.** Structure-based humanization. Comparison between the murine and the human 5wn9 light chains and proposed humanized sequence (excluding CDRs and stems). Boxes and squares indicate the aminoacids changes introduced (see Examples section for a detailed explanation).

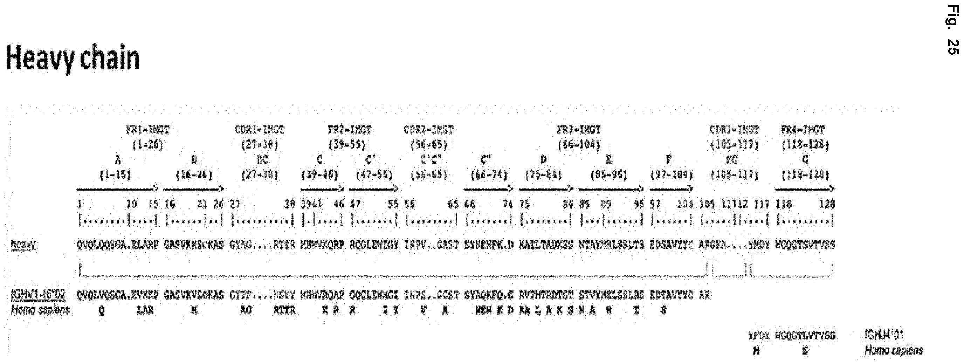

**Figure 25****.** Sequence-based humanization. Alignment between the murine heavy chain and the germline IGHV1-46*02 sequence (Note that the sequences of the CDRs in the heavy chain are slightly different from the original. This was done to not submit the real sequence to a public server).

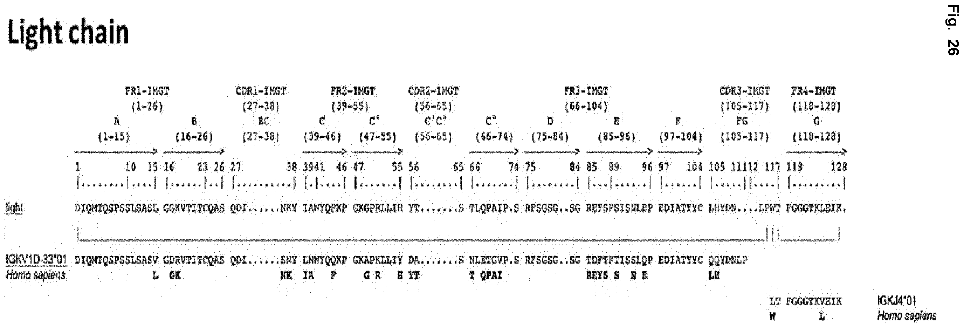

**Figure 26****.** Sequence-based humanization. Alignment between the murine light chain and the germline IGKVD 1D-33*01 sequence (note that the sequences of the CDRs in the heavy chain are slightly different from the original. This was done to not submit the real sequence to a public server).

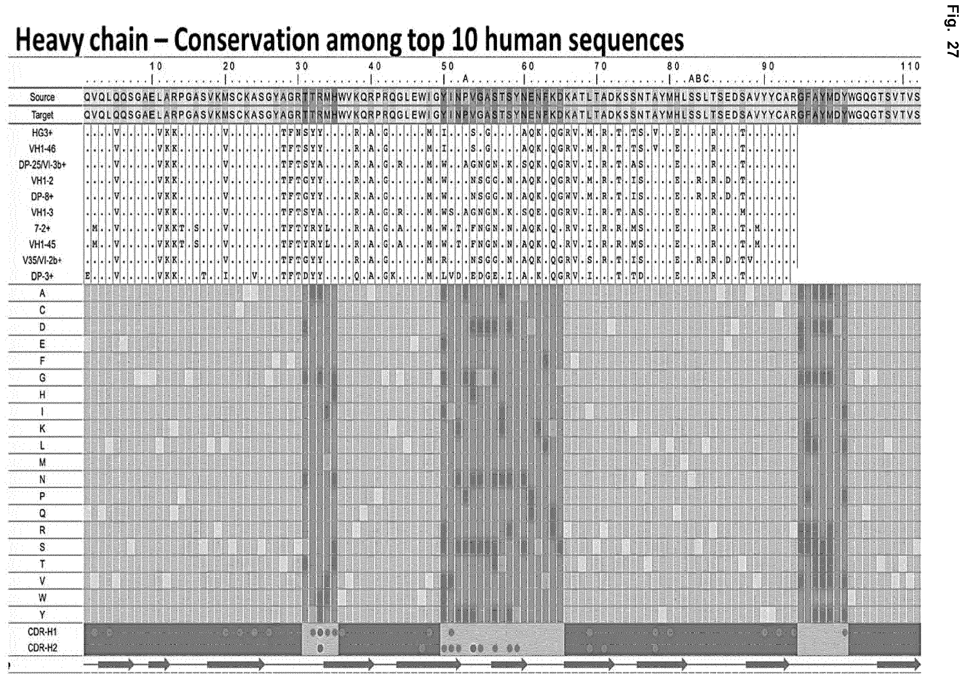

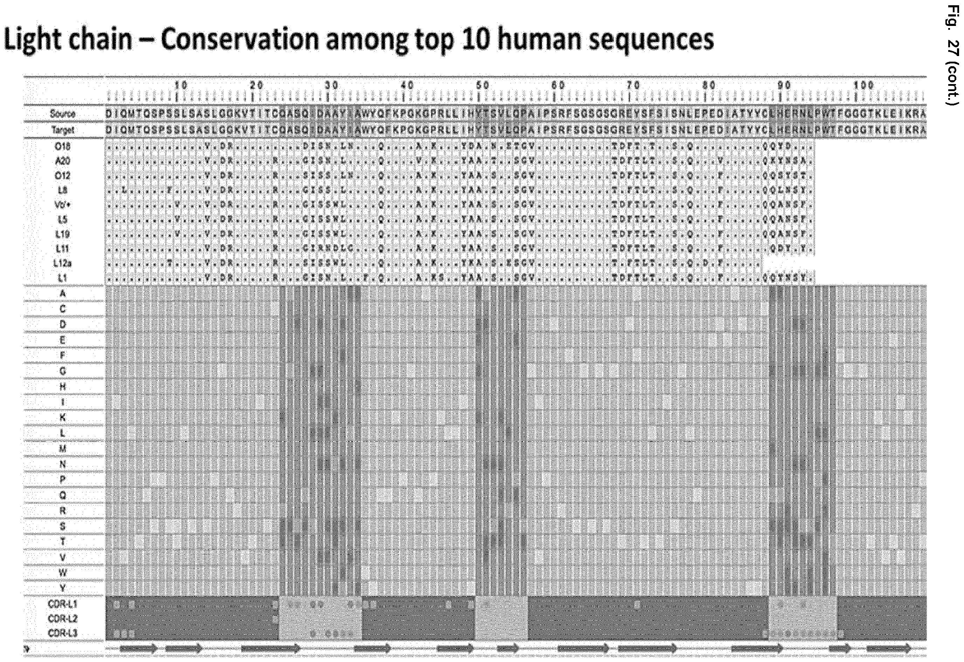

**Figure 27****.** Sequence-based humanization. Heavy and light chain conservation among the different human sequences.

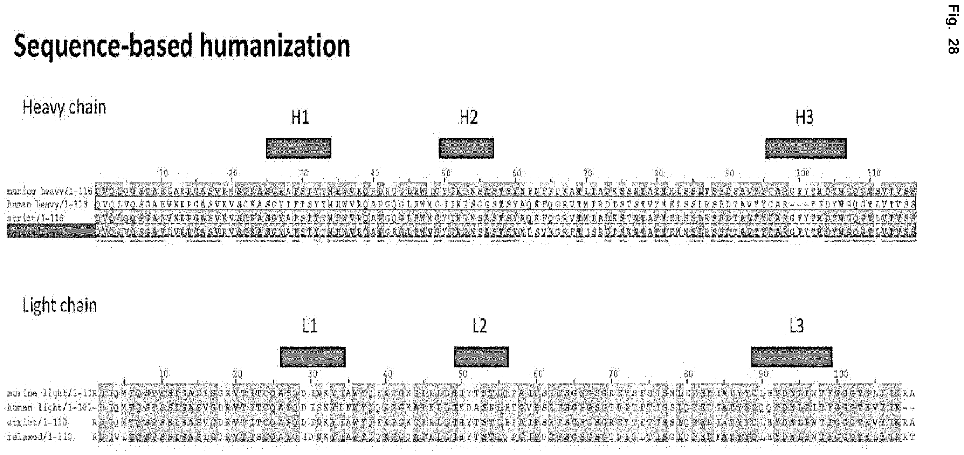

**Figure 28****.** Sequence-based humanization. Upper panel, sequence-based "strict" (minimum changes were introduced) humanized candidate for the heavy chain. Lower panel, sequence-based "relaxed" (more changes were allowed) humanized candidate.

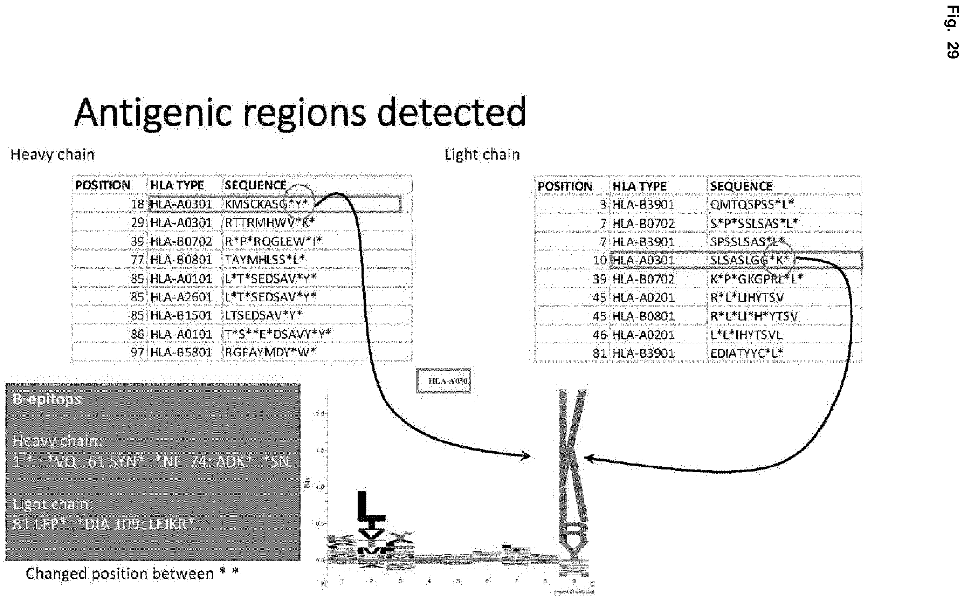

**Figure 29****.** "De-murinenization" approach. Murine scFv protein sequence was submitted to NetMHC - 4.0 NetMHC service that returns the prediction of peptides to MHC class I molecules binding and the corresponding sequence motifs. In addition, the DiscoTope server predicts discontinuous B cell epitopes (square).

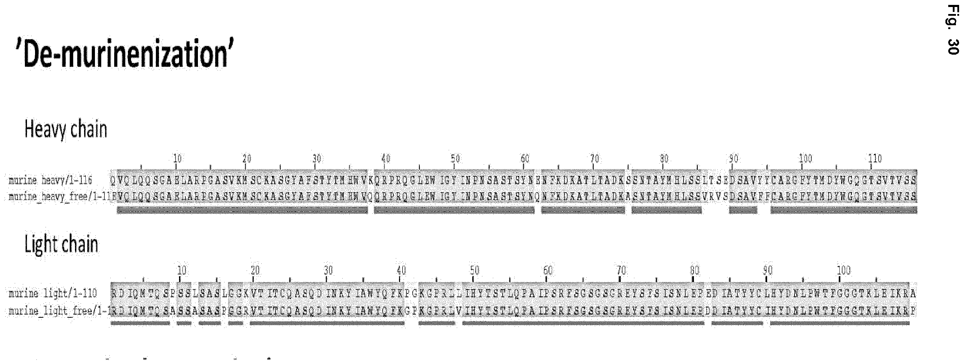

**Figure 30****.** "De-murinenization" approach. Alignment between murine scFv protein sequence (upper) and the corresponding "de-murinenized" candidate (lower) for the heavy and light chain. Un-changed aminoacid sequence are underlined and aminoacid changes have been highlighted.

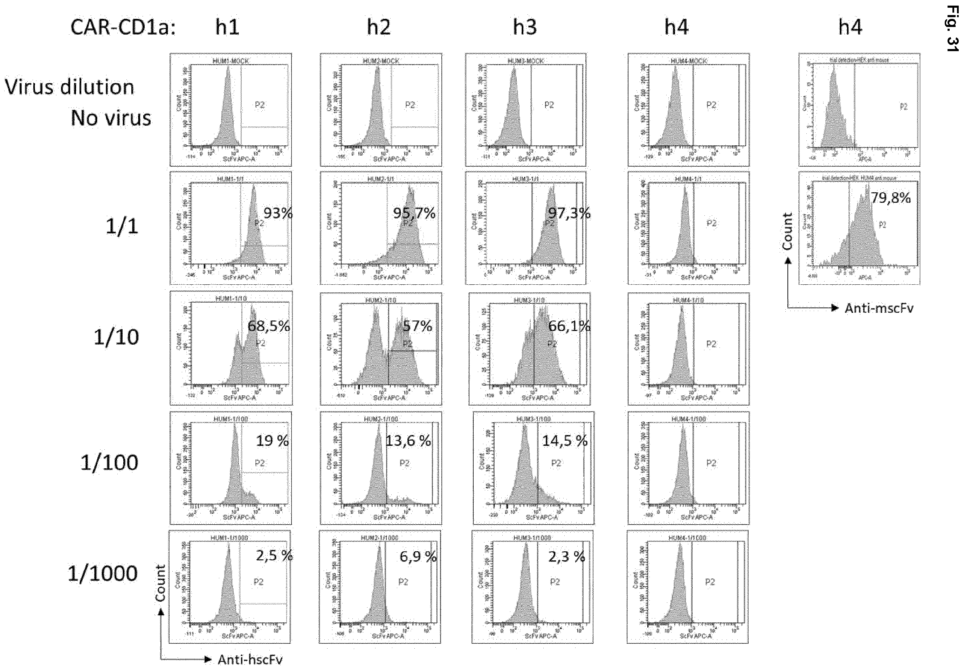

**Figure 31****.** Virus titration with HEK293T of the different Humanized CAR-CD1a constructs and detection with anti-human scFv (anti -hscFv) antibodies shows that all of them may be detected except h4CAR-CD1a that is still detected with only anti-murine antibodies (anti-mscFv, right). Percentages show membrane expression of scFvs respect to non-infected cells.

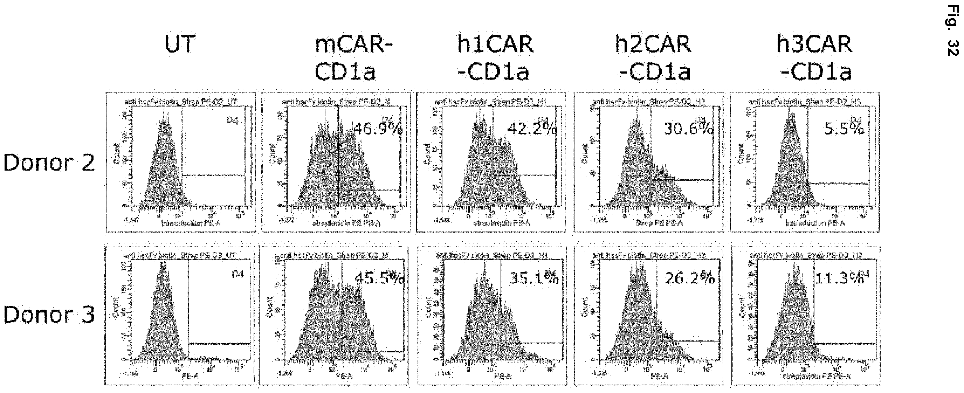

**Figure 32****.** Transduction of 2 different donors with lentiviral particles encoding for mCAR-CD1a and hCAR-CD1a versions h1 to h3. Percentages show membrane expression of scFvs respect to untransduced cells (UT).

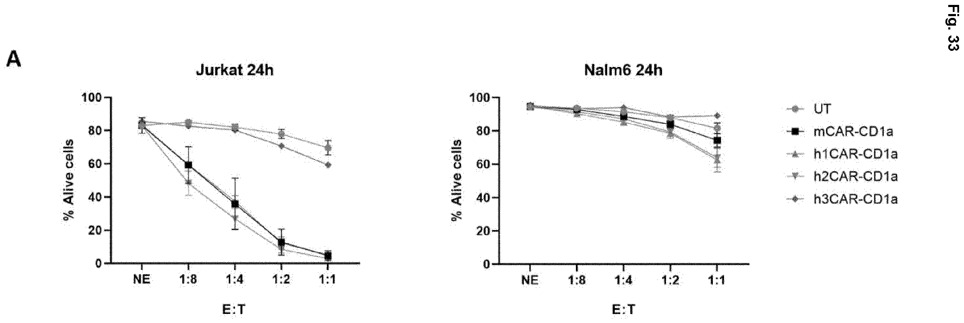

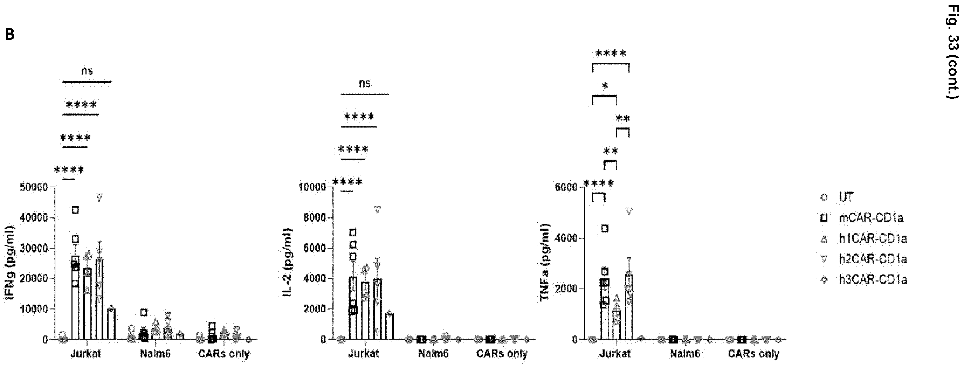

**Figure 33****.** A) Cytotoxicity assays with indicated cell lines (Jurkat CD1a+, Nalm6 CD1a- control) and the different scFv versions anti-CD1a of humanized and murine CAR-Ts at different effector/target ratios. Levels of viable cells were quantified by FACS analysis of eFluor positive cells at 24h of co-culture. UT, untransduced PBMCs. NE, non-effector cells. B) Levels of pro-inflammatory cytokines determined by ELISA. Data were pooled from two independent experiments with 3 different donors *p<0.05, **p<0.01, ***p<0.001, ****p<0.0001; ns, not significant. Two-way ANOVA for multiple comparisons. ELISA, enzyme-linked immunosorbent assay; UT, untransduced PBMCs.

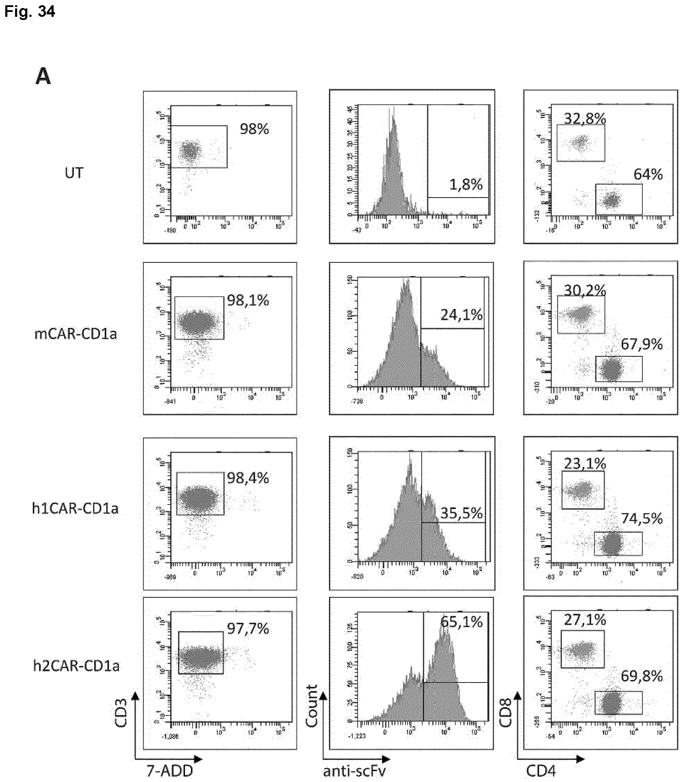

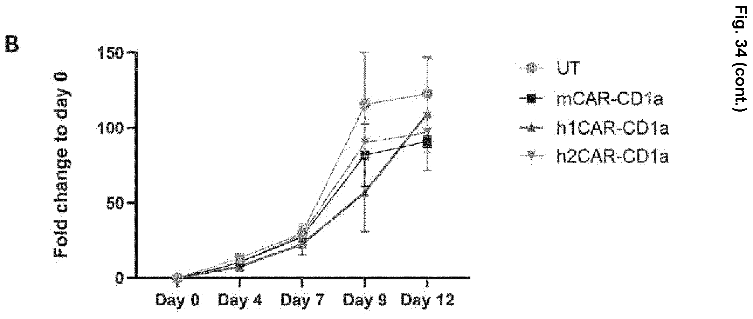

**Figure 34****.** A) FACS analysis of transduced PBMCs with mCAR-CD1a and h1 and h2 CAR-CD1a. Percentages show membrane expression of scFvs, CD4+ and CD8+ cells. UT, untransduced PBMCs. B) Proliferation of CAR-Ts transduced with the indicated constructs.
**Figure 35****.** *In vivo* activity of mCAR-CD1a and hCAR-CD1a (h1 and h2). A) IVIS imaging of tumor burden monitored by BLI at the indicated time points (days, D). (A) Representative BLI images from one experiment are shown. Color bar indicates intensity levels. (B) Average radiance quantification (p/sec/cm2/sr) at the indicated days shows all CAR-CD1a versions efficiently abolish progression of coT-ALL cells in mice xenografts (n=6 mice/group). C) Circulating Jurkat cells in PB and in BM after animals were euthanized. BLI, bioluminescence; BM, bone marrow; PB, peripheral blood; UT, untransduced PBMCs. Statistical analysis: ANOVA test (Tukey correction for multiple comparisons) related to UT. *p<0.05, **p<0.01, ***p<0.001, ****p<0.0001. Arbitrary cutoffs were indicated.

### Summary of the invention

In one aspect, the present invention provides a humanized CD1a targeting moiety, wherein the CD1a targeting moiety is an antibody, F(ab')2, Fab, scFab or scFv, comprising a VL domain consisting of SEQ ID NO: 1 and a VH domain consisting of SEQ ID NO: 2.

Preferably, the CD1a targeting moiety is a scFv comprising a VL domain consisting of SEQ ID NO: 1 and a VH domain consisting of SEQ ID NO: 2. More preferably, the CD1a targeting moiety is a scFv consisting of SEQ ID NO:3

In a further aspect, the present invention provides a chimeric antigen receptor (CAR) comprising:
a) an extracellular domain comprising a CD1a targeting moiety, wherein the CD1a targeting moiety is a scFv comprising a VL domain consisting of SEQ ID NO: 1 and a VH domain consisting of SEQ ID NO: 2;
b) a transmembrane domain; and
c) an intracellular signaling domain.

Preferably the transmembrane domain comprises the transmembrane domain of CD28, CD3, CD45, CD4, CD8, CD9, CD16, CD22, CD33, CD37, CD64, CD80, CD86, CD134, CD137, or CD154. Preferably, the intracellular domain of CD3ζ, FcRy, CD3y, CD3δ, CD3ε, CD5, CD22, CD79a, CD79b or CD66b. Preferably, the CAR further comprises a costimulatory signaling domain, preferably the costimulatory signaling domain comprises the intracellular domain of CD27, CD28, CD137, CD134, CD30, CD40, lymphocyte function-associated antigen-1 (LFA-1), CD2, CD7, LIGHT, NKG2C, or CD276. Most preferably, the CAR consists of SEQ ID NO: 13.

In a further aspect, the present invention provides a nucleic acid encoding the CAR according to the previous aspect.

In one aspect, the present invention provides a cell comprising the nucleic acid according to the previous aspect. Preferably, the cell is a T-cell.

In another aspect, the present invention provides a pharmaceutical composition comprising a plurality of cells according to the previous aspect and a pharmaceutically acceptable carrier or diluent.

In another aspect, the present invention provides a cell according to the previous aspect for use as a medicament. Preferably, the use is in a method of treating a CD1a-positive cancer, wherein the method comprises administering the cell or composition to a patient in need thereof. Preferably, the CD1a-positive cancer is cortical T-cell acute lymphoblastic leukemia, preferably, relapsed/refractory cortical T-cell acute lymphoblastic leukemia.

### Definitions

"Administering" or "administration of" a medicament to a patient (and grammatical equivalents of this phrase) refers to direct administration, which may be administration to a patient by a medical professional or may be self-administration, and/or indirect administration, which may be the act of prescribing a drug. E.g., a physician who instructs a patient to self-administer a medicament or provides a patient with a prescription for a drug is administering the drug to the patient.

The term "affibody" refers to a protein that is derived from the Z domain of protein A and that been engineered to bind to a specific target (see Frejd & Kim, 2017. Exp Mol Med. 49(3): e306).

The term "antibody" refers to a molecule comprising at least one immunoglobulin domain that binds to, or is immunologically reactive with, a particular target. The term includes whole antibodies and any antigen binding portion or single chains thereof and combinations thereof; for instance, the term "antibody" in particular includes bivalent antibodies and bivalent bispecific antibodies.

A typical type of antibody comprises at least two heavy chains ("HC") and two light chains ("LC") interconnected by disulfide bonds.

Each "heavy chain" comprises a "heavy chain variable domain" (abbreviated herein as "VH") and a "heavy chain constant domain" (abbreviated herein as "CH"). The heavy chain constant domain typically comprises three constants domains, CH1, CH2, and CH3.

Each "light chain" comprises a "light chain variable domain" (abbreviated herein as "VL") and a "light chain constant domain" ("CL"). The light chain constant domain (CL) can be of the kappa type or of the lambda type. The VH and VL domains can be further subdivided into regions of hypervariability, termed Complementarity Determining Regions ("CDR"), interspersed with regions that are more conserved, termed "framework regions" ("FW").

Each VH and VL is composed of three CDRs and four FWs, arranged from amino-terminus to carboxy-terminus in the following order: FW1, CDR1, FW2, CDR2, FW3, CDR3, FW4. The present disclosure inter alia presents VH and VL sequences as well as the subsequences corresponding to CDR1, CDR2, and CDR3.

The precise amino acid sequence boundaries of a given CDR can be determined using any of a number of well-known schemes, including those described by Kabat et al. (1991), "Sequences of Proteins of Immunological Interest," 5th Ed. Public Health Service, National Institutes of Health, Bethesda, MD ("Kabat" numbering scheme), Al-Lazikani et al., (1997) JMB 273,927-948 ("Chothia" numbering scheme).

Accordingly, a person skilled in the art would understand that the sequences of FW1, FW2, FW3 and FW4 are equally disclosed. For a particular VH, FW1 is the subsequence between the N-terminus of the VH and the N-terminus of H-CDR1, FW2 is the subsequence between the C-terminus of H-CDR1 and the N-terminus of H-CDR2, FW3 is the subsequence between the C-terminus of H-CDR2 and the N-terminus of H-CDR3, and FW4 is the subsequence between the C-terminus of H-CDR3 and the C-terminus of the VH. Similarly, for a particular VL, FW1 is the subsequence between the N-terminus of the VL and the N-terminus of L-CDR1, FW2 is the subsequence between the C-terminus of L-CDR1 and the N-terminus of L-CDR2. FW3 is the subsequence between the C-terminus of L-CDR2 and the N-terminus of L-CDR3, and FW4 is the subsequence between the C-terminus of L-CDR3 and the C-terminus of the VL.

The variable domains of the heavy and light chains contain a region that interacts with a binding target, and this region interacting with a binding target is also referred to as an "antigen-binding site" or "antigen binding site" herein. The constant domains of the antibodies can mediate the binding of the antibody to host tissues or factors, including various cells of the immune system (e.g., effector cells) and the first component (C1q) of the classical complement system. Exemplary antibodies of the present disclosure include typical antibodies, but also bivalent fragments and variations thereof such as a F(ab')2.

As used herein, the term "antibody" encompasses intact polyclonal antibodies, intact monoclonal antibodies, bivalent antibody fragments (such as F(ab')2), multispecific antibodies such as bispecific antibodies, chimeric antibodies, humanized antibodies, human antibodies, and any other modified immunoglobulin molecule comprising an antigen binding site.

An antibody can be of any the five major classes (isotypes) of immunoglobulins: IgA, IgD, IgE, IgG, and IgM, or subclasses thereof (e.g. IgG1, IgG2, IgG3, IgG4, IgA1 and IgA2), based on the identity of their heavy-chain constant domains referred to as alpha, delta, epsilon, gamma, and mu, respectively. The different classes of immunoglobulins have different and well known subunit structures and three-dimensional configurations. Antibodies can be naked or conjugated to other molecules such as therapeutic agents or diagnostic agents to form immunoconjugates.

The term "anticalin" refers to a protein that is derived from the lipocalin and that been engineered to bind to a specific target (see Skerra, 2008. FEBS J. 275(11):2677-83).

The term "antigen-binding fragment" or "Fab" refers to an antibody fragment comprising one constant and one variable domain of each of the heavy and light chain. A Fab fragment may be obtained by digesting an intact monoclonal antibody with papain.

The term "cancer" refers to a group of diseases, which can be defined as any abnormal benign or malignant new growth of tissue that possesses no physiological function and arises from uncontrolled usually rapid cellular proliferation and has the potential to invade or spread to other parts of the body.

The term "CD1a" refers to a non-polymorphic MHC Class 1 related cell surface glycoprotein, expressed in association with β-2-microglobulin. CD1a is expressed by cortical thymocytes, Langerhans cells and by interdigitating cells. CD1a is also expressed by some malignancies of T cell lineage and in Langerhans cell histiocytosis. CD1a is expressed on cortical thymocytes, epidermal Langerhans cells, dendritic cells, on certain T-cell leukemias, and in various other tissues. CD1a is structurally related to the major histocompatibility complex (MHC) proteins and form heterodimers with β-2-microglobulin. Exemplary sequence and data related to human CD1a has been deposited in the UniProtKB database under ID number P06126.

"CD1a-positive" cancer, including a "CD1a-positive" cancerous disease, is one comprising cells, which have CD1a present at their cell surface. The term "CD1a-positive" also refers to a cancer that produces sufficient levels of CD1a at the surface of cells thereof, such that a CAR-comprising cell of the present invention has a therapeutic effect, mediated by the binding of the CAR to CD1 a. In some embodiments, the CD1a-positive cancer is cortical T-cell acute lymphoblastic leukemia or Langerhans cell histiocytosis (LCH).

The term "CD1a-targeting moiety" refers to a substance that is able to bind CD1a. Within the context of a CAR, a CD1a-targeting moiety targets T cells to a CD1a-positive cell, preferably a cancer cell. Within the context of a CAR, it is to be understood that the CD1a-targeting moiety is genetically encodable.

The term "chimeric antigen receptor" or "CAR" refers to a synthetic receptor that targets T cells to a chosen antigen and reprograms T cell function, metabolism and persistence (see Rivière & Sadelain, 2017. Mol Ther. 25(5):1117-1124). Similarly, the term "CART" refers to a T cell that comprises a CAR.

"Combination therapy", "in combination with" or "in conjunction with" as used herein denotes any form of concurrent, parallel, simultaneous, sequential or intermittent treatment with at least two distinct treatment modalities (i.e., compounds, components, targeted agents or therapeutic agents). As such, the terms refer to administration of one treatment modality before, during, or after administration of the other treatment modality to the subject. The modalities in combination can be administered in any order. The therapeutically active modalities are administered together (e.g., simultaneously in the same or separate compositions, formulations or unit dosage forms) or separately (e.g., on the same day or on different days and in any order as according to an appropriate dosing protocol for the separate compositions, formulations or unit dosage forms) in a manner and dosing regimen prescribed by a medical care taker or according to a regulatory agency. In general, each treatment modality will be administered at a dose and/or on a time schedule determined for that treatment modality. Optionally, three or more modalities may be used in a combination therapy. Additionally, the combination therapies provided herein may be used in conjunction with other types of treatment. For example, other anti-cancer treatment may be selected from the group consisting of chemotherapy, surgery, radiotherapy (radiation) and/or hormone therapy, amongst other treatments associated with the current standard of care for the subject.

A "complete response" or "complete remission" or "CR" indicates the disappearance of all target lesions as defined in the RECIST v1.1 guideline. This does not always mean the cancer has been cured.

The term "costimulatory signaling domain" refers to a signaling moiety that provides to T cells a signal which, in addition to the primary signal provided by for instance the CD3ζ chain of the TCR/CD3 complex, mediates a T cell response, including, but not limited to, activation, proliferation, differentiation, cytokine secretion, and the like. A co-stimulatory domain can include all or a portion of, but is not limited to, CD27, CD28, 4-1BB (CD137), OX40 (CD134), CD30, CD40, 1COS, lymphocyte function-associated antigen-1 (LFA-1), CD2, CD7, LIGHT, NKG2C, B7-H3, and a ligand that specifically binds with CD83. In some embodiments, the co-stimulatory signaling domain is an intracellular signaling domain that interacts with other intracellular mediators to mediate a cell response including activation, proliferation, differentiation and cytokine secretion, and the like.

The term "designed ankyrin repeat proteins" or "DARPin" refers to a protein that is derived from an ankyrin repeat that has been engineered to bind to a specific target (see Plückthun, 2015. Annu Rev Pharmacol Toxicol. 55:489-511).

"Disease free survival" (DFS) refers to the length of time during and after treatment that the patient remains free of disease.

As used herein, the term "effective amount" of an agent, e.g., a therapeutic agent such as a CART, is that amount sufficient to effect beneficial or desired results, for example, clinical results, and, as such, an "effective amount" depends upon the context in which it is being applied. For example, in the context of administering a therapeutic agent that treats T-ALL, an effective amount can reduce the number of cancer cells; reduce the tumor size or burden; inhibit (i.e., slow to some extent and in a certain embodiment, stop) cancer cell infiltration into peripheral organs; inhibit (i.e., slow to some extent and in a certain embodiment, stop) tumor metastasis; inhibit, to some extent, tumor growth; relieve to some extent one or more of the symptoms associated with the cancer; and/or result in a favorable response such as increased progression-free survival (PFS), disease-free survival (DFS), or overall survival (OS), complete response (CR), partial response (PR), or, in some cases, stable disease (SD), a decrease in progressive disease (PD), a reduced time to progression (TTP) or any combination thereof. The term "effective amount" can be used interchangeably with "effective dose," "therapeutically effective amount," or "therapeutically effective dose".

The term "fynomer" refers to a protein that is derived from the SH3 domain of human Fyn kinase that has been engineered to bind to a specific target (see Bertschinger et al., 2007. Protein Eng Des Sel. 20(2):57-68).

The terms "individual", "patient" or "subject" are used interchangeably in the present application to designate a human being and are not meant to be limiting in any way. The "individual", "patient" or "subject" can be of any age, sex and physical condition. The term "patient in need thereof" usually refers to a patient who suffers from a CD1a-positive cancer.

"Infusion" or "infusing" refers to the introduction of a therapeutic agent-containing solution into the body through a vein for therapeutic purposes. Generally, this is achieved via an intravenous bag.

"Intracellular signaling domain" as used herein refers to all or a portion of one or more domains of a molecule (here the chimeric receptor molecule) that provides for activation of a lymphocyte. Intracellular domains of such molecules mediate a signal by interacting with cellular mediators to result in proliferation, differentiation, activation and other effector functions. Examples of intracellular signaling domains for use in a CAR of the invention include the intracellular sequences of the CD3ζ chain, and/or co-receptors that act in concert to initiate signal transduction following CAR engagement, as well as any derivative or variant of these sequences and any synthetic sequence that has the same functional capability. T cell activation can be said to be mediated by two distinct classes of cytoplasmic signaling sequence: those that initiate antigen-dependent primary activation and provide a T cell receptor like signal (primary cytoplasmic signaling sequences) and those that act in an antigen- independent manner to provide a secondary or co-stimulatory signal (secondary cytoplasmic signaling sequences). Primary cytoplasmic signaling sequences that act in a stimulatory manner may contain signaling motifs which are known as receptor tyrosine-based activation motifs or ITAMs. Examples of ITAM containing primary cytoplasmic signaling sequences include those derived from CD3ζ, FcRy, CD3γ, CD3δ, CD3ε, CD5, CD22, CD79a, CD79b, and CD66d.

The term "monobody" refers to a protein that is derived from a fibronectin type III domain that has been engineered to bind to a specific target (see Koide et al., 2013. J Mol Biol. 415(2):393-405).

The term "nanobody" refers to a protein comprising the soluble single antigen-binding V-domain of a heavy chain antibody, preferably a camelid heavy chain antibody (see Bannas et al., 2017. Front Immunol. 8:1603).

"Overall Survival" (OS) refers to the time from patient enrollment to death or censored at the date last known alive. OS includes a prolongation in life expectancy as compared to naive or untreated individuals or patients. Overall survival refers to the situation wherein a patient remains alive for a defined period of time, such as one year, five years, etc., e.g., from the time of diagnosis or treatment.

A "partial response" or "PR" refers to at least a 30% decrease in the sum of diameters of target lesions, taking as reference the baseline sum diameter, in response to treatment, as defined in the RECIST v1.1 guideline.

The term "peptide aptamer" refers to a short, 5-20 amino acid residue sequence that can bind to a specific target. Peptide aptamers are typically inserted within a loop region of a stable protein scaffold (see Reverdatto et al., 2015. Curr Top Med Chem. 15(12):1082-101).

As used herein, "pharmaceutically acceptable carrier" or "pharmaceutically acceptable diluent" means any and all solvents, dispersion media, coatings, antibacterial and antifungal agents, isotonic and absorption delaying agents, compatible with pharmaceutical administration. The use of such media and agents for pharmaceutically active substances is well known in the art. Acceptable carriers, excipients, or stabilizers are nontoxic to recipients at the dosages and concentrations employed and, without limiting the scope of the present invention, include: additional buffering agents; preservatives; co-solvents; antioxidants, including ascorbic acid and methionine; chelating agents such as EDTA; metal complexes (e.g., Zn-protein complexes); biodegradable polymers, such as polyesters; salt-forming counterions, such as sodium, polyhydric sugar alcohols; amino acids, such as alanine, glycine, glutamine, asparagine, histidine, arginine, lysine, ornithine, leucine, 2-phenylalanine, glutamic acid, and threonine; organic sugars or sugar alcohols, such as lactitol, stachyose, mannose, sorbose, xylose, ribose, ribitol, myoinisitose, myoinisitol, galactose, galactitol, glycerol, cyclitols (e.g., inositol), polyethylene glycol; sulfur containing reducing agents, such as glutathione, thioctic acid, sodium thioglycolate, thioglycerol, [alpha]-monothioglycerol, and sodium thiosulfate; low molecular weight proteins, such as human serum albumin, bovine serum albumin, gelatin, or other immunoglobulins; and hydrophilic polymers, such as polyvinylpyrrolidone. Other pharmaceutically acceptable carriers, excipients, or stabilizers, such as those described in Remington's Pharmaceutical Sciences 16th edition, Osol, A. Ed. (1980) may also be included in a pharmaceutical composition described herein, provided that they do not adversely affect the desired characteristics of the pharmaceutical composition.

"Progressive disease" or "disease that has progressed" refers to the appearance of one more new lesions or tumors and/or the unequivocal progression of existing non-target lesions as defined in the RECIST v1.1 guideline. Progressive disease or disease that has progressed can also refer to a tumor growth of more than 20 percent since treatment began, either due to an increase in mass or in spread of the tumor.

"Progression free survival" (PFS) refers to the time from enrollment to disease progression or death. PFS is generally measured using the Kaplan-Meier method and Response Evaluation Criteria in Solid Tumors (RECIST) 1.1 standards. Generally, progression free survival refers to the situation wherein a patient remains alive, without the cancer getting worse.

The term "RECIST" means Response Evaluation Criteria in Solid Tumours. RECIST guideline, criteria, or standard, describes a standard approach to solid tumor measurement and definitions for objective assessment of change in tumor size for use in adult and pediatric cancer clinical trials. RECIST v1.1 means version 1.1 of the revised RECIST guideline and it is published in European Journal of Cancers 45 (2009) 228-247.

The term "repebody" refers to a protein that is derived from a leucine-rich repeat module and that been engineered to bind to a specific target (see Lee et al., 2012. PNAS. 109(9): 3299-3304).

The term "respond favorably" generally refers to causing a beneficial state in a subject. With respect to cancer treatment, the term refers to providing a therapeutic effect on the subject. Positive therapeutic effects in cancer can be measured in a number of ways (See, Weber, 2009. J Nucl Med. 50 Suppl 1:1S-10S). For example, tumor growth inhibition, molecular marker expression, serum marker expression, and molecular imaging techniques can all be used to assess therapeutic efficacy of an anti-cancer therapeutic. A favorable response can be assessed, for example, by increased progression-free survival (PFS), disease-free survival (DFS), or overall survival (OS), complete response (CR), partial response (PR), or, in some cases, stable disease (SD), a decrease in progressive disease (PD), a reduced time to progression (TTP) or any combination thereof.

The term "sequence identity" refers to a percentage value obtained when two sequences are compared using a pairwise sequence alignment tool. In the present case, the sequence identity is obtained using the global alignment tool "EMBOSS Needle" using the default settings (Rice et al., 2000. Trends Genet. 16(6):276-7; Li et al., 2015. Nucleic Acids Res. 43(W1):W580-4). The global alignment tool is available at: https://www.ebi.ac.uk/Tools/psa/ .

The term "single-chain antigen-binding fragment" or "scFab" refers to a fusion protein comprising one variable and one constant domain of the light chain of an antibody attached to one variable and one constant domain of the heavy chain of an antibody, wherein the heavy and light chains are linked together through a short peptide.

The term "single-chain variable fragment" or "scFv" refers to a fusion protein comprising the variable domains of the heavy chain and light chain of an antibody linked to one another with a peptide linker. The term also includes a disulfide stabilized Fv (dsFv). Methods of stabilizing scFvs with disulfide bonds are disclosed in Reiter et al., 1996. Nat Biotechnol. 14(10):1239-45.

"Stable disease" refers to disease without progression or relapse as defined in the RECIST v1.1 guideline. In stable disease there is neither sufficient tumor shrinkage to qualify for partial response, nor sufficient tumor increase to qualify as progressive disease.

"Time to Tumor Progression" (TTP) is defined as the time from enrollment to disease progression. TTP is generally measured using the RECIST v1.1 criteria.

The terms "treatment" and "therapy", as used in the present application, refer to a set of hygienic, pharmacological, surgical and/or physical means used with the intent to cure and/or alleviate a disease and/or symptoms with the goal of remediating the health problem. The terms "treatment" and "therapy" include preventive and curative methods, since both are directed to the maintenance and/or reestablishment of the health of an individual or animal. Regardless of the origin of the symptoms, disease and disability, the administration of a suitable medicament to alleviate and/or cure a health problem should be interpreted as a form of treatment or therapy within the context of this application.

### Detailed description of the invention

In the present invention, three humanization approaches (named h1, h2 and h3) and a de-murinenization approach (h4) have been tested in order to develop new and optimized CD1a-targeting moieties with high cytotoxicity potential while being less immunogenic than their murine counterparts. After *in silico* and functional comparative analyses of murine and the different humanized or de-murenized scFvs (h1-h4), it was found that that h1 and h2 presented no liabilities and a higher stability than the h3 **(Example 3).** The de-murenized version h4 was discarded at the very beginning because it was not recognized by anti-human antibodies **(Example 4).** In addition, comparative functional analysis revealed that h3 was totally inefficient in its killing potential and for this reason was also discarded **(Example 4).** H2 showed higher killing potency than h1 or murine **(Example 4),** and the lowest immunogenicity compared to the rest of humanized versions **(Example 3).** For these reasons, h2 was finally selected for further experimentation and it was named h2CD1a-CAR or OC 1 (see summary of properties in **Table** 12).

Once the optimal humanization of the CAR-CD1a was achieved and the best candidate was chosen (h2CD1a-CART, h2), a first experiment to analyse the CAR expression and expansion of the h2CAR-CD1a (OC_1) was performed. As shown in **Example** 6, OC_1 scFv CAR-CD1a can be expressed in the membrane of transduced peripheral blood mononuclear cells (PBMCs) with similar expression and proportion of CD4⁺/CD8⁺ cells than mCAR-CD1a. Next, two studies were performed to evaluate the activity of OC_1 in **Example 7:** i) a first study to evaluate the product cytotoxicity, and ii) a second assay to determine the pro-inflammatory cytokines levels induced by the product. In summary, the results showed that i) h2CAR-CD1a strongly decreased cell viability at all the ratios analyzed in both Jurkat **(****Figure 7 A)** and MOLT4 **(****Figure 7 B)** cells, being h2CD1a-CART more potent than the murine version; in addition, the killing efficiency of OC_1 was stronger and evidenced at shorter times than the murine (see **Figure 8** **B,** at 2h) and at most of the E:T ratios analyzed **(****Figure 8** **B and 8 C);** and ii) OC_1 induces production of pro-inflammatory cytokines in Jurkat cells at the same level as the mCAR-CD1a.

Next, the *in vivo* potency and activity of the h2CAR-CD1a was also compared to that of mCAR-CD1a. In NSG animals, tumors develop very rapidly without treatment. **Figure 10** showed the *in vivo* activity of mCAR-CD1a and h2CAR-CD1a. Data showed that both mice groups (mCD1a-CAR-T and h2CD1a-CAR-T) were practically leukaemia free at day 17, in contrast to the mice receiving UT T cells, which showed massive tumour burden by BLI **(****Figure 10 A)****.** Quantification of BLI by IVIS showed an improvement effect of h2CAR-CD1a related to the murine version **(****Figure 10 B)****.** FACS of tumour burden in PB and BM confirmed all CD1a CAR-T cells efficiently eliminated Jurkat cells **(****Figure 10 C)** but the number of mice with less than 0,1% (selected as arbitrary cutoff) of circulating blast cells in PB was lower in those receiving h2CD1a-CAR-T (10 mice from 12) compared to mCD1a-CAR-T cells (5 mice from 12). These results demonstrate that h2CD1a-CAR-Ts exhibit potent antileukemic activity that was higher than to that of mCD1a-CAR-Ts **(****Figure 10B****).** Furthermore, the number of mice with more than 1% of CAR-T cells detected in the BM are significantly higher in those receiving h2CD1a-CAR-T (6 mice from 12) compared to mCD1a-CAR-T cells (2 mice from 12). Comparative data with h1 is also shown in **Example 4.** In addition to this, using a PDX in vivo model OC_1 is able to better abolish tumor progression (measured as tumor engraftment in bone marrow and peripheral blood) at lower doses compared to the murine version **(****Figure 11****).** All these data suggest that humanization of CAR-CD1a improves the capacity of CAR-Ts to reduce tumor burden and reduces the amount required to have a significant biological effect.

Additionally, off target and fratricide effect of the newly developed h2CD1a-CAR-T was assayed, and the results showed that h2CD1a-CAR-T cells are unable to affect cell viability of CD1a negative cells lines from different origins, and that humanization is not affecting the CAR-CD1a specificity **(Example 9,** **Figures 14-15****).** Further, h2CD1a-CAR-Ts are fratricide resistant **(Example 10,** **Figure 16****).**

To finish with, affinity assays were performed in order to understand the improved and unexpected increased cytotoxic of the h2CAR-CD1a developed in the present study. The results showed in **Example 10** are completely unpredicted as they show that the binding of rCD1a to h2CD1a-CAR-T cells is greatly improved in comparison to the binding of the murine counterpart **(****Figures 17-****18).** In fact, the method described here with the recombinant protein improves the detection of OC_1 used in the clinical trial (see **Figure 19****,** 58% with anti-scFv and 71 % with rCD1a).

Therefore, the present invention provides a humanized CD1a-targeting moiety that is not only superior in terms of being less immunogenic than its murine counterpart, but it also has a surprising increased cytotoxicity and affinity to rCD1a, which lead to tumor reduction in vivo efficacy assays.

In view of the above results, in a **first aspect,** the present invention provides a humanized CD1a targeting moiety comprising a VL domain and a VH domain, wherein said VL domain comprises LCDR1, LCDR2 and LCDR3 polypeptides and said VH domain comprises HCDR1, HCDR2 and HCDR3 polypeptides, and wherein:
- LCDR1 comprises, consists, or consists essentially of [QASQDINKYIA] **(SEQ ID NO: 4),** or a sequence with at least 90%, 91%, 92%, 93%, 94%, 95%, 96%, 97%, 98%, or 100% sequence identity to SEQ ID NO: 4;
- LCDR2 comprises, consists, or consists essentially of [IHYTSTL] **(SEQ ID NO: 5),** or a sequence with at least 90%, 91%, 92%, 93%, 94%, 95%, 96%, 97%, 98%, or 100% sequence identity to SEQ ID NO: 5;
- LCDR3 comprises, consists, or consists essentially of [LHYDNLPWT] **(SEQ ID NO: 6),** or a sequence with at least 90%, 91%, 92%, 93%, 94%, 95%, 96%, 97%, 98%, or 100% sequence identity to SEQ ID NO: 6;
- HCDR1 comprises, consists, or consists essentially of [SGYAFSTYTMH] **(SEQ ID NO: 7),** or a sequence with at least 90%, 91%, 92%, 93%, 94%, 95%, 96%, 97%, 98%, or 100% sequence identity to SEQ ID NO: 7;
- HCDR2 comprises, consists, or consists essentially of [YINPNSASTS] **(SEQ ID NO: 8),** or a sequence with at least 90%, 91%, 92%, 93%, 94%, 95%, 96%, 97%, 98%, or 100% sequence identity to SEQ ID NO: 8; and
- HCDR3 comprises, consists, or consists essentially of [ARGFYTMDY] **(SEQ ID NO: 9),** or a sequence with at least 90%, 91%, 92%, 93%, 94%, 95%, 96%, 97%, 98%, or 100% sequence identity to SEQ ID NO: 9.

In some embodiments, the CD1a-targeting moiety is an antibody, anticalin, repebody, monobody, scFv, Fab, scFab, affibody, fynomer, DARPin, nanobody, or peptide aptamer that specifically binds to CD1a.

In some embodiments, the CD1a-targeting moiety is an antibody, F(ab')2, Fab, preferably scFv, or scFab comprising a VL domain and VH domain, wherein said VL domain comprises LCDR1, LCDR2 and LCDR3 polypeptides and said VH domain comprises HCDR1, HCDR2 and HCDR3 polypeptides, and LCDR1 consists of [QASQDINKYIA] (SEQ ID NO: 4), LCDR2 consists of [IHYTSTL] (SEQ ID NO: 5), LCDR3 consists of [LHYDNLPWT] (SEQ ID NO: 6), HCDR1 consists of [SGYAFSTYTMH] (SEQ ID NO: 7), HCDR2 consists of [YINPNSASTS] (SEQ ID NO: 8), and HCDR3 consists of [ARGFYTMDY] (SEQ ID NO: 9).

Preferably, the humanized CD1a targeting moiety is an antibody, F(ab')2, Fab, preferably scFv, or scFab comprising a VL domain and a VH domain, wherein the VL domain comprises, consists, or consists essentially of SEQ ID NO: 1, or a sequence with at least 90%, 91%, 92%, 93%, 94%, 95%, 96%, 97%, 98%, or 100% sequence identity to SEQ ID NO: 1; and wherein the VH domain comprises, consists, or consists essentially of SEQ ID NO: 2, or a sequence with at least 90%, 91%, 92%, 93%, 94%, 95%, 96%, 97%, 98%, or 100% sequence identity to SEQ ID NO: 2.
**VL domain SEQ ID NO: 1:** 
RDIQMTQSPSSLSASVGDRVTITCQASQDINKYIAWYQFKPGKGPRLLIHYTSTLEPAIPSRFSG
SGSGREYTFTISSLQPEDIATYYCLHYDNLPWTFGGGTKVEIKRA
**VH domain SEQ ID NO:** 2:

QVQLQQSGAEVKKPGASVKVSCKASGYAFSTYTMHWVRQAPGQGLEWMGYINPNSASTSYA
QKFQGRVTMTADKSTNTAYMELSSLRSEDTAVYYCARGFYTMDYWGQGTLVTVSS

In some embodiments, the CD1a-targeting moiety is an antibody, scFv, Fab, or scFab comprising a VL domain and VH domain, wherein the VL domain consists of SEQ ID NO: 1 and the VH domain consists of SEQ ID NO: 2. In a preferred embodiment, the VL and the VH domains comprised in the CD1a-targeting moiety are linked by a peptide linker. In some embodiments, the linker comprises at least 5 amino acids, preferably between 5 and 25, preferably between 10-20 amino acids, most preferably 20 amino acids. Preferably, the amino acid is G. Preferably, the peptide linker comprises, consists or consists essentially of SEQ ID NO: 14, or a sequence that has 90%, 91%, 92%, 93%, 94%, 95%, 96%, 97%, 98%, or 100% sequence identity to SEQ ID NO: 14.

### Linker between heavy and light chains SEQ ID NO: 14: GGGGSGGGGSGGGGSGGGGS

In a preferred embodiment, the CD1a-targeting moiety further comprises a signal peptide that is placed preferably at the N-terminal region of the CD1a-targeting moiety. Preferably, the signal peptide comprises, consists or consists essentially of SEQ ID NO: 15, or a sequence that has 90%, 91%, 92%, 93%, 94%, 95%, 96%, 97%, 98%, or 100% sequence identity to SEQ ID NO: 15.

### Signal Peptide (SEQ ID NO: 15): MALPVTGLLLSLGLLLHAARPTG

Preferably, the CD1a targeting moiety comprises, consists, or consists essentially of SEQ ID NO: 3, or a sequence that has 90%, 91%, 92%, 93%, 94%, 95%, 96%, 97%, 98%, or 100% sequence identity to SEQ ID NO: 3. Most preferably, the CD1a targeting moiety consists of SEQ ID NO: 3.

### Humanized CD1a targeting moiety SEQ ID NO: 3:

QVQLQQSGAEVKKPGASVKVSCKASGYAFSTYTMHWVRQAPGQGLEWMGYINPNSASTSYA
QKFQGRVTMTADKSTNTAYMELSSLRSEDTAVYYCARGFYTMDYWGQGTLVTVSSGGGGSG
GGGSGGGGSGGGGSRDIQMTQSPSSLSASVGDRVTITCQASQDINKYIAWYQFKPGKGPRLLI
HYTSTLEPAIPSRFSGSGSGREYTFTISSLQPEDIATYYCLHYDNLPWTFGGGTKVEIKRA

In some embodiments, the humanized CD1a targeting moiety is a scFv comprising a VL domain and a VH domain, wherein said VL domain comprises LCDR1, LCDR2 and LCDR3 polypeptides and said VH domain comprises HCDR1, HCDR2 and HCDR3 polypeptides, and wherein:
- LCDR1 comprises, consists, or consists essentially of [QASQDINKYIA] **(SEQ ID NO: 4),** or a sequence with at least 90%, 91%, 92%, 93%, 94%, 95%, 96%, 97%, 98%, or 100% sequence identity to SEQ ID NO: 4;
- LCDR2 comprises, consists, or consists essentially of [IHYTSTL] **(SEQ ID NO: 5),** or a sequence with at least 90%, 91%, 92%, 93%, 94%, 95%, 96%, 97%, 98%, or 100% sequence identity to SEQ ID NO: 5;
- LCDR3 comprises, consists, or consists essentially of [LHYDNLPWT] **(SEQ ID NO: 6),** or a sequence with at least 90%, 91%, 92%, 93%, 94%, 95%, 96%, 97%, 98%, or 100% sequence identity to SEQ ID NO: 6;
- HCDR1 comprises, consists, or consists essentially of [SGYAFSTYTMH] **(SEQ ID NO: 7),** or a sequence with at least 90%, 91%, 92%, 93%, 94%, 95%, 96%, 97%, 98%, or 100% sequence identity to SEQ ID NO: 7;
- HCDR2 comprises, consists, or consists essentially of [YINPNSASTS] **(SEQ ID NO: 8),** or a sequence with at least 90%, 91%, 92%, 93%, 94%, 95%, 96%, 97%, 98%, or 100% sequence identity to SEQ ID NO: 8; and
- HCDR3 comprises, consists, or consists essentially of [ARGFYTMDY] **(SEQ ID NO: 9),** or a sequence with at least 90%, 91%, 92%, 93%, 94%, 95%, 96%, 97%, 98%, or 100% sequence identity to SEQ ID NO: 9.

In some embodiments, the CD1a-targeting moiety is a scFv comprising a VL domain and VH domain, wherein said VL domain comprises LCDR1, LCDR2 and LCDR3 polypeptides and said VH domain comprises HCDR1, HCDR2 and HCDR3 polypeptides, and LCDR1 consists of [QASQDINKYIA] (SEQ ID NO: 4), LCDR2 consists of [IHYTSTL] (SEQ ID NO: 5), LCDR3 consists of [LHYDNLPWT] (SEQ ID NO: 6), HCDR1 consists of [SGYAFSTYTMH] (SEQ ID NO: 7), HCDR2 consists of [YINPNSASTS] (SEQ ID NO: 8), and HCDR3 consists of [ARGFYTMDY] (SEQ ID NO: 9).

Preferably, the humanized CD1a targeting moiety is a scFv comprising a VL domain and a VH domain, wherein the VL domain comprises, consists, or consists essentially of SEQ ID NO: 1, or a sequence with at least 90%, 91%, 92%, 93%, 94%, 95%, 96%, 97%, 98%, or 100% sequence identity to SEQ ID NO: 1; and wherein the VH domain comprises, consists, or consists essentially of SEQ ID NO: 2, or a sequence with at least 90%, 91%, 92%, 93%, 94%, 95%, 96%, 97%, 98%, or 100% sequence identity to SEQ ID NO: 2. In some embodiments, the CD1a-targeting moiety is a scFv comprising a VL domain and VH domain, wherein the VL domain consists of SEQ ID NO: 1 and the VH domain consists of SEQ ID NO: 2.

Preferably, the CD1a targeting moiety is a scFv that comprises, consists, or consists essentially of SEQ ID NO: 3, or a sequence that has 90%, 91%, 92%, 93%, 94%, 95%, 96%, 97%, 98%, or 100% sequence identity to SEQ ID NO: 3. Most preferably, the CD1a targeting moiety is a scFv consists of SEQ ID NO: 3.

Binding molecules that bind specifically to CD1a may be very useful in the diagnosis and treatment of the disorders mentioned above. Several murine monoclonal antibodies against CD1a are known in the field. However, murine antibodies are limited for *in vivo* use due to issues associated with the administration of murine antibodies to humans, such as short serum half-life, the inability to trigger certain human effector functions and the generation of an undesired immune response against the murine antibody. New human antibodies have been developed in recent years overcoming these previously mentioned drawbacks. Besides NA1/34.HLK, other hybridomas are commercially available, e.g. OKT6 (IgG1 isotype), from SIGMA ALDRICH.

Phage display and combinatorial methods for generating antibodies are known in the art. Further, methods of generating and selecting non-immunoglobulin scaffolds that bind to a particular target are known in the art.

In an **alternative first aspect,** the present invention provides a humanized CD1a targeting moiety comprising a VL domain and a VH domain, wherein said VL domain comprises, consists, or consists essentially of SEQ ID NO: 17, or a sequence with at least 90%, 91%, 92%, 93%, 94%, 95%, 96%, 97%, 98%, or 100% sequence identity to SEQ ID NO: 17; and wherein the VH domain comprises, consists, or consists essentially of SEQ ID NO: 16, or a sequence with at least 90%, 91%, 92%, 93%, 94%, 95%, 96%, 97%, 98%, or 100% sequence identity to SEQ ID NO: 16.
Heavy chain (SEQ ID NO: 16) 
QVQLVQSGPEVAKPGASVRLSCKASGYAFSTYTMHWVRQAPGQGLEWMGYINPNSASTSYN
QNFKARVTLTADKSTNTAYMHLSSLTSDDTAVYYCGRGFYTMDYWGQGT LVTVSSA
Light chain (SEQ ID NO: 17)

DIQMTQSPSSVSASVGDRVTISCQASQDINKYIAWYQFKPGKGPRLLIHYTSTLQPAIPSRFSGS
GSGREYTLTISSLQPEDFATYYCLHYDNLPWTFGGGTKVEVRRA

In some embodiments, the CD1a-targeting moiety of the alternative first aspects is an antibody, scFv, Fab, or scFab comprising a VL domain and VH domain, wherein the VL domain consists of SEQ ID NO: 17 and the VH domain consists of SEQ ID NO: 16. Preferably, the humanized CD1a targeting moiety is a scFv.

In a preferred embodiment, the VL and the VH domains comprised in the CD1a-targeting moiety are linked by a peptide linker. The peptide linker is the same as previously defined. In a preferred embodiment, the CD1a-targeting moiety further comprises a signal peptide that is preferably placed at the N-terminal region of the CD1a-targeting moiety. The signal peptide is the same as previously defined.

Preferably, the **CD1a** targeting moiety comprises, consists, or consists essentially of SEQ ID NO: 22, or a sequence that has 90%, 91%, 92%, 93%, 94%, 95%, 96%, 97%, 98%, or 100% sequence identity to SEQ ID NO: 22. Most preferably, the CD1a targeting moiety consists of SEQ ID NO: 22. Preferably, the **CD1a** targeting moiety is a scFV.

### Humanized CD1a targeting moiety SEQ ID NO: 22:

QVQLVQSGPEVAKPGASVRLSCKASGYAFSTYTMHWVRQAPGQGLEWMGYINPNSASTSYN
QNFKARVTLTADKSTNTAYMHLSSLTSDDTAVYYCGRGFYTMDYWGQGT LVTVSSA
GGGGSGGGGSGGGGSGGGGSDIQMTQSPSSVSASVGDRVTISCQASQDINKYIAWYQFKPG
KGPRLLIHYTSTLQPAIPSRFSGSGSGREYTLTISSLQPEDFATYYCLHYDNLPWTFGGGTKVE
VRRA

The humanized CD1a targeting moieties as defined above (both in the first aspect and in the alternative first aspect) can be part of a chimeric antigen receptor. Thus, in a preferred embodiment of the first aspect, the present invention provides a chimeric antigen receptor (CAR) comprising:
a) an extracellular domain comprising a CD1a targeting moiety as defined in the first aspect or in any of the embodiments disclosed above,
b) a transmembrane domain; and
c) an intracellular signaling domain.

Each of the elements of the CAR according to this embodiment of the first aspect are further developed below:

### a) extracellular domain comprising a CD1a targeting moiety as defined in the first aspect or any of its embodiments.

In some embodiments, the CD1a-targeting moiety is a scFv comprising a VL domain and VH domain, wherein said VL domain comprises LCDR1, LCDR2 and LCDR3 polypeptides and said VH domain comprises HCDR1, HCDR2 and HCDR3 polypeptides, and wherein
- LCDR1 comprises, consists, or consists essentially of [QASQDINKYIA] (SEQ ID NO: 4), or a sequence with at least 90%, 91%, 92%, 93%, 94%, 95%, 96%, 97%, 98%, or 100% sequence identity to SEQ ID NO: 4;
- LCDR2 comprises, consists, or consists essentially of [IHYTSTL] (SEQ ID NO: 5), or a sequence with at least 90%, 91%, 92%, 93%, 94%, 95%, 96%, 97%, 98%, or 100% sequence identity to SEQ ID NO: 5;
- LCDR3 comprises, consists, or consists essentially of [LHYDNLPWT] (SEQ ID NO: 6), or a sequence with at least 90%, 91%, 92%, 93%, 94%, 95%, 96%, 97%, 98%, or 100% sequence identity to SEQ ID NO: 6;
- HCDR1 comprises, consists, or consists essentially of [SGYAFSTYTMH] (SEQ ID NO: 7), or a sequence with at least 90%, 91%, 92%, 93%, 94%, 95%, 96%, 97%, 98%, or 100% sequence identity to SEQ ID NO: 7;
- HCDR2 comprises, consists, or consists essentially of [YINPNSASTS] (SEQ ID NO: 8), or a sequence with at least 90%, 91%, 92%, 93%, 94%, 95%, 96%, 97%, 98%, or 100% sequence identity to SEQ ID NO: 8; and
- HCDR3 comprises, consists, or consists essentially of [ARGFYTMDY] (SEQ ID NO: 9), or a sequence with at least 90%, 91%, 92%, 93%, 94%, 95%, 96%, 97%, 98%, or 100% sequence identity to SEQ ID NO: 9.

In some embodiments, the CD1a-targeting moiety is a scFv comprising a VL domain and VH domain, wherein said VL domain comprises LCDR1, LCDR2 and LCDR3 polypeptides and said VH domain comprises HCDR1, HCDR2 and HCDR3 polypeptides, and LCDR1 consists of [QASQDINKYIA] (SEQ ID NO: 4), LCDR2 consists of [IHYTSTL] (SEQ ID NO: 5), LCDR3 consists of [LHYDNLPWT] (SEQ ID NO: 6), HCDR1 consists of [SGYAFSTYTMH] (SEQ ID NO: 7), HCDR2 consists of [YINPNSASTS] (SEQ ID NO: 8), and HCDR3 consists of [ARGFYTMDY] (SEQ ID NO: 9).

In an embodiment of the first aspect, the extracellular domain comprising a CD1a-targeting moiety is a scFv comprising a VL domain and a VH domain, wherein the VL domain comprises, consists, or consists essentially of SEQ ID NO: 1, or a sequence that has 90%, 91%, 92%, 93%, 94%, 95%, 96%, 97%, 98%, or 100% sequence identity to SEQ ID NO: 1; and wherein the VH domain comprises, consists, or consists essentially of SEQ ID NO: 2, or a sequence that has 90%, 91%, 92%, 93%, 94%, 95%, 96%, 97%, 98%, or 100% sequence identity to SEQ ID NO: 2.

Most preferably, the CD1a targeting moiety is a scFv that comprises, consists, or consists essentially of SEQ ID NO: 3, or a sequence that has 90%, 91%, 92%, 93%, 94%, 95%, 96%, 97%, 98%, or 100% sequence identity to SEQ ID NO: 3. Most preferably, the CD1a targeting moiety is a scFV that consists of SEQ ID NO: 3.

In an alternative embodiment of the first aspect, the extracellular domain comprising a CD1a-targeting moiety is a scFv comprising a VL domain and a VH domain, wherein the VL domain comprises, consists, or consists essentially of SEQ ID NO: 17, or a sequence with at least 90%, 91%, 92%, 93%, 94%, 95%, 96%, 97%, 98%, or 100% sequence identity to SEQ ID NO: 17; and wherein the VH domain comprises, consists, or consists essentially of SEQ ID NO: 16, or a sequence with at least 90%, 91%, 92%, 93%, 94%, 95%, 96%, 97%, 98%, or 100% sequence identity to SEQ ID NO: 16. Preferably, the humanized CD1a targeting moiety is a scFv. Preferably, the VL domain consists of SEQ ID NO: 17 and the VH domain consists of SEQ ID NO: 16.

In some embodiments, the CD1a targeting moiety is a scFv and it comprises, consists, or consists essentially of SEQ ID NO: 22, or a sequence that has 90%, 91%, 92%, 93%, 94%, 95%, 96%, 97%, 98%, or 100% sequence identity to SEQ ID NO: 22. Most preferably, the CD1a targeting moiety consists of SEQ ID NO: 22.

In a preferred embodiment, the VL and the VH domains comprised in the scFv are linked by a peptide linker. Preferably, the peptide linker comprises, consists or consists essentially of SEQ ID NO: 14, or a sequence that has 90%, 91%, 92%, 93%, 94%, 95%, 96%, 97%, 98%, or 100% sequence identity to SEQ ID NO: 14.

In a preferred embodiment, the CD1a-targeting moiety is a scFv and it further comprises a signal peptide that is placed at the N-terminal region of the CD1a-targeting moiety. Preferably, the signal peptide comprises, consists or consists essentially of SEQ ID NO: 15, or a sequence that has 90%, 91%, 92%, 93%, 94%, 95%, 96%, 97%, 98%, or 100% sequence identity to SEQ ID NO: 15.

### b) transmembrane domain

The transmembrane domain may be derived either from a natural or a synthetic source. When the source is natural, the domain may be derived from any membrane-bound or transmembrane protein. Transmembrane regions may comprise at least the transmembrane region(s) of the α-, β- or ζ- chain of CD28, CD3, CD45, CD4, CD8, CD9, CD16, CD22, CD33, CD37, CD64, CD80, CD86, CD134, CD137, or CD154.

A transmembrane domain may be synthetic or a variant of a naturally occurring transmembrane domain. In some embodiments, synthetic or variant transmembrane domains comprise predominantly hydrophobic residues such as leucine and valine.

In some embodiments, the transmembrane domain comprises the transmembrane domain of CD28, CD3, CD45, CD4, CD8, CD9, CD16, CD22, CD33, CD37, CD64, CD80, CD86, CD134, CD137, CD154, or a variant thereof, wherein the variant thereof has a 95% sequence identity.

In some embodiments, the transmembrane domain comprises the transmembrane domain of CD28, CD3, CD45, CD4, CD8, CD9, CD16, CD22, CD33, CD37, CD64, CD80, CD86, CD134, CD137, CD154, or a variant thereof, wherein the variant thereof has a 98% sequence identity.

In some embodiments, the transmembrane domain comprises the transmembrane domain of CD28, CD3, CD45, CD4, CD8, CD9, CD16, CD22, CD33, CD37, CD64, CD80, CD86, CD134, CD137, or CD154.

In some embodiments, the transmembrane domain comprises the transmembrane domain of CD8 or a variant thereof, wherein the variant thereof has a 95% sequence identity.

In some embodiments, the transmembrane domain comprises the transmembrane domain of CD8 or a variant thereof, wherein the variant thereof has a 98% sequence identity.

In some embodiments, the transmembrane domain comprises the transmembrane domain of CD8.

In some embodiments, the transmembrane domain comprises SEQ ID NO: 10 or a sequence that has 95% sequence identity to SEQ ID NO: 10.

In some embodiments, the transmembrane domain comprises SEQ ID NO: 10 or a sequence that has 98% sequence identity to SEQ ID NO: 10.

In some embodiments, the transmembrane domain comprises SEQ ID NO: 10. In some embodiments, the transmembrane domain consists of SEQ ID NO: 10.

### Transmembrane domain derived from CD8 (SEQ ID NO: 10)

TTTPAPRPPTPAPTIASQPLSLRPEACRPAAGGAVHTRGLDFACDIYIWAPLAGTCGVLLLSLVI
TLYC

### c) Intracellular signaling domain

The intracellular signaling domain provides for the activation of at least one function of the cell expressing the CAR upon binding to the ligand expressed on tumor cells. In some embodiments, the intracellular signaling domain contains one or more intracellular signaling domains. In some embodiments, the intracellular signaling domain is a portion of and/or a variant of an intracellular signaling domain that provides for activation of at least one function of the CAR-comprising cell.

In some embodiments, the intracellular signaling domain comprises the intracellular domain of CD3ζ, FcRy, CD3y, CD3δ, CD3ε, CD5, CD22, CD79a, CD79b, CD66b, or a variant thereof, wherein the variant thereof has a 95% sequence identity.

In some embodiments, the intracellular signaling domain comprises the intracellular domain of CD3ζ, FcRy, CD3y, CD3δ, CD3ε, CD5, CD22, CD79a, CD79b, CD66b, or a variant thereof, wherein the variant thereof has a 98% sequence identity.

In some embodiments, the intracellular signaling domain comprises the intracellular domain of CD3ζ, FcRy, CD3γ, CD3δ, CD3ε, CD5, CD22, CD79a, CD79b or CD66b.

In some embodiments, the intracellular signaling domain comprises the intracellular domain of CD3ζ or a variant thereof, wherein the variant thereof has a 95% sequence identity.

In some embodiments, the intracellular signaling domain comprises the intracellular domain of CD3ζ or a variant thereof, wherein the variant thereof has a 98% sequence identity.

In some embodiments, the intracellular signaling domain comprises the intracellular domain of CD3ζ.

In some embodiments, the intracellular signaling domain comprises SEQ ID NO: 11 or a sequence that has 95% sequence identity to SEQ ID NO: 11.

In some embodiments, the intracellular signaling domain comprises SEQ ID NO: 11 or a sequence that has 98% sequence identity to SEQ ID NO: 11.

In some embodiments, the intracellular signaling domain comprises SEQ ID NO: 11 or a sequence that has 99% sequence identity to SEQ ID NO: 11.

In some embodiments, the intracellular signaling domain comprises SEQ ID NO: 11. In some embodiments, the intracellular signaling domain consists of SEQ ID NO: 11.

### Intracellular signaling domain derived from CD3ζ (SEQ ID NO: 11)

RVKFSRSADAPAYQQGQNQLYNELNLGRREEYDVLDKRRGRDPEMGGKPQRRKNPQEGLY
NELQKDKMAEAYSEIGMKGERRRGKGHDGLYQGLSTATKDTYDALHMQALPPR

Optionally, at least a costimulatory signaling domain may also be present in the CAR according to the first aspect or any of its embodiments:

### Costimulatory signaling domain

In some embodiments, the CAR may further comprise a costimulatory signaling domain. In some embodiments, the costimulatory signaling domain comprises the intracellular domain of CD27, CD28, CD137, CD134, CD30, CD40, lymphocyte function-associated antigen-1 (LFA-1), CD2, CD7, LIGHT, NKG2C, CD276 or a variant thereof, wherein the variant thereof has a 95% sequence identity.

In some embodiments, the costimulatory signaling domain comprises the intracellular domain of CD27, CD28, CD137, CD134, CD30, CD40, lymphocyte function-associated antigen-1 (LFA-1), CD2, CD7, LIGHT, NKG2C, CD276 or a variant thereof, wherein the variant thereof has a 98% sequence identity.

In some embodiments, the costimulatory signaling domain comprises the intracellular domain of CD27, CD28, CD137, CD134, CD30, CD40, lymphocyte function-associated antigen-1 (LFA-1), CD2, CD7, LIGHT, NKG2C, or CD276.

In some embodiments, the costimulatory signaling domain comprises the intracellular domain of CD137 or a variant thereof, wherein the variant thereof has a 95% sequence identity.

In some embodiments, the costimulatory signaling domain comprises the intracellular domain of CD137 or a variant thereof, wherein the variant thereof has a 98% sequence identity.

In some embodiments, the costimulatory signaling domain comprises the intracellular domain of CD137.

In some embodiments, the costimulatory signaling domain comprises SEQ ID NO: 12 or a sequence that has 95% sequence identity to SEQ ID NO: 12.

In some embodiments, the costimulatory signaling domain comprises SEQ ID NO: 12 or a sequence that has 98% sequence identity to SEQ ID NO: 12.

In some embodiments, the costimulatory signaling domain comprises SEQ ID NO: 12 or a sequence that has 99% sequence identity to SEQ ID NO: 12.

In some embodiments, the costimulatory signaling domain comprises SEQ ID NO: 12. In some embodiments, the costimulatory signaling domain consists of SEQ ID NO: 12.

### Costimulatory signaling domain derived from CD137 (SEQ ID NO: 12)

### KRGRKKLLYIFKQPFMRPVQTTQEEDGCSCRFPEEEEGGCEL

### Full sequence CARs according to the first aspect of the present invention

In some embodiments, the CAR comprises:
(i) a scFv comprising a VL domain and VH domain, wherein said VL domain comprises LCDR1, LCDR2 and LCDR3 polypeptides and said VH domain comprises HCDR1, HCDR2 and HCDR3 polypeptides, and LCDR1 consists of [QASQDINKYIA] (SEQ ID NO: 4), LCDR2 consists of [IHYTSTL] (SEQ ID NO: 5), LCDR3 consists of [LHYDNLPWT] (SEQ ID NO: 6), HCDR1 consists of [SGYAFSTYTMH] (SEQ ID NO: 7), HCDR2 consists of [YINPNSASTS] (SEQ ID NO: 8), and HCDR3 consists of [ARGFYTMDY] (SEQ ID NO: 9);
(ii) a transmembrane domain comprising SEQ ID NO: 10 or a sequence that has 95% sequence identity to SEQ ID NO: 10;
(iii) an intracellular signaling domain comprising SEQ ID NO: 11 or a sequence that has 95% sequence identity to SEQ ID NO: 11; and
(iv) a costimulatory signaling domain comprising SEQ ID NO: 12 or a sequence that has 95% sequence identity to SEQ ID NO: 12.

In some embodiments, the CAR comprises:
(i) a scFv comprising a VL domain and VH domain, wherein said VL domain comprises LCDR1, LCDR2 and LCDR3 polypeptides and said VH domain comprises HCDR1, HCDR2 and HCDR3 polypeptides, and LCDR1 consists of [QASQDINKYIA] (SEQ ID NO: 4), LCDR2 consists of [IHYTSTL] (SEQ ID NO: 5), LCDR3 consists of [LHYDNLPWT] (SEQ ID NO: 6), HCDR1 consists of [SGYAFSTYTMH] (SEQ ID NO: 7), HCDR2 consists of [YINPNSASTS] (SEQ ID NO: 8), and HCDR3 consists of [ARGFYTMDY] (SEQ ID NO: 9);
(ii) a transmembrane domain comprising SEQ ID NO: 10 or a sequence that has 98% sequence identity to SEQ ID NO: 10;
(iii) an intracellular signaling domain comprising SEQ ID NO: 11 or a sequence that has 98% sequence identity to SEQ ID NO: 11; and
(iv) a costimulatory signaling domain comprising SEQ ID NO: 12 or a sequence that has 98% sequence identity to SEQ ID NO: 12.

In some embodiments, the CAR comprises:
(i) a scFv comprising a VL domain and VH domain, wherein said VL domain comprises LCDR1, LCDR2 and LCDR3 polypeptides and said VH domain comprises HCDR1, HCDR2 and HCDR3 polypeptides, and LCDR1 consists of [QASQDINKYIA] (SEQ ID NO: 4), LCDR2 consists of [IHYTSTL] (SEQ ID NO: 5), LCDR3 consists of [LHYDNLPWT] (SEQ ID NO: 6), HCDR1 consists of [SGYAFSTYTMH] (SEQ ID NO: 7), HCDR2 consists of [YINPNSASTS] (SEQ ID NO: 8), and HCDR3 consists of [ARGFYTMDY] (SEQ ID NO: 9);
(ii) a transmembrane domain comprising SEQ ID NO: 10 or a sequence that has 98% sequence identity to SEQ ID NO: 10;
(iii) an intracellular signaling domain comprising SEQ ID NO: 11 or a sequence that has 99% sequence identity to SEQ ID NO: 11; and
(iv) a costimulatory signaling domain comprising SEQ ID NO: 12 or a sequence that has 99% sequence identity to SEQ ID NO: 12.

In some embodiments, the CAR comprises:
(i) a scFv comprising a VL domain and VH domain, wherein said VL domain comprises LCDR1, LCDR2 and LCDR3 polypeptides and said VH domain comprises HCDR1, HCDR2 and HCDR3 polypeptides, and LCDR1 consists of [QASQDINKYIA] (SEQ ID NO: 4), LCDR2 consists of [IHYTSTL] (SEQ ID NO: 5), LCDR3 consists of [LHYDNLPWT] (SEQ ID NO: 6), HCDR1 consists of [SGYAFSTYTMH] (SEQ ID NO: 7), HCDR2 consists of [YINPNSASTS] (SEQ ID NO: 8), and HCDR3 consists of [ARGFYTMDY] (SEQ ID NO: 9);
(ii) a transmembrane domain comprising SEQ ID NO: 10;
(iii) an intracellular signaling domain comprising SEQ ID NO: 11; and
(iv) a costimulatory signaling domain comprising SEQ ID NO: 12.

In some embodiments, the CAR comprises:
(i) a scFv comprising a VL domain and VH domain, wherein said VL domain comprises LCDR1, LCDR2 and LCDR3 polypeptides and said VH domain comprises HCDR1, HCDR2 and HCDR3 polypeptides, and LCDR1 consists of [QASQDINKYIA] (SEQ ID NO: 4), LCDR2 consists of [IHYTSTL] (SEQ ID NO: 5), LCDR3 consists of [LHYDNLPWT] (SEQ ID NO: 6), HCDR1 consists of [SGYAFSTYTMH] (SEQ ID NO: 7), HCDR2 consists of [YINPNSASTS] (SEQ ID NO: 8), and HCDR3 consists of [ARGFYTMDY] (SEQ ID NO: 9);
(ii) a transmembrane domain consisting of SEQ ID NO: 10;
(iii) an intracellular signaling domain consisting of SEQ ID NO: 11; and
(iv) a costimulatory signaling domain consisting of SEQ ID NO: 12.

In some embodiments, the CAR comprises:
(i) a scFv comprising a VL domain and VH domain, wherein the VL domain consists of SEQ ID NO: 1 and the VH domain consists of SEQ ID NO: 2;
(ii) a transmembrane domain comprising SEQ ID NO: 10 or a sequence that has 95% sequence identity to SEQ ID NO: 10;
(iii) an intracellular signaling domain comprising SEQ ID NO: 11 or a sequence that has 95% sequence identity to SEQ ID NO: 11; and
(iv) a costimulatory signaling domain comprising SEQ ID NO: 12 or a sequence that has 95% sequence identity to SEQ ID NO: 12.

In some embodiments, the CAR comprises:
(i) a scFv comprising a VL domain and VH domain, wherein the VL domain consists of SEQ ID NO: 17 and the VH domain consists of SEQ ID NO: 16;
(ii) a transmembrane domain comprising SEQ ID NO: 10 or a sequence that has 95% sequence identity to SEQ ID NO: 10;
(iii) an intracellular signaling domain comprising SEQ ID NO: 11 or a sequence that has 95% sequence identity to SEQ ID NO: 11; and
(iv) a costimulatory signaling domain comprising SEQ ID NO: 12 or a sequence that has 95% sequence identity to SEQ ID NO: 12.

In some embodiments, the CAR comprises:
(i) a scFv comprising a VL domain and VH domain, wherein the VL domain consists of SEQ ID NO: 1 and the VH domain consists of SEQ ID NO: 2;
(ii) a transmembrane domain comprising SEQ ID NO: 10 or a sequence that has 98% sequence identity to SEQ ID NO: 10;
(iii) an intracellular signaling domain comprising SEQ ID NO: 11 or a sequence that has 98% sequence identity to SEQ ID NO: 11; and
(iv) a costimulatory signaling domain comprising SEQ ID NO: 12 or a sequence that has 98% sequence identity to SEQ ID NO: 12.

In some embodiments, the CAR comprises:
(i) a scFv comprising a VL domain and VH domain, wherein the VL domain consists of SEQ ID NO: 17 and the VH domain consists of SEQ ID NO: 16;
(ii) a transmembrane domain comprising SEQ ID NO: 10 or a sequence that has 98% sequence identity to SEQ ID NO: 10;
(iii) an intracellular signaling domain comprising SEQ ID NO: 11 or a sequence that has 98% sequence identity to SEQ ID NO: 11; and
(iv) a costimulatory signaling domain comprising SEQ ID NO: 12 or a sequence that has 98% sequence identity to SEQ ID NO: 12.

In some embodiments, the CAR comprises:
(i) a scFv comprising a VL domain and VH domain, wherein the VL domain consists of SEQ ID NO: 1 and the VH domain consists of SEQ ID NO: 2;
(ii) a transmembrane domain comprising SEQ ID NO: 10 or a sequence that has 98% sequence identity to SEQ ID NO: 10;
(iii) an intracellular signaling domain comprising SEQ ID NO: 11 or a sequence that has 99% sequence identity to SEQ ID NO: 11; and
(iv) a costimulatory signaling domain comprising SEQ ID NO: 12 or a sequence that has 99% sequence identity to SEQ ID NO: 12.

In some embodiments, the CAR comprises:
(i) a scFv comprising a VL domain and VH domain, wherein the VL domain consists of SEQ ID NO: 17 and the VH domain consists of SEQ ID NO: 16;
(ii) a transmembrane domain comprising SEQ ID NO: 10 or a sequence that has 98% sequence identity to SEQ ID NO: 10;
(iii) an intracellular signaling domain comprising SEQ ID NO: 11 or a sequence that has 99% sequence identity to SEQ ID NO: 11; and
(iv) a costimulatory signaling domain comprising SEQ ID NO: 12 or a sequence that has 99% sequence identity to SEQ ID NO: 12.

In some embodiments, the CAR comprises:
(i) a scFv comprising a VL domain and VH domain, wherein the VL domain consists of SEQ ID NO: 1 and the VH domain consists of SEQ ID NO: 2;
(ii) a transmembrane domain comprising SEQ ID NO: 10;
(iii) an intracellular signaling domain comprising SEQ ID NO: 11; and
(iv) a costimulatory signaling domain comprising SEQ ID NO: 12.

In some embodiments, the CAR comprises:
(i) a scFv comprising a VL domain and VH domain, wherein the VL domain consists of SEQ ID NO: 17 and the VH domain consists of SEQ ID NO: 16;
(ii) a transmembrane domain comprising SEQ ID NO: 10;
(iii) an intracellular signaling domain comprising SEQ ID NO: 11; and
(iv) a costimulatory signaling domain comprising SEQ ID NO: 12.

In some embodiments, the CAR comprises:
(i) a scFv comprising a VL domain and VH domain, wherein the VL domain consists of SEQ ID NO: 1 and the VH domain consists of SEQ ID NO: 2;
(ii) a transmembrane domain consisting of SEQ ID NO: 10;
(iii) an intracellular signaling domain consisting of SEQ ID NO: 11; and
(iv) a costimulatory signaling domain consisting of SEQ ID NO: 12.

In some embodiments, the CAR comprises:
(i) a scFv comprising a VL domain and VH domain, wherein the VL domain consists of SEQ ID NO: 17 and the VH domain consists of SEQ ID NO: 16;
(ii) a transmembrane domain consisting of SEQ ID NO: 10;
(iii) an intracellular signaling domain consisting of SEQ ID NO: 11; and
(iv) a costimulatory signaling domain consisting of SEQ ID NO: 12.

In some embodiments, the CAR comprises:
(i) a scFv consisting of SEQ ID NO: 3;
(ii) a transmembrane domain comprising SEQ ID NO: 10 or a sequence that has 95% sequence identity to SEQ ID NO: 10;
(iii) an intracellular signaling domain comprising SEQ ID NO: 11 or a sequence that has 95% sequence identity to SEQ ID NO: 11; and
(iv) a costimulatory signaling domain comprising SEQ ID NO: 12 or a sequence that has 95% sequence identity to SEQ ID NO: 12.

In some embodiments, the CAR comprises:
(i) a scFv consisting of SEQ ID NO: 3;
(ii) a transmembrane domain comprising SEQ ID NO: 10 or a sequence that has 98% sequence identity to SEQ ID NO: 10;
(iii) an intracellular signaling domain comprising SEQ ID NO: 11 or a sequence that has 98% sequence identity to SEQ ID NO: 11; and
(iv) a costimulatory signaling domain comprising SEQ ID NO: 12 or a sequence that has 98% sequence identity to SEQ ID NO: 12.

In some embodiments, the CAR comprises:
(i) a scFv consisting of SEQ ID NO: 3;
(ii) a transmembrane domain comprising SEQ ID NO: 10 or a sequence that has 98% sequence identity to SEQ ID NO: 10;
(iii) an intracellular signaling domain comprising SEQ ID NO: 11 or a sequence that has 99% sequence identity to SEQ ID NO: 11; and
(iv) a costimulatory signaling domain comprising SEQ ID NO: 12 or a sequence that has 99% sequence identity to SEQ ID NO: 12.

In some embodiments, the CAR comprises:
(i) a scFv consisting of SEQ ID NO: 3;
(ii) a transmembrane domain comprising SEQ ID NO: 10;
(iii) an intracellular signaling domain comprising SEQ ID NO: 11; and
(iv) a costimulatory signaling domain comprising SEQ ID NO: 12.

In some embodiments, the CAR comprises:
(i) a scFv consisting of SEQ ID NO: 3;
(ii) a transmembrane domain consisting of SEQ ID NO: 10;
(iii) an intracellular signaling domain consisting of SEQ ID NO: 11; and
(iv) a costimulatory signaling domain consisting of SEQ ID NO: 12.

In some embodiments, the CAR comprises:
(v) a scFv consisting of SEQ ID NO: 22;
(vi) a transmembrane domain comprising SEQ ID NO: 10 or a sequence that has 95% sequence identity to SEQ ID NO: 10;
(vii) an intracellular signaling domain comprising SEQ ID NO: 11 or a sequence that has 95% sequence identity to SEQ ID NO: 11; and
(viii) a costimulatory signaling domain comprising SEQ ID NO: 12 or a sequence that has 95% sequence identity to SEQ ID NO: 12.

In some embodiments, the CAR comprises:
(v) a scFv consisting of SEQ ID NO: 22;
(vi) a transmembrane domain comprising SEQ ID NO: 10 or a sequence that has 98% sequence identity to SEQ ID NO: 10;
(vii) an intracellular signaling domain comprising SEQ ID NO: 11 or a sequence that has 98% sequence identity to SEQ ID NO: 11; and
(viii) a costimulatory signaling domain comprising SEQ ID NO: 12 or a sequence that has 98% sequence identity to SEQ ID NO: 12.

In some embodiments, the CAR comprises:
(v) a scFv consisting of SEQ ID NO: 22;
(vi) a transmembrane domain comprising SEQ ID NO: 10 or a sequence that has 98% sequence identity to SEQ ID NO: 10;
(vii) an intracellular signaling domain comprising SEQ ID NO: 11 or a sequence that has 99% sequence identity to SEQ ID NO: 11; and
(viii) a costimulatory signaling domain comprising SEQ ID NO: 12 or a sequence that has 99% sequence identity to SEQ ID NO: 12.

In some embodiments, the CAR comprises:
(v) a scFv consisting of SEQ ID NO: 22;
(vi) a transmembrane domain comprising SEQ ID NO: 10;
(vii) an intracellular signaling domain comprising SEQ ID NO: 11; and
(viii) a costimulatory signaling domain comprising SEQ ID NO: 12.

In some embodiments, the CAR comprises:
(v) a scFv consisting of SEQ ID NO: 22;
(vi) a transmembrane domain consisting of SEQ ID NO: 10;
(vii) an intracellular signaling domain consisting of SEQ ID NO: 11; and
(viii) a costimulatory signaling domain consisting of SEQ ID NO: 12.

In some embodiments, the CAR comprises or consists of SEQ ID NO: 13 or a sequence that has 95% sequence identity with SEQ ID NO: 13. In some embodiments, the CAR comprises or consists of SEQ ID NO: 13 or a sequence that has 98% sequence identity with SEQ ID NO: 13. In some embodiments, the CAR comprises or consists of SEQ ID NO: 13 or a sequence that has 99% sequence identity with SEQ ID NO: 13. In some embodiments, the CAR comprises or consists of SEQ ID NO: 13.

In some embodiments, the CAR comprises or consists of SEQ ID NO: 23 or a sequence that has 95% sequence identity with SEQ ID NO: 23. In some embodiments, the CAR comprises or consists of SEQ ID NO: 23 or a sequence that has 98% sequence identity with SEQ ID NO: 23. In some embodiments, the CAR comprises or consists of SEQ ID NO: 13 or a sequence that has 99% sequence identity with SEQ ID NO: 23. In some embodiments, the CAR comprises or consists of SEQ ID NO: 23.
Full sequence of the CAR (SEQ ID NO: 13) 
MALPVTGLLLSLGLLLHAARPTGQVQLQQSGAEVKKPGASVKVSCKASGYAFSTYTMHWVRQ
APGQGLEWMGYINPNSASTSYAQKFQGRVTMTADKSTNTAYMELSSLRSEDTAVYYCARGFY
TMDYWGQGTLVTVSSGGGGSGGGGSGGGGSGGGGSRDIQMTQSPSSLSASVGDRVTITCQ
ASQDINKYIAWYQFKPGKGPRLLIHYTSTLEPAIPSRFSGSGSGREYTFTISSLQPEDIATYYCLH
YDNLPWTFGGGTKVEIKRATTTPAPRPPTPAPTIASQPLSLRPEACRPAAGGAVHTRGLDFAC
DIYIWAPLAGTCGVLLLSLVITLYCKRGRKKLLYIFKQPFMRPVQTTQEEDGCSCRFPEEEEGG
CELRVKFSRSADAPAYQQGQNQLYNELNLGRREEYDVLDKRRGRDPEMGGKPQRRKNPQE
GLYNELQKDKMAEAYSEIGMKGERRRGKGHDGLYQGLSTATKDTYDALHMQALPPR
Full sequence of the CAR (SEQ ID NO: 23)

MALPVTGLLLSLGLLLHAARPTGQVQLVQSGPEVAKPGASVRLSCKASGYAFSTYTMHWVRQ
APGQGLEWMGYINPNSASTSYNQNFKARVTLTADKSTNTAYMHLSSLTSDDTAVYYCGRGFY
TMDYWGQGTLVTVSSAGGGGSGGGGSGGGGSGGGGSDIQMTQSPSSVSASVGDRVTISCQ
ASQDINKYIAWYQFKPGKGPRLLIHYTSTLQPAIPSRFSGSGSGREYTLTISSLQPEDFATYYCL
HYDNLPWTFGGGTKVEVRRATTTPAPRPPTPAPTIASQPLSLRPEACRPAAGGAVHTRGLDFA
CDIYIWAPLAGTCGVLLLSLVITLYCKRGRKKLLYIFKQPFMRPVQTTQEEDGCSCRFPEEEEG
GCELRVKFSRSADAPAYQQGQNQLYNELNLGRREEYDVLDKRRGRDPEMGGKPQRRKNPQ
EGLYNELQKDKMAEAYSEIGMKGERRRGKGHDGLYQGLSTATKDTYDALHMQALPPR

### Nucleic acid

In a second aspect, the present invention provides a nucleic acid encoding any one of the CD1a-targeting moiety of the present invention, including any one of the CARs disclosed above. The nucleic acid sequence that encodes the chimeric receptor links together a number of modular components that can be excised and replaced with other components in order to customize the chimeric receptor for efficient T cell activation and recognition of CD1a.

In some embodiments, the nucleic acid is suitable for transducing or transforming a cell. In some embodiments, the nucleic acid is suitable for transducing or transforming a T cell for use in adoptive immunotherapy.

In some embodiments, the nucleic acid is codon optimized for expression in mammalian cells. Codon optimization methods are known in the art.

The nucleic acid of the present invention may be comprised in a γ-retroviral or lentiviral vector which can be used to transduce or transform a T cell. The nucleic acid may also be inserted into a cell through the use of DNA transposons, RNA transfection or genome editing techniques such as TALEN, ZFN and CRISPR/Cas9.

### Cells

In a third aspect, the present invention provides a cell comprising the nucleic acid of the present invention and/or the CAR of the present invention. In some embodiments, the cell is a T-cell (referred to as a CART).

In some embodiments, the cell is a naïve T cell, memory stem T cell or central memory T cell. It is currently thought that these cells are better suited for adaptive immunotherapy.

In some embodiments, the cell is an autologous T cell. The term "autologous cell" refers to a cell obtained from the same patient that is to be treated using any one of the methods of the present invention. It is noted that flow cytometric analysis of peripheral blood obtained from 40 patients with active T-cell acute lymphoblastic leukemia revealed the presence of normal CD3+CD1a- T-cells in all the patients. Thus, it is entirely possible to treat a patient using an autologous T cell comprising the nucleic acid and/or CAR of the present invention.

In some embodiments, the cell is an allo-tolerant T cell. The term "all-tolerant cell" refers to a cell that has been engineered to decrease the risk of a Graft-versus-host disease response. In some embodiments, this is achieved by genomic editing-mediated deletion of TCR and/or β2-microglobulin. Allo-tolerant cells are known in the art (see section of allogeneic T cells in Rivière & Sadelain, 2017. Mol Ther. 25(5):1117-1124).

In some embodiments, the T cell is a CD3-positive and CD1a-negative T cell.

In some embodiments, the cell is a lymphoid precursor, embryonic stem cell or an induced pluripotent stem cell with the capacity to differentiate into a mature T cell.

### Pharmaceutical composition

In **a fourth** aspect, the present invention provides a pharmaceutical composition comprising a plurality of cells of the present invention and a pharmaceutically acceptable carrier or diluent.

A pharmaceutical composition as described herein may also contain other substances. These substances include, but are not limited to, cryoprotectants, surfactants, anti-oxidants, and stabilizing agents. The term "cryoprotectant" as used herein, includes agents which provide stability to the CARTs against freezing-induced stresses. Non-limiting examples of cryoprotectants include sugars, such as sucrose, glucose, trehalose, mannitol, mannose, and lactose; polymers, such as dextran, hydroxyethyl starch and polyethylene glycol; surfactants, such as polysorbates (e.g., PS-20 or PS-80); and amino acids, such as glycine, arginine, leucine, and serine. A cryoprotectant exhibiting low toxicity in biological systems is generally used.

In some embodiments, the cells are formulated by first harvesting them from their culture medium, and then washing and concentrating the cells in a medium and container system suitable for administration (a "pharmaceutically acceptable" carrier) in a therapeutically effective amount. Suitable infusion medium can be any isotonic medium formulation, typically normal saline, Normosol R (Abbott) or Plasma-Lyte A (Baxter), but also 5% dextrose in water or Ringer's lactate can be utilized. The infusion medium can be supplemented with human serum albumin, fetal bovine serum or other human serum components.

In one aspect, the present invention provides a cell according to the present invention or a pharmaceutical composition according to the present invention for use as a medicament.

### Methods of treatment

In a **fifth** aspect, the present invention provides a method of treating a CD1a-positive cancer comprising administering the cell of the present invention or the pharmaceutical composition of the present invention to a patient in need thereof.

In some embodiments, the patient is administered a therapeutically effective amount of cells. In some embodiments, the patient is administered at least 10², 10³, 10⁴, 10⁵, 10⁶, 10⁷, 10⁸, 10⁹ or 10¹⁰ cells. The number of cells will depend upon the ultimate use for which the composition is intended as will the type of cells included therein. For example, if cells that are specific for a particular antigen are desired, then the population will contain greater than 70%, generally greater than 80%, 85% and 90-95% of such cells. For uses provided herein, the cells are generally in a volume of a liter or less, can be 500 ml or less, even 250 ml or less, or 100 ml or less. The clinically relevant number of cells can be apportioned into multiple infusions that cumulatively equal or exceed 10², 10³, 10⁴, 10⁵, 10⁶, 10⁷, 10⁸, 10⁹ or 10¹⁰ cells.

In some embodiments, the cell or pharmaceutical composition is administered intravenously, intraperitoneally, into the bone marrow, into the lymph node, and /or into cerebrospinal fluid.

In some embodiments, the method comprises a combination therapy. In some embodiments, the method comprises further administering an immune checkpoint. In a further embodiment, the method comprises further administering an immune checkpoint inhibitor and/or an IAP inhibitor (see WO 2016/054555).

In some embodiments, the cell or pharmaceutical composition as described herein is administered in combination with chemotherapeutic agents and/or immunosuppressants. In an embodiment, a patient is first treated with a chemotherapeutic agent that inhibits or destroys other immune cells followed by the cell or pharmaceutical composition described herein. In some cases, chemotherapy may be avoided entirely.

In some embodiments, the CD1a-positive cancer is cortical T-cell acute lymphoblastic leukemia or Langerhans cell histiocytosis. In some embodiments, the CD1a-positive cancer is cortical T-cell acute lymphoblastic leukemia. In some embodiments, the CD1a-positive cancer is relapsed/refractory cortical T-cell acute lymphoblastic leukemia.

In general, the relapse of leukemia can manifest several months or years after the initial remission; however, most relapses occur within two years after the initial treatment. Refractoriness is a term that implies that the patient has no longer responded to at least one therapy strategy after a relapse.

There is a broad consensus in first-line trials for ALL, specifically in adults that a relapse is defined as "detection of more than 5% of blast cells in the bone marrow after a previous achievement of complete remission (CR) or unequivocal demonstration of extramedullary leukemia participation" (see Gökbuget (2017)). The European Working Group on Adult *ALL* (EWALL) has documented this statement in a consensus recommendation, (see Dohner (2010)) with the additional explanation that "in the case of 5 to 20% of cell blasts at some stage during the intensive treatment phase and / or during regeneration, the evaluation of the bone marrow should be repeated one week later to distinguish among bone marrow relapse and regeneration phenomenon". The cited definition is based on international recommendations for outcome parameters in acute myeloid leukemia (see Cheson (2003) and Chantepie (213)); that has been extrapolated to several subtypes of ALL, as in the case of T-ALL.

More recently, some trials did not even define the concept of relapse. Therefore, studies with chimeric antigen receptor (CAR) T cells included patients with "measurable disease" and also included patients with haematological relapse (no additional specification) or minimal residual disease (MRE) (see Lee (2015) and Maude (2014) and Gökbuget (2017)).

In some embodiments, the patient to be treated with the method of the present invention is in complete or near-complete remission after treatment with another therapy. It may be preferable desirable to decrease the tumor burden before using the methods of the present invention because since there are several alternative effector T-cells in cases of patients with highly active relapsed/refractory cortical T-cell acute lymphoblastic leukemia. In some embodiments, the patient to be treated with the method of the present invention has previously been treated with another therapy which resulted in a partial response, complete response, stable disease, decrease in progressive disease, reduced time to tumor progression or any combination thereof.

### Methods of quantification

As shown in Example 10, the detection and quantification of transduced cells with the h2CAR-CD1a has been greatly improved when the recombinant rCD1a was used, while the detection with scFv resulted to be around 15% lower (see **Figure 19****:** 58% positive cells detected with scFv versus 71 % of positive cells detected with rCD1a). These results are the consequence of the increased affinity between the h2CAR-CD1a developed herein and its target protein, CD1a. In addition, the detection with rCD1a is similar to GFP when co-expressed by a T2A-GFP modification of the h2CAR-CD1a construct (se **Figure 19****:** 65% GFP positive cells and 65% of rCD1a positive cells) and 15% higher than the one detected with anti-scFv. Consequently, the method used herein represents an improved method for detecting and/or quantifying the binding between rCD1a with h2CAR-CD1a. Thus, in a **sixth aspect,** the present invention provides a method for the detection of the binding between a CD1a protein, preferably the rCD1a, with the h2CAR-CD1a defined above. Preferably, the CD1a protein is a recombinant CD1a human protein. Preferably, the CD1a protein consists of the extracellular part of said protein. Preferably, the CD1a protein comprises a tag sequence, preferably, containing a 6xHIS tag. Preferably, the tag sequence is located at the C-terminus. Preferably, the CD1a is in the form of a dimer protein which is bound to a cell membrane human b2-microglobulin (hB2M).

In some embodiments, the method comprises the steps of i) mixing and incubating a sample comprising the h2CAR-CD1a with a sample comprising at least one dimer formed between CD1a and cell membrane b2-microglobulin (hB2M), ii) washing the mixture to wash away unbound proteins, and iii) detecting the presence of the binding between CD1a and h2CAR-CD1a in the sample.

In some embodiments, the method comprises the steps of i) incubating a sample comprising at least one dimer formed between CD1a and cell membrane b2-microglobulin (hB2M) with a sample comprising h2CAR-CD1a, ii) washing the mixture to wash away unbound proteins, and iii) detecting the presence of the binding between CD1a and h2CAR-CD1a in the mixture.

In a **seventh aspect,** the present invention relates to a recombinant CD1a protein as defined in the sixth aspect.

### EXAMPLES

List of Abbreviations and Acronyms used in the Examples
- **ALL/LL**: Acute lymphoblastic leukaemia (ALL)/lymphoblastic lymphoma (LL) is a clonal haematopoietic stem cell disorder of B- or T-cell origin considered as an aggressive haematologic neoplasm.
- **CAR T**: Generic term that refers to differentiated T lymphocytes transduced with any vector to express a chimeric antigen receptor
- **CAR**: Chimeric antigen receptor
- **CARCD1a**: Chimeric antigen receptor that recognizes the CD1a molecule
- **CD**: Cluster of differentiation. Acronym that are used together with a sequential number to define leukocyte membrane molecules, as is the case with CD1a.
- **CRS**: Cytokine Release Syndrome
- **NSG**: Immunodeficient mouse (NOD scid gamma) derived from crossing mice without a gamma chain of the cytokine receptors of the IL2 family, and with the molecule encoded by the mutated Prkdc gene, on a substrate of NOD mice (a strain of diabetic mice non-obese, non-obese diabetic); these mice cannot develop T lymphocytes, B lymphocytes, or NK cells.
- **OC_1**: Specific term referring to expanded autologous peripheral blood differentiated T lymphocytes transduced with a lentivirus to express a chimeric antigen receptor with anti-CD1a specificity conjugated to costimulatory regions 4-1BB and CD3z. The term also refers to the specific construction h2CD1a-CAR.
- **scFv**: Variable regions of the single chain fragment variable antibody. The term refers to the molecular construction that fuses in a sequence the variable regions of the heavy and light chains of a conventional antibody through a spacer sequence that allows to maintain the functionality of the specific antigen recognition and the function as an antibody.

### Example 1: Introduction and Summary of experiments

OC_1 (also called humanized h2CD1a-CAR-T) is a product classified as advanced therapy - gene therapy because the active substance is autologous T lymphocytes, transduced (genetically modified) by means of a vector lentiviral, so that they express in their membrane a chimeric antigen receptor, CARCD1a, with anti-CD1a specificity. OC_1 is indicated in patients with refractory or relapsed T-cell acute lymphoblastic leukaemia/lymphoblastic lymphoma (T-ALL/LL). The CD1a recognition on the lymphoproliferative surface of the cells by the CART, allows the cytotoxic function of T lymphocytes to eliminate tumour cells. OC_1 is administered by intravenous infusion in a fractionated dose posology.

CAR-Ts are based on modifying the T lymphocytes of a patient by inducing the expression on the surface of a specific antitumor receptor that is in the form of a chimeric membrane molecule; a chimeric antigen receptor (CAR). CAR combines a recognition zone by a construct that combines the specificity of a single chain antibody (scFv) with structural and signaling domains that activate the function of the CAR-expressing T lymphocyte. In the present case, hCD1a-CAR T, the CAR molecule recognizes the CD1a molecule through the scFv, a specific marker that is exclusively expressed in cortical T-ALL and T-LL (T-cell acute lymphoblastic leukemia and lymphoblastic lymphoma) [16].

CARs are usually expressed as dimers in the cell membrane and native antigens can be recognized in a non-MHC-restricted manner and therefore can be used in all individuals regardless of their HLA type [17, 18]. However, in the normal physiological situation, the sustained and complete activation and proliferation of T cells from a T cell receptor (TCR) occurs through complex processes involving a primary initiation signal (signal 1) in addition to activation of the secondary costimulatory receptor (signal 2) and the participation of a third receptor for survival such as those of the TNF family or cytokine receptors (signal 3). As CAR T cells do not function in an MHC-restricted manner, their lack of interaction with antigen-presenting cells (APCs) is generally insufficient, and therefore signal 2 and signal 3 are seriously compromised if not provide additions or modifications to the CAR T. Modifications and additions to the CAR design have cumulatively improved over a few "generations" of development to reach the third generation of design. Thus, it has been shown that the inclusion of CD137 (4-1BB) together with CD28 and CD3ζ in CAR gene constructs increases cytokine secretion and more potently induces greater inhibition of tumor growth in mice [18]. Indeed, first-generation CARs include only CD3ζ as an intracellular signaling domain, while second-generation CARs add a unique costimulatory domain derived from CD28 or 4-1BB; third-generation CARs include two costimulatory domains, such as CD28, 4-1BB or other costimulatory molecules [17]. Recently, "fourth generation CARs" approaches have been postulated that have been developed to modulate antigen-independent proliferation and the consequent increase in cell-mediated toxicity and / or raise concerns about the immortalization of infused CAR-T cells, for example, harboring an inducible suicide gene in the construction of CAR. OC_1 is a second-generation CAR-T that contains a 4-1BB as a costimulatory domain.

Thus, the mechanism of action of OC_1 (h2CD1a-CAR-T), like other CAR-Ts consists in generating an immune response against the antigens for which they have been designed (CD1a in this case). This response results in the expansion and activation of populations of T lymphocytes with cytotoxic capacity. When OC_1 is cultured against tumor cells with the antigen (CD1a), they generate the activation of the immune system against this antigen. This activation of effector T lymphocyte populations has the ability to lyse and kill tumor cells when specific recognition occurs, also *in vivo,* resulting in clinical responses.

### In summary:

1. OC_1 are human T cells expressing the h2CD1a-CAR, a humanized version of the previously described murine CAR. Moreover, OC_1 targets specifically human CD1a.
2. The active substances of OC_1 is autologous human cells and the preferred route of administration of cells is intravenous (infusion) in patients.
3. OC_1 only recognize the human CD1a molecule. In this sense, it is preferred that the T cell (in which the CAR is expressed) and the target cell are both from human origin.

For the development of OC_1, different experiments were performed in order to evaluate the efficacy of the product. As already mentioned, the initial development of OC_1 was performed with the murine scFv and one lentiviral vector. The CD1a-specific murine scFv was derived from the NA1/34.HLK clone of CD1a-specific antibody and obtained by using commercial synthesis with the mouse IgG Library Primer Set (Progen). This murine scFv was cloned (upstream of the CAR backbone) into a pCCL DNA lentiviral-based second-generation CAR backbone containing a human CD8 transmembrane domain, human 4-1BB and CD3ζ end domains, and a T2A-GFP (green fluorescent protein) cassette with the ampicilin (AMP) resistance gene (lentiviral vector pCCLsinPPT-hEF1A-IntronGFP.WPRE). This original CAR T-cell product was named mCAR-CD1a-AMP-GFP and is the one used in the study published by Sánchez-Martínez *et al* [16] and is the product disclosed in PCT/EP2020/053769.

Subsequently, in order to have a new vector suitable for clinical use, the GFP cassette was removed to generate the mCAR-CD1a-AMP vector and subsequently, the AMP resistance gene was replaced by a kanamycin (KAN) resistant gene, resulting in a product containing KAN and the murine scFv, called murine CAR-CD1a (mCAR-CD1a) (Figure 1).

Finally, as mCAR-CD1a was of murine origin, humanization of the murine scFv was performed to avoid immunogenic response in humans and to make the CAR T-cell product more suitable for using in the clinics. Overall, the lentiviral vector containing KAN (without AMP/GFP) and the humanized sequence of scFv in the CAR structure constitute the humanized CAR-CD1a (hCAR-CD1a or h2CAR-CD1a) or OC_1 **(****Figure 1****).**

Lentiviral particles were produced with the original mCAR-CD1a-AMP-GFP and with the modified versions mCAR-CD1a-AMP, mCAR-CD1a and hCAR-CD1a.

The following **Table 1** summarises all studies performed with the original CAR T-cell product (containing AMP/GFP and a murine scFv) that were included in the previous patent PCT/EP2020/053769. **Table 1** also contains bridge experiments with the mCAR-CD1a (containing KAN and a murine scFv) and finally with OC_1 (hCAR-CD1a, containing KAN and a humanized scFv). The experiments marked with a circle were included in the PCT/EP2020/053769, as they are part of the scientific paper published by Sánchez-Martínez et al [16], while all the black crosses are detailed in the different sections of this invention.

**Table 1. Non-clinical studies with the original CAR T-cell product, the mCAR-CD1a and OC_1 product. # A version with GFP of construct hCAR-CD1a (KAN + humanized scFv) has also been generated for binding experiments. AMP: ampicillin; CAR, chimeric antigen receptor; CD, cluster of differentiation; KAN, kanamycin; mCAR-CD1a, murine CAR-CD1a; scFv, single-chain variable fragment; T-ALL, T-cell acute lymphoblastic leukaemia.**

| **Non-clinical studies** | Original CAR T-cell (AMP/GFP+murine scFv)¹ | mCAR-CD1a(KAN+murine scFv) | **OC_1** hCAR-CD1A(KAN+humanized scFv) | Others |
|---|---|---|---|---|
| ***In vitro* studies** | | | | |
| CAR expression and expansion | O | **X** | X | |
| *In vitro* cytotoxicity with cell lines: Jurkat (CD1a+), MOLT4 (CD1a+), Nalm6 (CD1a-) | O | **X** | **X** | |
| Pro-inflammatory cytokines levels | O | **X** | **X** | |
| Cytotoxicity with primary cortical CD1a+ T-ALL samples | O | | | |
| Specificity studies: Off-target analysis | O | X | X | |
| Binding assays with recombinant CD1a | **X** | **X** | **X**^{#} | |

| ***In vivo* studies** | | | | |
|---|---|---|---|---|
| Jurkat infusion | O | **X** | **X** | |
| Primary cortical CD1a+ T-ALL blasts infusion | O | **X** | **X** | |

| **Pharmacokinetic studies** | | | | |
|---|---|---|---|---|
| Rechallenge (Jurkat)^{†} | O | | | |
| Rechallenge (Primary cortical CD1a+ T-ALL blasts) ^{†} | O | | | |

| **Toxicology studies** | | | | |
|---|---|---|---|---|
| Fratricide effect | O | | **X** | |
| Immunogeneicity** | | | | **X** |

The efficacy and safety of the original murine CAR T-cell product (AMP-GFP) was previously described by Sánchez-Martínez *et al* demonstrating a potent and specific antileukemic activity against coT-ALL cell lines and primary blasts in vitro and potent antileukemic activity *in vivo* [16]. All these experiments were described in PCT/EP2020/053769 and may be summarized as follows:
- *In vitro,* murine CD1a CARTs specifically eliminated CD1a+ T-ALL cell lines Jurkat and MOLT4, and not the B-ALL cell line NALM6 (as a negative control). *In vitro* murine CD1a CARTs produced high levels of the proinflammatory cytokines IL-2, TNFα, and IFN-γ on coculture with CD1a+ cell lines, confirming their cytotoxicity.
- *In vitro,* murine CD1a CARTs specifically eliminated primary CD1a+ coT-ALL cells but not BM normal hematopoietic cells (CD1a-) as well as CD1a- T-ALL blasts, further confirming the specificity of the murine CD1a CAR. High-levels of IFN-α and TNF γ were also secreted on coculture with CD1a+ primary T-ALL cells.
- *In vivo,* murine CD1a CARTs avoids leukemia establishment in mice transplanted with both Luc-expressing Jurkat T-ALL cells and a primary coT-ALL xenograft model.
- *In vivo,* murine **CD1a** CARTs are functional and persistent in rechallenge assays; that is, T-ALL-transplanted mice in which the leukemia was cleaned on treatment with murine CD1a CARTs were re-injected with either Luc/Jurkat cells or primary T-ALLs from primografts. The existence of persisting effector T cells in PB, BM, and spleen of rechallenged animals were able to control the disease.

### Example 2: HUMANIZATION STRATEGIES

Humanization of mCAR-CD1a has followed 3 strategies (Figure 20):
1) Structure-based approach
2) Sequence-based "Strict" mode
3) Sequence-base "Relaxed" mode
4) De-murinenization approach.

*Note:* the fourth strategy named 'de-murinenization' has also been followed but it is not *per se* an humanization approach. This approach relies on identifying regions on the murine sequence that are immunologically liable and make changes on the sequence to remove such liability. However, because the clone remains fully murine (it is recognized only by anti-murine antibodies, but not with anti-human antibodies, as shown in Example 4 below) this strategy was not further evaluated.

The main characteristics of each approach are the following:

### 1. Structure-based approach

It consists on:
- Modeling of scFv CD1a **(****Figure 21****)**
- Search on the PDB databank (www.rcsb.org) for known structures of a similar human scFv
- Perform an structural alignment between human scFv and murine scFv **(****Figures 21-22****)**
- Identify the most structurally and sequence similar human scFv **(****Figures 21-22****)**
- Replace CDRs + Stems on human by murine **(****Figures 23-24****)**
- Identify other structurally compromised positions on murine scFv and transfer to human murine (e.g. buried residues, sterically compromised residues, etc.).

The CAR-CD1a generated following this method is named hereinafter as h1CAR-CD1a or h1.

After comparison between the structural model of murine scFv and the most similar human scFv (structure 5wn9), we can conclude that, besides the CDRs, the sequence similarity (and identity) is very high too **(****Figure 22****).** The human scFv has a longer H3 (hence the gap in murine scFv). In summary, 5wn9 is a good candidate as human scaffold to transfer murine CDRs. Once similarities are identified, it is possible to replace murine CDRs + Stems on the human 5wn9 sequence by murine (see **Figure 23** for the heavy chain).

Next, we decided for each amino acid modification to keep the human or the murine version based on biochemical/structural criteria, as indicated in **Figure 23** for the heavy chain amino acids :
- Position a: N-terminal, murine Q, human V. This aa is exposed before the first beta-sheet. We keep the human V.
- Position b: Murine A, human P. This aa is exposed in a loop and away from CDRs and stem regions. Moreover P is a very special aa from a physic-chemical stand and thus difficult to substitute. We keep the human P.
- Position c: Murine LAR, human VKK. This is an important change between two parallel b-strands, second before H1. We decide to change to VAK. So aa A is kept (change to murine, less bulky)
- Position d: Murine R, human A. Changes in a distal loop (from CDRs) and fully exposed. We keep the human A.
- Position e: Murine NENFKDKATLTADK, human AQKLKARVTMTTDT. This is a long stretch comprising loop after H2 and following a beta strand. We keep some murine aminoacids that are close to H2 (NNF) and Vernier (LAK): NENFKDKATLTADK (kept aminoacids indicated in bold). The final humanized NQNFKARVTLTADK sequence (changed aminoacids indicated in gray, maintained indicated in bold).
- Position f: Murine HLSSLTS, human ELRSLRS. Region structurally adjacent to region 'e'. We keep some murine aminoacids as they pair to the murine ones kept in 'e': HLSSLT (kept aminoacids in bold). The final humanized sequence is the same than the murine (HLSSLTS).
- Position g: Murine CA, human GR. Both aminoacids are very different but are located in a region that appear not to be important for antigen recognition. We keep the human (GR).
- Position h: Murine S, human L. Not conserved change but not comprised structurally (exposed in last beta-strand). We keep the human.

Regarding the light chain, we followed a similar strategy and the alignment is shown in **Figure 24** and the aminoacids substitutions has also been indicated (from "a" to "h"). For the light chain we have the following changes similarly as for the heavy chain:
- Position a: Murine IQ, human TP. N-terminal region, facing L3 stems. We decided keep the murine.
- Position b: Murine L, human V. Packing b-strand distal. We decided to keep the human.
- Position c: Murine G, human D. Fully exposed mid-loop. We decided to keep the human.
- Position d: Changing all to murine as these aminoacids are located in the interface with the CDR H3 of the heavy chain. Moreover, the nature of the amino acids makes them important for loop structure (P, G, and R)
- Position e: Murine REYSFS, human TDFTLT. We decided to keep some murine as interact with CDR H2 of the heavy chain: REYSFS (bold maintained). Final humanized REYTLT (gray changed).
- Position f: murine NLE, human SLQ. Loop exposed on distal position. We decided to keep human SLQ.
- Position g: murine I, human F. Buried in distal position, it might be an important structure scFv. We decided to keep human F.
- Position h: murine G, human P. Basal position on L3. Might be important for entropy of L3. We decided to change to murine G.

In summary, the proposed humanized sequence based on the structural approach is the following:
Heavy chain (SEQ ID NO: 16) 
QVQLVQSGPEVAKPGASVRLSCKASGYAFSTYTMHWVRQAPGQGLEWMGYINPNSASTSYN
QNFKARVTLTADKSTNTAYMHLSSLTSDDTAVYYCGRGFYTMDYWGQGT LVTVSSA
Light chain (SEQ ID NO: 17)

DIQMTQSPSSVSASVGDRVTISCQASQDINKYIAWYQFKPGKGPRLLIHYTSTLQPAIPSRFSGS
GSGREYTLTISSLQPEDFATYYCLHYDNLPWTFGGGTKVEVRRA

### 2. Sequence-based "Strict" mode

This approach follows the subsequent protocol:
- Use the sequence of the scFv to query the database of IgG (http://www.imgt.org/).
- The search returns a list of human Ig genes ranked by E-value.
- Choose the one with the highest sequence identity to both heavy and light chains **(****Figures 25-26****).**
- The changes in the sequence of the human Ig gene were restricted to the CDRs and stems regions and also on non-conserved position (see next).
- Remaining non-conserved positions in the sequence were assessed in an individual basis. For instance, a non-conserved position was compared among the different human sequences retrieved in the search (second point). Depending on the level of conservation the change will be made, i.e. change from human to murine or the human will be preserved.
- Two different strategies to decide how many changes to perform on the non-conserved position were devised: strict and relaxed. The sequence-based "strict mode" tries to make the minimum changes to achieve humanization. In contrast, the "relaxed mode" allows more changes to the human sequence (see point 3).

The CAR-CD1a generated following this method is named hereinafter as h2CAR-CD1a or h2.

Regarding the heavy chain the query to the IgG database gives the Germline IGHV 1-46*02 that shares the highest sequence identity to the heavy chain (66%) **(****Figure 25****).** In this case the number of different residues without considering CRDs is 26.

Regarding the light chain, we found the Germline IGKVD 1D-33*01 that shares the highest sequence identity to the heavy chain (72%). In this case, the number of different residues without considering CRDs is 21 **(****Figure 26****).** The conservation among the different human sequences for the heavy and light chains is indicated in **Figure 27** and is used to assess the level of conservation among human sequences in non-converved positions (that are not the CRDs and stem regions) between murine and human sequences.

According with this methodology, the final selected sequence in the 'strict mode' is the next:
Heavy chain (SEQ ID NO: 2) 
QVQLQQSGAEVKKPGASVKVSCKASGYAFSTYTMHWVRQAPGQGLEWMGYINPNSASTSYA
QKFQGRVTMTADKSTNTAYMELSSLRSEDTAVYYCARGFYTMDYWGQGTLVTVSS
Light chain (SEQ ID NO: 1)

RDIQMTQSPSSLSASVGDRVTITCQASQDINKYIAWYQFKPGKGPRLLIHYTSTLEPAIPSRFSG
SGSGREYTFTISSLQPEDIATYYCLHYDNLPWTFGGGTKVEIKRA

### 3. Sequence-based "Relaxed" mode

A different humanized sequence was selected allowing more amino acid changes in the human sequence with respect to the murine sequence. In **Figure 28** is indicated both the "strict" and "relaxed" sequences together with the murine. The "relaxed" sequence is indicated below:
Heavy chain (SEQ ID NO: 18) 
QVQLVQSGAELVKPGASVRVSCKASGYAFSTYTMHWVRQAPGKGLEWVGYINPNSASTSYN
DSVKGRFTISRDTSKNTAYMEMNSLRSEDTAVYYCARGFYTMDYWGQGTLVTVSS
Light chain (SEQ ID NO: 19)

RDIVLTQSPSSLSASLGQRVTISCQASQIDNKYIAWYQQKPGQAPKLLIHYTSTLQPGIPDRFSG
SGSGTDFTLTISGLQPEDFATYYCLHYDNLPWTFGGGTKLEIKRT

The CAR-CD1a generated following this method is named hereinafter as h3CAR-CD1a or h3.

### 4. De-murinenization approach.

In this approach we identified immunogenic regions in the murine sequence and perform single amino acids changes to eliminate such liabilities. The identification of immunogenic regions is based on computational predictions. This is, *per se,* not a humanization process.

The protocol followed was the next:
- Predict antigenic regions on the murine scFv sequence: MCH-I binders and B-cell Epitopes **(****Figure 29****)** using computational approaches (see next).
- Of the regions identified as potential immunogenic, identify amino-acid(s) responsible and replace by a different one(s) from the multiple sequence alignments derived before **(****Figure 30****)**
- 3 different computational approaches were used; 2 sequence- and 1 structure-based:
   - Sequence-base method: MCH-I (HLA supertype):
      - Artificial neural networks: application to the MHC class I system [19].
      - Prediction of T-cell epitopes [20].

These two prediction methods are sequence-based artificial intelligent computational methods.
- Structure-based method: B-cell epitopes (3D) [21]. The structural model of the murine scFv generated for the structure-based humanization was used in here.

The CAR-CD1a generated following this method is named hereinafter as h4CAR-CD1a or h4. The final "de- murinenized" candidate is the following:
Heavy chain (SEQ ID NO: 20) 
EVQLQQSGAELARPGASVKMSCKASGYAFSTYTMHWVQQRPRQGLEWIGYINPNS
ASTSYNQNFKDKATLTADKASNTAYMHLSSVRVSDSAVFFCARGFYTMDYWGQGT
SVTVSS
Light chain (SEQ ID NO: 21)

RDIQMTQSASSASASPGGRVTITCQASQDINKYIAWYQFKGPKGPRLVIHYTSTL
QPAIPSRFSGSGSGREYSFSISNLEPDDIATYYCIHYDNLPWTFGGGTKLEIKRP

### Example 3: In silico analyses of murine and humanized scFvs

A comparative analysis of murine and the three humanized version: structure-based (h1), sequence-based 'strict' (h2) and sequence-based "relaxed" (h3) has been performed following 3 different types of analysis:
- Immunogenicity (sequence-based & structure-based)
- Sequence 'liabilities' (sequence-based)
- Stability (structure-based):
   ∘ Protein: Rosetta and SPServer
   ∘ Interface: Rosetta and SPServer

### 1) Immunogenicity

With the aim to assess the level of immunogenicity of the different scFv, we conducted a bioinformatic approach for the prediction of potential MHC-I/II binders. The next resources were employed:
- NetMHCcons

Artificial neural network (NN) to predict MCH-I binders [22]
- NetMHCstabpan

Artificial NN to predict the stability of MCH-I binders [23]
- NetMHCIIpan

Artificial NN to predict MCH-II binders [24]

The number of immunogenic peptides obtained with the different bioinformatic analysis are summarized in **Table 2.** The humanized scFv that presented a stronger reduction of immunogenic peptides is h2.

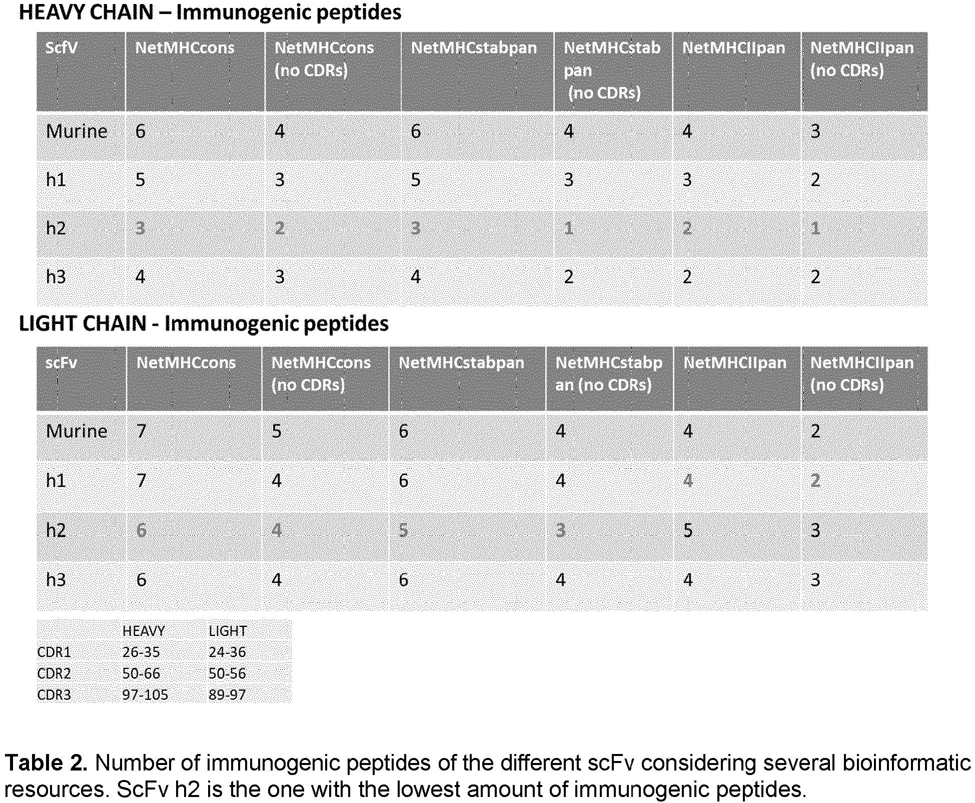

### 2) Sequence 'liabilities' (sequence-based)

The process of humanization necessary involves amino acid substitutions of the original murine scFv sequence. Sometimes these modifications may introduce "liabilities" that are sequence potential changes at the protein most of the times post-translational modifications that can affect the function and stability of the scFv. **Table 3** summarizes the different sequence liabilities that has been analyzed and **Table 4** the ones that has been found in the different scFvs. As shown in **Table 4,** h3 presents an N-linked new glycosylation site NDS in aminoacid positions 61-63 from the heavy chain, absent in the rest of humanized forms and murine. The rest of liabilities observed were already present in the murine scFv.

**Table 3. Summary of sequence liabilities.**

| Types of liabilities | | References |
|---|---|---|
| Unpaired Cys (C) | Brych SR et al. 2010. J Pharm Sci. Feb;99(2):764-81 | |
| N-linked glycosylation (NXS/T,X not P) | Gavel Y, von HG. 1990. Protein Eng 3:433-442 | |
| Met oxidation (M) | Jarasch A et al. 2015. Jun;104(6):1885-98 | |
| Trp oxidation (W) | Jarasch A et al. 2015. Jun;104(6):1885-98 | |
| Asn deamidation (NG NS NT) | Sydow JF et al. 2014. PLoS ONE. 9(6):e100736. | |
| Asp isomerisation (DG DS DT DD DH) | Sydow JF et al. 2014. PLoS ONE. 9(6):e100736. | |
| Lysine Glycation (KE KD EK ED) | Jarasch A et al. 2015. Jun;104(6):1885-98 | |
| N-terminal glutamates (E) | Liu YD et al. 2011. J Biol Chem. Apr 1;286(13):11211-7 | |
| Integrin binding (RGD, RYD, LDV) | Ruoslahti E. 1996. Annu Rev Cell Dev Biol 12:697-715. | |
| | Humphries JD et al. 2006. J Cell Sci. Oct 1;119(Pt 19):3901-3. | |
| | Plow EF et al. 2000. Annu Rev Cell Dev Biol. 12:697-715. | |
| CD11c/CD18 binding (GPR) | Loike et al. 1991. PNAS. 88(3): 1044-1048. | |
| Fragmentation (DP) | Vlasak J. and lonescu R. 2011. MAbs. May-Jun; 3(3): 253-263. | |

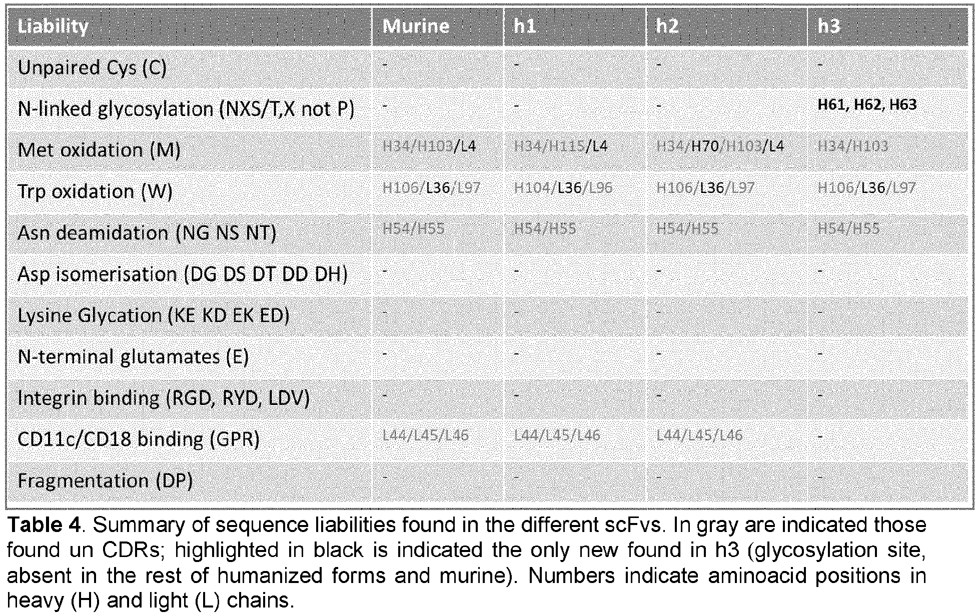

### 3) Stability

The stability of the different scFvs has been evaluated by using different structural predictions and calculations. The SPServer analyzes protein folds and protein interfaces that allows to asset the quality of structures based on statistical potentials that are converted into energies. This analysis reports that h1 and h2 have a more robust protein fold and better interface energies than murine. Results are available at **Table 5** (Fold analysis, PAIR energies of h1 better than the rest, ES3DC energies of h2 better than murine) and at **Table 6** (Interface analysis, PAIR energies of h1 and h2 better than murine).

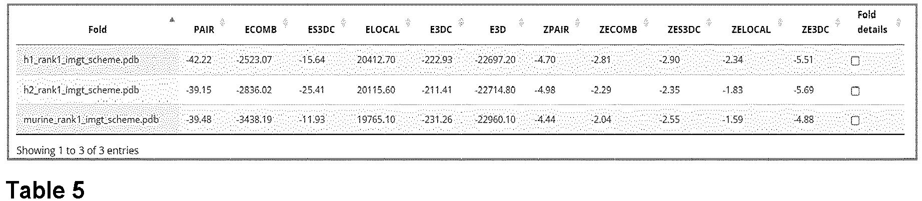

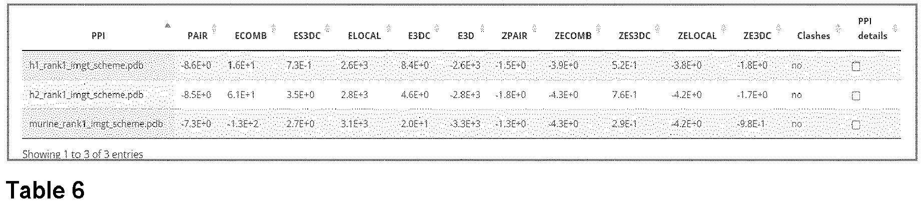

By contrast h3 were similar to murine in terms of protein fold (Table 7) and interface analysis (Table 8).

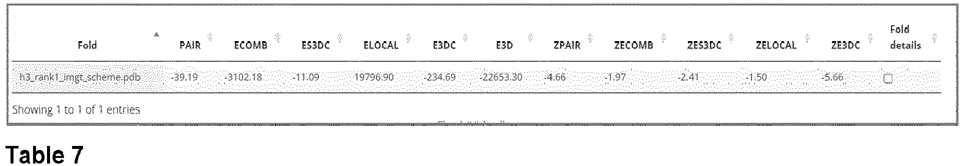

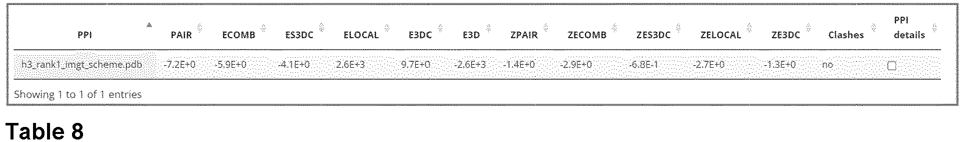

On the other hand, the Rosetta computational suite allows computational modeling and analysis of protein structures estimating a number of metrics included an estimation of change in free energy (dG). Analysis with Rosetta indicates that h3 appears to have a less favorable stability (score and free energy (dG) and worst interface scores than the rest of humanized constructs and the murine.

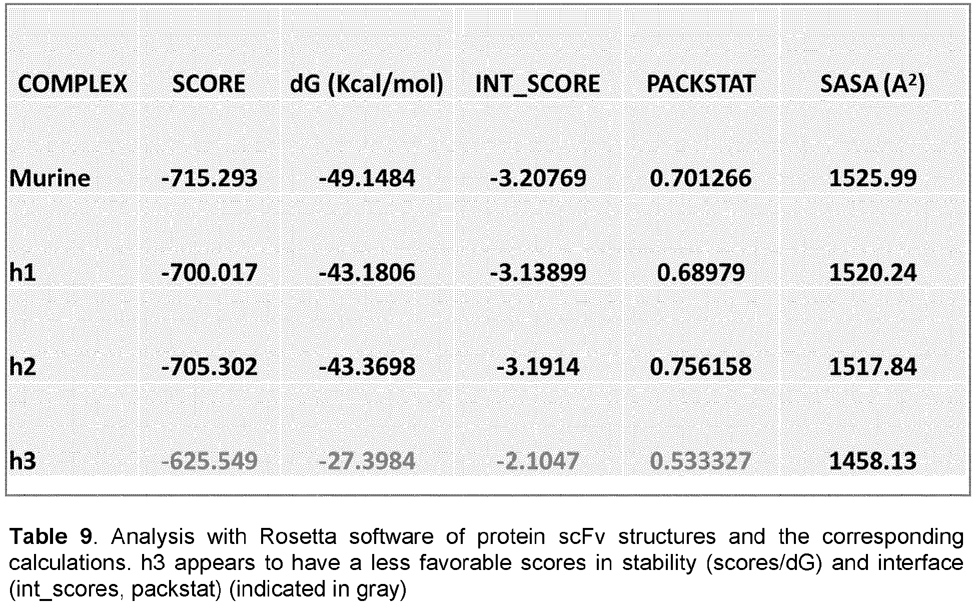

### 4) Summary of results

As a summary of the different analysis, we can conclude that all humanized scFv versions has lower immunogenic capacity and that h2 is the best one with a lower number of putative immunogenic peptides. Regarding liabilities, only the h3 had an important concern with a new N-glycosylation site that was not present in the murine. Importantly, h1 or h2 had not observed liabilities. Glycosylation of h3 may compromise the CAR function and impair the functional target recognition by the scFv. Regarding stability, both h1 and h2 had improved protein stabilities, depending on the analysis by SPServer and in any case, h3 has worse stability by using the Rosetta approach. By all these *in silico* predictions we can conclude that h3 is the worst of all humanized scFv **(Table 10).**

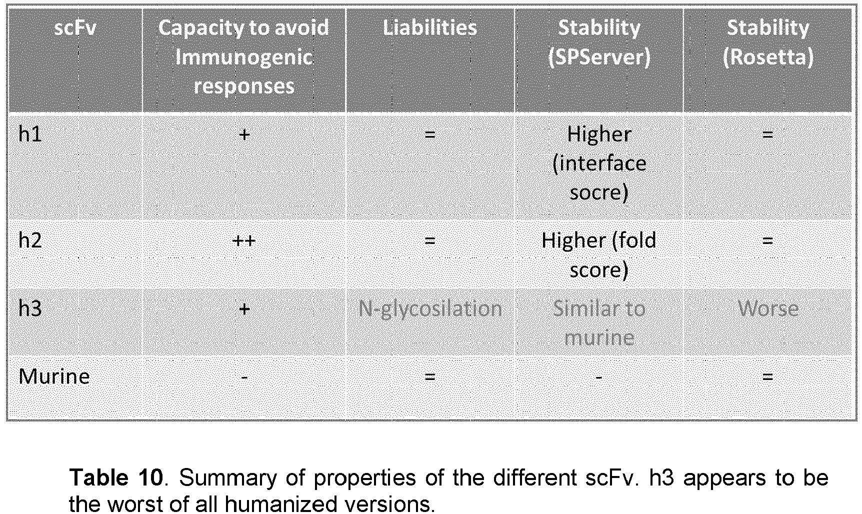

### Example 4: Functional comparative analysis of humanized scFvs

Apart from the *in silico* prediction reported in the previous example, we have performed some functional analysis in order to select a final scFv humanized candidate. First, we have analyzed if the four humanized scFvs may produce CAR lentiviruses in HEK293T with a similar efficiency and can be detected with anti-human antibodies to check that humanization has effectively worked **(****Figure 31****).** Humanized scFvs 1, 2 and 3 can be detected with anti-human antibodies in a dose-dependent manner, except the humanized 4 scFv CAR that was discarded because it cannot be detected with anti-human antibodies but it does with anti-murine antibodies. This is because humanized 4 correspond to a de-murenization strategy (see **Example 2)** and is not a humanization *per se* and so it was not considered for further use.

Calculation of virus titer from different batches (n=6) gave similar results for h1 and h2 (similar to historically obtained with the murine indicated in the **Table 11,** meanwhile titers with h3 were much lower **(Table 11).** Infection efficiencies of PBMCs were also decreased with h3 despite virus volume was normalized accordingly to have a similar MOI of 10 **(****Figure 32****).** Infection of PBMC from three different donors and cytotoxicity assays with Jurkat (CD1a+) co-cultured cells during 24h revealed that humanized 1 was equally effective than the murine in killing assays **(****Figure 33****,** **A).** Interestingly, humanized 2 showed slightly better efficacy than the murine or humanized 1 at this observation time **(****Figure 33****, A).** By contrast, humanized 3 showed no or little killing capacity **(****Figure 33****, A).** No significant cytotoxic effect was observed with any of humanized constructs on NALM6 (CD1a-) cells **(****Figure 33****, A).** Subsequently, the amount of released proinflammatory cytokines IL-2 and IFNγ cytokines were produced at similarly high levels at 24 h with m, h1 and h2CD1a-CARs in Jurkat cells but not a significant increase was detected with Nalm6 cells (data were pooled from two independent experiments with 3 different donors). Interestingly, the levels of TNFa were lower with h1CD1a-CAR-Ts in comparison to h2CD1a and mCD1a-CAR-Ts and, as expected, there is a dramatic reduction with h3CD1a-CAR-Ts in all the cytokines quantified with no statistical significance difference related to UT cells **(****Figure 33****, B).**

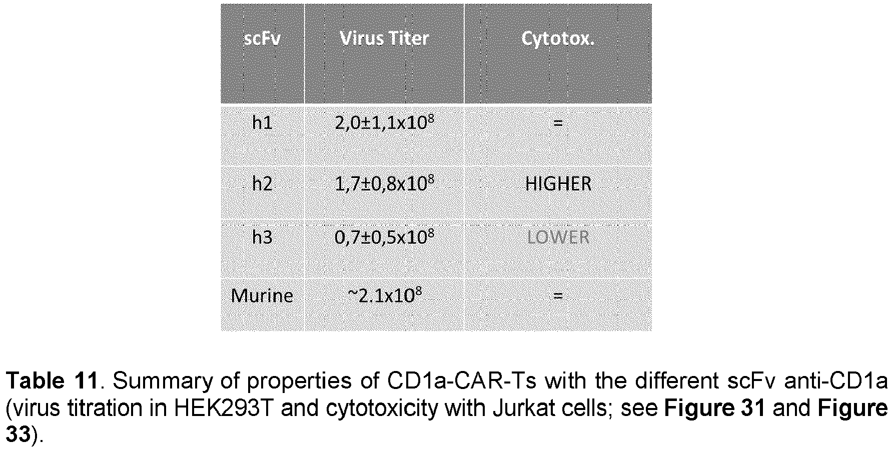

In summary, h3CAR-CD1a was discarded by its low titer viral production, low infection capacity and because its low cytotoxic activity **(Table 11).** These data are in agreement with the liabilities and lower stability found with h3CAR-CD1a. h1 and h2-CAR-CD1a appeared to be the best of all humanized versions and were selected for further studies.

H1 and h2CD1a-CAR-Ts can be expressed in the membrane of transduced peripheral blood mononuclear cells (PBMCs) with similar expression and proportion of CD4+/CD8+ cells than mCAR-CD1a **(****Figure 34****, A)** and transduced PBMCs expanded in a similar fashion **(****Figure 34****,** **B).**

*In vitro* data showed that h2CD1a-CAR-Ts are slightly better than h1CD1a-CAR-Ts **(****Figure 33****).** In NSG animals, as shown in **Figure 10** the *in vivo* activity of h2CAR-CD1a was slightly higher than mCAR-CD1a. Complete data with h1CAR-CD1a was also included in **Figure 35****.** Quantification of BLI by IVIS showed an improvement effect of h2CAR-CD1a related to the h1CAR-CD1a and the murine version **(****Figure 35 B)****.** FACS of tumour burden in PB and BM confirmed all CD1a CAR-T cells efficiently eliminated Jurkat cells **(****Figure 35 C)** but the number of mice with less than 0,1% (selected as arbitrary cutoff) of circulating blast cells in PB was lower in those mice receiving h2CD1a-CAR-T (10 mice from 12) compared to mCD1a-CAR-T (5 mice from 12) or h1CD1a-CAR T-cells (6 mice from 12). These results demonstrate that h2CD1a-CAR-Ts exhibit potent antileukemic activity that was higher than that of h1 or mCD1a-CAR-Ts **(****Figure 35B****).** Finally, the number of mice with more than 1% of CAR-T cells detected in the BM are significantly higher in those receiving h1 and h2 CD1a-CAR-Ts (6 mice from 12 in both groups) compared to mCD1a-CAR-T cells (2 mice from 12).

### Example 5: Bridge experiments between the murine and humanized construct

As mentioned above, the initial development of OC_1 was performed with a lentiviral vector containing a T2A-green fluorescent protein (GFP) cassette with the ampicillin (AMP) resistance gene, and for the CAR structure the lentiviral vector contained a murine CD1a-specific single-chain variable fragment (scFv) [16] **(****Figure 1A****).** Subsequently, in order to have a new vector suitable for clinical use, the GFP cassette was removed **(****Figure 1B****)** and the AMP resistance gene was replaced by a kanamycin (KAN) resistant gene, resulting in a product containing KAN and the murine scFv, called murine CAR-CD1a (mCAR-CD1a) **(****Figure 1C****).**

In order to demonstrate that vector modifications did not affect the biological function of the original CAR T-cell product, the efficacy of the different CD1a murine CARTs was assessed. The different CD1a murine CARTs, mCAR-CD1a-AMP-GFP (original construct, abbreviated AMP-GFP), mCAR-CD1a-AMP (abbreviated AMP) and mCAR-CD1a (abbreviated KAN to highlight the substitution of AMP per KAN), were tested *in vitro* using the CD1a expressing T-ALL cell lines Jurkat and MOLT4 (CD1a+ expression verified with two different antibodies in **Figure 2 A)****,** and the B-ALL cell line Nalm6, as negative control (CD1a- expression verified in the case of NALM6 in **Figure 2 A)****.** Compared with control (UT), all CD1a CARTs similarly eliminated CD1a+ T-ALL cells (Jurkat, MOLT4) at E:T ratio 1:1 in 72-hour assays **(****Figure 2B** absolute number of cells), without affecting CD1a- cell line (NALM6).

The activity of murine CD1a-CAR-Ts *in vivo* using Luc/GFP-expressing Jurkat T-ALL cells was also evaluated **(****Figure 3****).** NSG mice were transplanted with 3×10⁶ Luc-expressing Jurkat cells 3 days before i.v. infusion of either 4×10⁶ CD1a CARTs or UT PBMCs, and leukemia establishment was followed up weekly by using BLI quantification. In contrast to the mice receiving UT cells, which showed massive tumor burden by BLI, those mice given CD1a CARTs were practically leukemia free until day 17 **(****Figure 3A****).** Quantification of BLI by IVIS showed no differences among the different murine CAR-CD1a versions **(****Figure 3B****).** Flow cytometry analysis (FACS) of tumor burden in PB and BM confirmed all CD1a-CAR-T cells efficiently eliminated Jurkat cells **(****Figure 3C****).**

**Note:** *For the in vivo studies, the Jurkat cell line, which is considered equivalent to a pre-T precursor, similar to the defined by T-ALL, constitutes the paradigmatic example of implantation and growth of T-ALL tumor cells. To track the tumor within the mouse, cells are marked with traceability molecules. In our case, the Jurkat cell line has co-expressed a green fluorescent protein (GFP) and the luciferase enzyme. This enzyme when is oxidized with luciferin, it catalyzes a reaction that emits photons* as *detectable light with photonic detection equipment's (IVIR or Hamamatsu). The reaction catalyzed by luciferase uses D-Luciferin* as a *substrate that is further transformed into the luminescent product, and that can be detected from outside the animal. In this animal model, effector cells can also be infused, like in our case, mCD 1a-CAR T-cells. These transduced lymphocytes (or their equivalents but without the transduction, that are added* as a *control) make it possible to slow or eliminate tumor growth in vivo in mice. The most frequently used in vivo models are mice that can receive human elements by modeling these components in vitro. But, there have several and important limitations when the interaction between human cells and the murine environment takes place. The success of human T cell grafting in most immunocompromised mice is limited* as *residual elements of the mouse immune system continue to challenge the human T cell graft [25]. For this reason, highly immunocompromised models have been developed. The most widely used model is the so-called NSG which,* as a *mouse model without lymphocytes, allows the implantation of human cells without rejecting them. NSG or NOD-SCID gamma mice (NOD.Cg-Prkdcscid ll2rgtm1Wjl* / *SzJ) are a strain of inbred laboratory mice, among the most immunodeficient described to date. NSG mice lack mature T cells, B cells and natural killer (NK) cells. NSGs are also deficient in multiple cytokine signaling pathways and have various defects in innate immunity. Compound immunodeficiencies in NSG mice allow for the engraftment* of a *wide range of primary or derived human cells and allow for the sophisticated modeling of many areas of human biology and disease. NSG mice were developed by Dr. Leonard Shultz's group at the Jackson Laboratory. In our center, we have a colony of these mice. Although the tumor cell implant is not equivalent (in terms of distribution and quantity) to that which could be expected due to its natural development in humans, this xenogeneic model (NSG modified mice) constitutes the best animal model for the development of tumor cells of human origin in a complete animal environment.*

Overall, these results suggested that modified mCAR-CD1a versions are suitable for clinical use and indistinguishable of original vector in terms of efficacy and lack of off-target effects. Therefore, the change in the vector did not affect the biological function of the mCAR-CD1a.

### Example 6: scFv humanization, hCAR-CD1a expression and expansion of OC_1

In CARs of murine origin, the scFv sequence is usually derived from mouse mAbs, raising justified concerns on their potential rejection by the host immune system, either by humoral or cellular responses. Moreover, since CARs are synthetic biological products, the joining regions between the different portions may give rise to newly generated immunogenic peptides [26]. In non-human primate models, cellular immune responses against the rodent CAR scFv component have been documented, suggesting that xenogeneic immune barriers may pose a problem when using murine scFv [26, 27]. Furthermore, murine scFv sequences may be recognized by the host immune system rendering second and successive cell infusions ineffective. In some cases, this response has been associated with the early loss of CAR [28], an event that has been solved with the infusion of humanized or human CARs.

As mCAR-CD1a was of murine origin, humanization of the murine scFv was performed to avoid immunogenic response in humans and to make the CAR T-cell product more suitable for using in the clinics. For this purpose, four types of strategies were conducted that were explained in

### Example 2:

- A structure-based approach (h1CAR-CD1a, h1)
- A sequence-based approach with minimum changes in non-CDR regions: sequence-strict (h2CAR-CD1a, h2)
- A Sequence-based approach with more changes in non-CDR regions: sequence-relaxed (h3CAR-CD1a, h3)
- A de-murenization approach (h4CAR-CD1a, h4).

We first selected the structure-based (h1) and the sequence-based "strict" CAR (h2) based on *in silico* prediction results explained in the **Example 3** that reported no liabilities and stability higher than the sequence-based "relaxed" CAR h3. The "de-murenized" version h4 was discarded at the very beginning because it was not recognized by anti-human antibodies **(Example 4).** In addition, comparative functional analysis revealed that h3 was totally inefficient in its killing potential and for this reason was also discarded **(Example 4).** h2 showed higher killing potency than h1 or murine that behave very similar **(Example 4).** Finally, h2 showed the lowest immunogenicity compared to the rest of humanized versions **(Example 3). For all these reasons, h2 was finally selected and named h2CD1a-CAR or OC_1 in the next sections** (summary of properties in **Table 12).** At some points, when additional comparisons between h1 and h2 has been performed always the h2 has shown better *in vitro* and *in vivo* properties **(Example 4).**

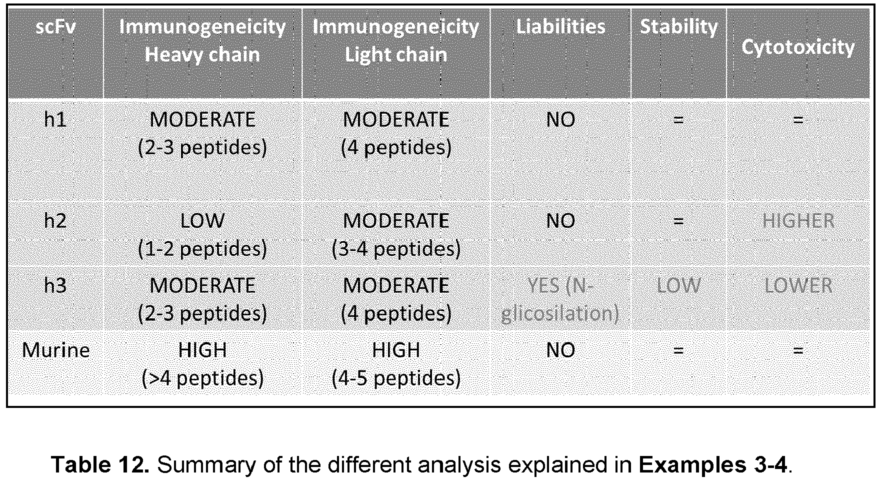

Humanization based on sequence is explain in detail in **Example 2.** In brief, with this method, complementarity determining regions (CDRs) of murine origin were grafted to a human scFv backbone. Firstly, CDRs and stem sequences were identified and are shown in **Figure 4** **B** (stems are positions before and after the CDRs, also called Vernier zone/regions). To prevent binding affinity loss, apart from CDR grafting, preservation of all mouse residues at the Vernier zone was also taken into account. The sequence of the murine scFv was used for searching a list of human immunoglobulin genes that were ranked by E-value similarities (http://www.imgt.org/). The gene with the highest sequence identity to both heavy and light chains was chosen. In this regard, the germline IGHV 1-46*02 shared the highest sequence identity to the variable heavy chain V_{L} (66%) and the number of different residues without considering CRDs was 26. Regarding the light chain V_{H}, the germline IGKVD 1D-33*01 was the one sharing the highest sequence identity to the V_{H} (72%) and the number of different residues without considering CRDs was 21. Besides the CDRs and stems regions, other changes (non-conserved) were analysed one by one and assessed whether to change it or not considering the conservation among the different human sequences, always trying to introduce the minimum number of changes*.

***Note:** *The decision to change or not a particular amino acid by the sequence-based method is not* as *straightforward* as *the structure-based approach (the fact that we know the structure allows to make changes that do not affect such structure)* so *at some point the based-sequence approach may follow other criteria more related with researcher experience.*

The proposed humanized sequence is shown in **Figure 4** **B,** for the V_{L} and V_{H} chains where CDR sequences and modified amino acids have been highlighted. The connector sequence from the heavy and light chains is also indicated and is the same than in the murine clone. Note that 21 and 9 amino acids have been changed in the V_{H} and V_{L} chains, respectively, to have the humanised version of scFv.

Overall, the lentiviral vector containing KAN (without AMP/GFP) and the humanized sequence of scFv in the CAR structure constitute the humanized CAR-CD1a (h2CAR-CD1a), the final product called OC_1 **(****Figure 1D****).**

In detail, the OC_1 structure (CART of second generation) contains **(****Figure 4A****):**
- Single-chain antibody variable fragment (scFv): to recognize CD1a on the membrane of T-ALL/LL malignant plasma cells. The scFv region is derived from a murine monoclonal antibody that has subsequently been humanized, while the signaling domains correspond exactly to the corresponding regions of native human proteins. The murine CD1a-specific single-chain variable fragment (scFv, with extracellular recognition sites: V_{H} (Heavy chain) and V_{L} (Light chain) domains), derived from the NA1/34.HLK clone of CD1a-specific antibody, was obtained by commercial synthesis (Millipore Sigma) with the mouse IgG Library Primer Set (Progen) [16]. The NA1/34.HLK monoclonal antibody has a proven *(in vitro* and *in vivo)* specificity against T-ALL/LL malignant cells. Later, this sequence has been humanized and the efficacy and safety of OC_1 (h2CD1a-CART) was demonstrated in several comparative assays **(****Figures 5 to 15****).**
- CD8a: transmembrane (TM) spacer.
- CD137 (4-1BB); signal 2 and co-stimulatory domain/combined intracellular signaling domain: The 4-1BB ligation induces a signaling cascade that results in cytokine production, expression of anti-apoptotic molecules and enhanced immune responses [29]. The combination of 4-1BB and CD3ζ in a CAR increases its ability to elicit T-cell expansion and anti-tumor activity [26, 29].
- CD3ζ; signal 1 and intracellular region: Signaling domain mediated by the ζ-chain of the TCR-CD3 complex. Clustering of CARs during interaction with tumor antigen (CD1a in this case) results in recruitment of associated molecules, phosphorylation of signaling domains and activation of downstream kinase cascades leading to gene transcription, cell activation and cellular responses to diseased cells. CD3ζ may mediate increased cytokine production in response to CD1a to mediate tumor regression.

### Immunogenicity

Clinical studies with CD19-CAR T cells using murine scFvs have reported immunogenicity as a major concern in subsets of patients and a worse response in those treated with repeated infusions [30]. A comparative analysis of both mCAR-CD1a and h2CAR-CD1a were performed *in silico* with different Artificial Neural Networks (ANNs) with the aim to assess the level of immunogenicity of scFvs. For this purpose, the prediction of potential MHC-I/II binders were conducted by using the following resources:
- NetMHCcons: ANN to predict MCH-I binders [22]
- NetMHCstabpan: ANN to predict the stability of MCH-I binders [23]
- NetMHCIIpan: ANN to predict MCH-II binders [24]

As shown in **Table** 13, the number of immunogenic peptides were significantly reduced to half or less depending on the algorithm employed in the case of humanised scFv related to the murine counterpart. When the analysis was performed without considering CDRs (regions that cannot be modified) immunogenicity was indeed lower. For a complete comparison between all humanized versions (h1, h2 and h3) and the murine see the **Example 3.**

**Table 13. Prediction of immunogenic peptides in both heavy and light chains of murine and humanised (h2) anti-CD 1a scFvs. Numbers in bold are indicative of a net reduction. Analysis has been perfomed with the complete scFv sequences or without the CDRs.**

| **HEAVY CHAIN** - **Immunogenic peptides** | | | | | | |
|---|---|---|---|---|---|---|
| scFv | NetMH Ccons | NetMHC cons (no CDRs) | NetMHCstdD pen | NetMHCstabpan (no CDRs) | NetMHC IIpan | NetMHCII pan (no CDRs) |
| Murine | 6 | 4 | 6 | 4 | 4 | 3 |
| Humanized (h2) | 3 | 2 | 3 | 1 | 2 | 1 |

| LIGHT CHAIN - Immunogenic peptides | | | | | | |
|---|---|---|---|---|---|---|
| scFV | NetMH Ccons | NetMHC cons (no CDRs) | NetMhCstab pan | NetMHCstabpan (no CDRs) | NetMHC II pan | NetMHCII pan (no CDRs) |
| Murine | 7 | 5 | 6 | 4 | 4 | 2 |
| Humanized (h2) | 6 | 4 | 5 | 3 | 5 | 3 |

Once the humanization of the CAR-CD1a was achieved, a first experiment to analyse the CAR expression and expansion of the h2CAR-CD1a (OC_1) was performed. Lentiviral particles for h2CAR-CD1a and mCAR-CD1a were generated to infect PBMCs. CAR-expressing lentiviral particles pseudotyped with vesicular stomatitis virus G glycoprotein were generated in HEK 293T cells by using standard polyethylenimine transfection protocols and concentrated by ultracentrifugation, as previously described by Sánchez-Martínez et al [16]. Viral titers were consistently in the range of 10⁷ to 10⁸ TU/mL (see **Example 4** for a complete comparison between all humanized constructs). PBMCs were isolated from buffy coats from healthy donors by using Ficoll-Hypaque gradient centrifugation. T-cells were activated by plate-bound anti-CD3 (1µg/ml) and anti-CD28 (1µg/ml) antibodies for 2 days. IL-17 and IL-15 (10 ng/mL) was added on the second day and kept throughout the transduction and expansion phase. On the third day the cells were collected, counted and then transduced. 0.5 or 1 million cells were used for transduction with CAR-expressing lentivirus (multiplicity of infection, MOI = 10). The cell surface expression of CAR-CD1a was traced by fluorescence-activated cell sorting (FACS) using a two-step staining method. First, the cells were incubated with either Biotin-SP-conjugated AffiniPure F(ab')2 Fragment Goat Anti-Mouse or Anti-human IgG and secondly with Streptavidin-PE antibody **(****Figure 5****).** Efficient activation of CAR-transduced T-cells was also checked by FACS staining for CD25 and CD69 after 2-day expansion (not shown). Frequency of T-cell subsets was analysed at day 5 after transduction by staining for CD3, CD4 and CD8 **(****Figure 6****).** The expression of CD1a on different cell lines was measured by FACs using anti-human-CD1a antibodies (Clone NA1/34-HLK, Invitrogen and Clone HI149, Becton Dickinson) **(****Figure 2A** **and** **Figure 12****).**

Firstly, it was demonstrated that scFv humanization effectively took place. As shown in **Figure 5****,** the scFv from h2CAR-CD1a transduced PBMCs can be readily detected with anti-human and anti-murine scFv antibodies, meanwhile mCAR-CD1a can be only detected by anti-murine antibodies, demonstrating that humanization has effectively worked **(****Figure 5 A)****.** Infection of 3 different donors gave transduction efficiencies of PBMCs ranging from 30 to 60% for h2CAR-CD1a, that were comparable to the expression detected for mCAR-CD1a detected anti-murine scFv antibodies **(****Figure 5 B)****.** Median membrane expression of mCAR-CD1a was 53% ± 15 and for h2CAR-CD1a was 56 ± 18 (n=3). In addition, transduced PBMCs expanded in a similar fashion **(****Figure 5 C)****.** A comparative analysis with h1, h2, h3-CARs-CD1a is also shown in **Example 4.**

Finally, CAR-T generated under these conditions showed similar proportion of CD4+ and CD8+ cytotoxic T-cells **(****Figure 6****).** With these results, it can be concluded that humanized scFv h2CAR-CD1a can be expressed in the membrane of transduced peripheral blood mononuclear cells (PBMCs) with similar expression and proportion of CD4⁺/CD8⁺ cells than mCAR-CD1a. A comparative analysis with h1-CAR-CD1a is also shown in **Example 4.**

### Example 7: In vitro activity studies of OC_1

The activity of **OC_1** was evaluated in different studies: i) a first study to evaluate the product cytotoxicity and ii) a second assay to determine the pro-inflammatory cytokines levels induced by the product. Each study is further explained in the following subsections.

### i) In vitro cytotoxicity with cell lines

The capacity of **h2CD1a-CAR** T-cells to eliminate target (T) CD1a+ Jurkat and MOLT4 cells was analyzed. For this purpose, target cells (100.000 cells per condition) were labeled with 3 µM eFluor 670 and incubated with UT PBMCs and CARTs at different effector (E):Target (T) ratios during 24 hours. The CD1a- NALM6 was employed as a control of specificity, as previously described by Sánchez-Martínez et al [16]. Following the protocol described by these authors, cell viability was monitored by eFluor labelling of target cells and detection by FACS. CART-mediated cytotoxicity was determined by analyzing the residual alive (7-amino actinomycin D negative) eFluor 670-positive target cells at each time point and E:T ratio. Percentage of cell populations were calculated by FACSDiva software analysis.

As shown in **Figure 7****,** h2CAR-CD1a strongly decreased cell viability at all the ratios analyzed in both Jurkat **(****Figure 7 A)** and MOLT4 **(****Figure 7 B)** being the humanized CAR more potent than the murine version. No mortality was observed with UT cells discarding non-specific effects. Similar analysis performed in NALM6 cells showed only a slight effect at the highest ratio E:T 1:1 in both murine and humanized CAR-CD1a, although non statistically significant related to UT cells **(****Figure 7 C)****.** Finally, the protocol was simplified by using Jurkat-GFP cells giving similar results than with eFluor staining **(****Figure 7 D)****.**

In all, these experiments demonstrate that the human T lymphocytes transduced with h2CAR-CD1a (OC_1) strongly decreases cell viability of CD1a+ Jurkat and MOLT4 cells, being more potent than mCAR-CD1a. However, no statistical-significant differences between murine and humanized CAR were observed at the observed time (24 h). Despite this, the difference was highly reproducible and indicate a clear trend. The lack of statistical significance can be due to an inappropriate observation time: that is, at 24 h cellular lysis is very intense, and it may have compensated small differences between both CAR types.

In order to reach statistical significance differences between both humanized and murine CARs a different methodology has been employed to evaluate cell death at early times. For this, Jurkat cells labelled with eFluor were co-cocultured with CAR-T cells from 2 to 24 hours and the fraction of Jurkat apoptotic cells were quantified after incubation with Annexin V protein and 7-ADD. The fraction of eFluor+/AnnexinV+/7ADD+ was considered as apoptotic/death cells (Figure 8A) and analysis with three different donors shows: at 2 h statistically significant differences were observed between UT and h2CAR-CD1a at E:T ratios 1:2 and 1:4 but not between UT and mCAR-CD1a (the murine CAR only shows differences with UT at 1:1 E:T ratio) and a tendency to higher cell death induced by h2CD1a-CAR T is observed; at 4 h, higher cell death was observed at E:T ratios 1:1, 1:2 and 1:4 in the presence of **h2CD1a-CAR** T cells compared to mCD1a-CAR T cells **(****Figure 9B);** at 1:8 significant differences were observed between UT and h2CAR-CD1a but not between UT and mCAR-CD1a. At later 6 h, the statistical differences are shifted to lower ratios and specially at 24h differences are only observed at 1:8 ratio.

Data showed in **Figure 8B** at the time of 6 h were re-analyzed as indicated in **Figure 8A** to show alive cells (eFluor+/AnnexinV-/7ADD-) **(Figure 8C)** at 6h. As expected, the fraction of alive cells is reduced at 6 h in the presence of h2CD1a-CAR T-cells more than with mCD1a-CAR T cells at ratios 1:8, 1:4 and 1:2. Altogether, these results clearly show that **h2CD1a-CAR** T cells are more effective, decreasing the time and the doses required to promote cell death in comparison with mCD1a-CAR-T cells.

### ii) Pro-inflammatory cytokines levels

Subsequently, the amount of released proinflammatory cytokines after co-culture of CD1a+ and CD1a- cell lines with mCD1a- and **h2CD1a-CAR** T-cells was determined at 24 h. The production of the proinflammatory cytokines interleukin (IL)-2, tumor necrosis factor α (TNFa), and interferon γ (IFNγ) was measured by using an enzyme-linked immunosorbent assay (ELISA) in supernatants harvested after 24 hours of co-culture. As shown in Figure 9, IL-2, TNFa, and IFNγ cytokines were produced at similarly high levels with both CD1a CARs in Jurkat cells but not a significant increase was detected with NALM6 cells (data were pooled from two independent experiments with 3 different donors). Comparative data including h1 and h3-CD1a-CAR Ts are also shown in **Example 4.**

In summary, these results show that OC_1 induces production of pro-inflammatory cytokines in Jurkat cells at the same level as the mCAR-CD1a.

### Example 8: Potency study in vivo

For the *in vivo* potency study of **OC_1** cells, several experiments were performed in a murine xenograft model of leukaemia, using Luc/GFP-expressing Jurkat T-ALL cells. Thus, like in *in vitro* studies, the *in vivo* potency and activity of the h2CAR-CD1a was also compared to that of mCAR-CD1a.

For the experiment, 6 to 12 week-old NSG mice were intravenously (i.v.) transplanted through the lateral tail vein with 3×10⁶ Luc/GFP-expressing Jurkat cells 3 days before i.v. infusion of either 5×10⁶ mCD1a-CAR-T and h2CD1a-CAR-T or untrasduced (UT) PBMCs, as a control. For the progression of the disease, the quantification of the bioluminescence emitted by the tumor cells was used, since these had previously been modified to express GFP-luciferase. In addition, this measurement allows a much more exact quantification since the disease ends up spreading systemically throughout the body. Thus, the leukaemia establishment or tumour burden was followed up weekly by bioluminescence (BLI). To measure bioluminescence, mice were given 150 mg/kg of D-luciferin intraperitoneally which, when interacting with luciferase, emitted a bioluminescent signal proportional to the number of tumor cells. This signal is measured on a Xenogen IVIS 50 Imaging System (Perkin Elmer). The tumour burden was monitored at day 0 (day of CAR infusion), 3, 6, 10, 14 and 17. Living Image software was used to visualise and calculate total luminescence as average radiance quantification (p/sec/cm²/sr).

For the analysis of tumour circulating cells and persistence of the transferred CARTs, the mice were sacrificed at the end of the experiment (day 17) and circulating PB and hindlimbs were taken. BM cells were isolated by flushing the hindlimbs with PBS containing 2%FBS. For the FACS analysis, 100 µl of PB and 100 µl of BM cell suspension was stained with PBS/2%FBS containing fluorochrome ligated antibodies for the following markers: human leucocyte antigen (HLA)-ABC, CD45, CD3, CD1a, CD38, for 30 min at 4 degrees. 1 ml of BD FACSTM fixing-lysis buffer (BD Biosciences) was added to each sample to eliminate erythrocytes. Samples were run in a FACSCantoTM-II flow cytometer.

To reduce the number of mice used per group without affecting statistical potential, previous work was considered [31]. Accordingly, the number of animals was reduced to a minimum per group (six), enough to prove the hypothesis without losing statistical significance. P values of IVIS quantification experiments were calculated by using the multiple unpaired two-sample t test, assuming a Gaussian distribution and correction using the Holm-Sidak method for multiple comparisons. P values of leukemic and CAR-T cells quantified by FACS after post-mortem were calculated by ANOVA test for multiple comparisons (Tukey's multiple comparisons test). P<0.05 (*) was considered statistically significant.

In NSG animals, tumors develop very rapidly without treatment. **Figure 10** showed the *in vivo* activity of mCAR-CD1a and h2CAR-CD1a. Data showed that both mice groups (mCD1a-CAR-T and h2CD1a-CAR-T) were practically leukaemia free at day 17, in contrast to the mice receiving UT T cells, which showed massive tumour burden by BLI **(****Figure 10 A)****.** Quantification of BLI by IVIS showed an improvement effect of h2CAR-CD1a related to the murine version **(****Figure 10 B)****.** FACS of tumour burden in PB and BM confirmed all CD1a CAR-T cells efficiently eliminated Jurkat cells **(****Figure 10** **C,** data pooled from two independent experiments). The number of mice with less than 0,1% (selected as arbitrary cutoff) of circulating blast cells in PB was lower in those receiving **h2CD1a-CAR** T-cells (10 mice from 12) compared to mCD1a-CAR T-cells (5 mice from 12). The number of mice with more than 1% of CAR-T cells detected in the BM are significantly higher in those receiving h2CD1a-CAR-T (6 mice from 12) compared to mCD1a-CAR T-cells (2 mice from 12). These results demonstrate that h2CD1a-CAR-Ts exhibit potent antileukemic activity that was slightly higher than to that of mCD1a-CAR-Ts **(****Figure 10 B)****.** These data also suggest that humanization of CAR-CD1a improves the capacity of CAR-Ts to reduce tumor burden. Comparative data with h1CD1a-CART is also shown in **Example 4.**

The Jurkat in vivo model develops leukemia engraftment in one week and should be stopped at day 17 to avoid animal suffering. We have also use a PDX mouse model that develops the disease slowly. For this, animals were randomly distributed in groups and injected with 0,5×10⁶ of blast cells. After 3 days, mice were distributed in three groups receiving 0,5×10⁶ or 1×10⁶ of CART or UT (untransduced control) cells. After 6 weeks mice were sacrificed and circulating PB and hindlimbs were isolated as described previously in **Figure 10** and analyzed with the following markers: human leucocyte antigen (HLA)-ABC, CD45, CD3, CD1a and CCR9 that was selectively and frequently expressed on T-ALL [32]. FACS analysis was performed as described in **Figure 11** **A.** The % of blasts were determined as HLA-ABC+ CD45+ CD34+ CD3- and confirmed to be CD1a+ (and CCR9+) and differentiated from CAR-T cells (HLA-ABC+ CD45+ CD3+). The results show a significant reduction of tumor burden in PB and BM in mice receiving 1 million of humanized or murine CAR-Ts related to UT. Interestingly, mice receiving a limiting amount of h2CD1a-CAR-Ts (0,5 million) had a decreased tumor engrafment in PB related to UT significantly better than mCD1a-CAR-Ts. Stronger and significant inhibition of tumor engrafment in PB and BM is observed with h2CD1a-CAR-T in comparison with mCD1a-CAR-Ts at the 0,5 M doses. At the highest dose of 1 million of CAR-Ts differences between humanized and murine CAR-Ts are minimized but still reduction is higher with h2CD1a-CAR-Ts compared with mCD1a-CAR-Ts **(****Figure 11 B)****.** All these results suggest that humanization of CAR-CD1a improves the capacity of CAR-Ts to reduce tumor burden and reduces the amount required to have a significant biological effect.

### Example 9: Specificity studies: Off-target analysis

In order to demonstrate **h2CD1a-CAR** T-cells are not binding nor affecting unspecifically the viability of CD1a negative cells, a series of off-target analysis were conducted in a panel of 10 cell lines from different embryonic origins. As shown in **Figure 12****,** cell lines derived from the kidney (HEK293T), colon (HCT-116), microvascular endothelium (HMEC), lung (L55), breast (MCF7, MDA-MB-231), pancreas (PANC-1, RWP-1), skin (SKMEL), ovarian (SKOV3) and brain (PG88) were all CD1a negative after checking its expression by using two different anti-CD1a antibodies: a commercial antibody (Becton Dickinson) **(****Figure 12 A)** and NA1/34-HLK, the same used to obtain the murine anti-CD1a scFv of the mCAR-CD1a **(****Figure 12 B)****.** As a positive control, a HEK293T cell line was generated ectopically expressing CD1a recognized by the two antibodies (see **Figures 12** **A and 12 B).**

Cell viability of adherent cells lines was evaluated in the presence of UT and mCAR-CD1a and h2CAR-CD1a T-cells and quantified by two different methods: an endpoint colorimetric method checking cell viability by using the MTT reagent at 72 hours of total exposition **(****Figure 13** and **Figure 14****),** and a real time quantification of proliferation by using the XCELLigence system **(****Figure 15****).** As shown in **Figure 13****,** only those HEK293T cells expressing CD1a (used as a positive control of cell death) are dying in the presence of CD1a-CARTs murine or humanized (as also demonstrated with naturally expressing CD1a, Jurkat and MOLT4 cell lines in **Figure 7****).** By contrast, cell lines negative for CD1a were not affected by CD1a-CARTs **(****Figures 13** and **14****).** Similar results were obtained by the XCELLigence system **(****Figure 15****).** Both methodologies clearly showed that adherent CD1a- cell lines were unaffected by co-culturing with mCAR-CD1a or h2CAR-CD1a T-cells during 3 days. These results clearly show that CD1a-CAR T cells are unable to affect cell viability of CD1a negative cells lines from different origins. In addition, these results indicate that humanization is not affecting the CAR-CD1a specificity.

### Example 10: Experiments to ensure no fratricide effect of h2CD1a-CAR T-cells

It was previously described that autologous mature T-cells recovered from the PB of patients with T-ALL, modified to express the mCAR-CD1a, retained cytolytic activity against the tumoral cells expressing the target antigen (CD1a+ T-ALL blasts), without effecting non-tumoral cells (CD1aT cells), suggesting that mCD1a-CARTs are fratricide resistant [16]. In order to ensure that h2CD1a-CAR-Ts are also fratricide resistant, CD3+CD1a- T-cells were isolated from a patient by Magnetic-activated cell sorting (MACS), activated with CD3/CD28, and lentivirally transduced with a 19.7% of transduction with h2CAR-CD1a and showing a similar proportion of CD4+/CD8+ respect to UT cells (see **Figure 16** **A,** for a general protocol scheme, and **Figure 16** **B** for the transduction efficiency). Subsequently, the cytolytic capacity of h2CD1a-CAR-Ts against total PBMCs as targets was investigated in order to assess both the autologous cytotoxicity potential and the degree of fratricide. Within eFluor 670-labeled target PBMCs, the great majority (>60%) are CD1a+ blasts, and less than 20% are CD3+CD1a- mature T-cells. Compared with UT cells, the h2CD1a-CAR-Ts exhibited massive and specific cytolytic capacity against autologous CD1a+ blasts **(****Figure 16** **C,** lower quantification panel) but not against CD1a- mature T-cells **(****Figure 16** **C,** upper quantification panel), further showing that h2CD1a-CAR-Ts are fratricide resistant. Similar results were obtained upon absolute cell number quantification by using TrueCount (BD Biosciences) **(****Figure 16** **D;** left panel absolute number of CD1a negative T-cells are not affected after incubation with h2CAR-CD1a-T or UT cells; right panel, CD1a positive cells are eliminated by h2CAR-CD1a T-cells but not by UT cells).

### Example 11: Experiments to compare affinity of h2CAR-CD1a and mCAR-CD1a to CD1a

The improved cytotoxic capacity o OC_1 (humanized 2 version) may be explained by a better recognition of the target. To check this, we have produced the human recombinant protein extracellular domain of CD1a (rCD1a, 34,9 KDa) as a dimer with its natural partner at the cell membrane b2-microglobulin (hB2M, 13,7 KDa) **(****Figure 17 A)****.** rCD1a was produced with a 6xHis tag that allow its purification and detection by using anti-HIS antibodies by FACS **(****Figure 17 B)****.** H2CD1a-CAR-T and mCD1a-CAR T-cells were generated from three different donors with a similar membrane CAR expression determined by anti-scFv staining as shown in **Figure 5** **B** and when membrane CAR expression was different its expression was compensated with UT cells. 1×10⁵ CAR T-cells were diluted in PBS-2% FBS in 100 µL containing AB-serum to block for non-specific binding. CAR T-cells were incubated with rCD1a at 200 nM during 15 minutes at 4°C, washed with PBS-2% FBS and re-incubated with anti-HIS-APC antibodies. A representative FACS analysis is shown in Figure 17B showing a high and specific binding of rCD1a to h2CAR-CD1a T-cells higher than 70% (Figure 17B). By contrast, mCAR-CD1a T-cells binding was around 20% as shown from the quantification from three different PBMCs donor samples **(****Figure 17 C)****.** Binding quantification based on mean fluorescent intensity (MFI, calculated as geometric mean using FACSDiva software) which is proportional to the number of antibodies that recognize and bind to rCD1a was also performed with similar results **(Figure 17 D).**

H2CD1a-CAR-T and mCD1a-CAR T-cells were generated by infecting with a construct co-expressing T2A-GFP **(****Figure 18 A)****.** This method allows to monitor infection efficiency with both constructs at the same level with great accuracy **(****Figure 18 B)****.** Furthermore, it allows to specifically quantify rCD1a binding in the population of GFP positive cells **(****Figure 18 C)****.** Incubation of h2CD1a-CAR-T(GFP) and mCD1a-CAR-T(GFP) cells with rCD1a-HIS at 200 nM during 15 minutes at 4°C results in a higher detection of h2CD1a-CAR-T positive cells **(****Figure 18** C) at the same level of GFP detection. By contrast, mCD1a-CAR-T cells showed a poor binding capacity with decreased labelling **(****Figure 18 C)****.** Quantification of median fluorescence intensity (MFI) from three different donors resulted in a strong and dose-dependent binding of rCD1a to CAR-Ts infected with h2CAR-CD1a compared to mCAR-CD1a **(****Figure 18 D)****.** Binding of rCD1a to h2CD1a CAR-T cells may be adjusted to one-site equation curve by non-linear regression after subtraction of nonspecific binding (Bmax=2304, Kd=3,41). A similar analysis has been performed with mCD1a CAR T-cells showing a much lower binding capacity (Bmax=91,63, Kd=1,26). Robust data from three different PBMC donors, clearly show an improved binding of rCD1a to h2CD1a-CAR T-cells measured as % of rCD1a positive/GFP positive cells **(****Figure 18 E)****.**

The results showing binding of rCD1a to h2CD1a-CAR-T cells are completely unexpected. In fact, the detection and quantification of transduced cells with the humanized CAR has been greatly improved and will be the method used in a clinical trial with OC_1. In comparative analysis with h2CD1a-CAR T-cells co-expressing GFP, quantification with rCD1a gives the same results than GFP (65% in the example shown in **Figure 19** **A and 19 B)** respect the detection with scFv that was 15% lower. In fact, the method described here with the recombinant protein improves the detection of the h2CAR-CD1a (without GFP) used in the clinical trial (see **Figure 19** **A and 19 B,** 58% with scFv and 71 % with rCD1a). For all these reasons, we can conclude that the unexpected binding of h2CD1a-CAR T-cells to rCD1a not only is a strong difference with the murine original version, is in fact a new quantification method with clinical use.

### Bibliography

1. Karrman, K. and B. Johansson, Pediatric T-cell acute lymphoblastic leukemia. Genes Chromosomes Cancer, 2017. 56(2): p. 89-116.
2. Weng, A.P., et al., Activating mutations of NOTCH1 in human T cell acute lymphoblastic leukemia. Science, 2004. 306(5694): p. 269-71.
3. Hunger, S.P. and C.G. Mullighan, Acute Lymphoblastic Leukemia in Children. N Engl J Med, 2015. 373(16): p. 1541-52.
4. Litzow, M.R. and A.A. Ferrando, How I treat T-cell acute lymphoblastic leukemia in adults. Blood, 2015. 126(7): p. 833-41.
5. Karrman, K., et al., Clinical and cytogenetic features of a population-based consecutive series of 285 pediatric T-cell acute lymphoblastic leukemias: rare T-cell receptor gene rearrangements are associated with poor outcome. Genes Chromosomes Cancer, 2009. 48(9): p. 795-805.
6. Sutton, R., et al., Persistent MRD before and after allogeneic BMT predicts relapse in children with acute lymphoblastic leukaemia. Br J Haematol, 2015. 168(3): p. 395-404.
7. Qasim, W. and A.J. Thrasher, Progress and prospects for engineered T cell therapies. Br J Haematol, 2014. 166(6): p. 818-29.
8. Humphries, C., Adoptive cell therapy: Honing that killer instinct. Nature, 2013. 504(7480): p. S13-5.
9. Gardner, R.A., et al., Intent-to-treat leukemia remission by CD19 CAR T cells of defined formulation and dose in children and young adults. Blood, 2017. 129(25): p. 3322-3331.
10. Fry, T.J., et al., CD22-targeted CAR T cells induce remission in B-ALL that is naive or resistant to CD19-targeted CAR immunotherapy. Nat Med, 2018. 24(1): p. 20-28.
11. Cooper, M.L., et al., An "off-the-shelf' fratricide-resistant CAR-T for the treatment of T cell hematologic malignancies. Leukemia, 2018. 32(9): p. 1970-1983.
12. Gomes-Silva, D., et al., CD7-edited Tcells expressing a CD7-specific CAR forthe therapy of T-cellmalignancies. Blood, 2017. 130(3): p. 285-296.
13. Png, Y.T., et al., Blockade of CD7 expression in T cells for effective chimeric antigen receptor targeting of T-cell malignancies. Blood Adv, 2017. 1(25): p. 2348-2360.
14. Mamonkin, M., et al., A T-cell-directed chimeric antigen receptor for the selective treatment of T-cellmalignancies. Blood, 2015. 126(8): p. 983-92.
15. Rasaiyaah, J., et al., TCRalphabeta/CD3 disruption enables CD3-specific antileukemic T cell immunotherapy. JCI Insight, 2018. 3(13**).**
16. Sanchez-Martinez, D., et al., Fratricide-resistant CD1a-specific CAR T cells for the treatment of cortical T-cell acute lymphoblastic leukemia. Blood, 2019. 133(21): p. 2291-2304.
17. Maus, M.V., et al., Antibody-modified T cells: CARs take the front seat for hematologic malignancies. Blood, 2014. 123(17): p. 2625-35.
18. Kershaw, M.H., J.A. Westwood, and P.K. Darcy, Gene-engineered T cells for cancer therapy. Nat Rev Cancer, 2013. 13(8): p. 525-41.
19. Andreatta, M. and M. Nielsen, Gapped sequence alignment using artificial neural networks: application to the MHC class I system. Bioinformatics, 2016. 32(4): p. 511-7.
20. Nielsen, M., et al., Reliable prediction of T-cellepitopes using neural networks with novel sequence representations. Protein Sci, 2003. 12(5): p. 1007-17.
21. Kringelum, J.V., et al., Reliable B cell epitope predictions: impacts of method development and improved benchmarking. PLoS Comput Biol, 2012. 8(12): p. e1002829.
22. Karosiene, E., et al., NetMHCcons: a consensus method for the major histocompatibility complex class Ipredictions. Immunogenetics, 2012. 64(3): p. 177-86.
23. Rasmussen, M., et al., Pan-Specific Prediction of Peptide-MHC Class I Complex Stability, a Correlate of T Cell Immunogenicity. J Immunol, 2016. 197(4): p. 1517-24.
24. Reynisson, B., et al., Improved Prediction of MHC II Antigen Presentation through Integration and Motif Deconvolution of Mass Spectrometry MHC Eluted Ligand Data. J Proteome Res, 2020. 19(6): p. 2304-2315.
25. Kalaitsidou, M., et al., CAR T-cell therapy: toxicity and the relevance of preclinical models. Immunotherapy, 2015. 7(5): p. 487-97.
26. Norelli, M., et al., Clinical pharmacology of CAR-T cells: Linking cellular pharmacodynamics to pharmacokinetics and antitumor effects. Biochim Biophys Acta, 2016. 1865(1): p. 90-100.
27. Berger, C., et al., Safety of targeting ROR1 in primates with chimeric antigen receptormodified T cells. Cancer Immunol Res, 2015. 3(2): p. 206-16.
28. Wang, J., Y. Hu, and H. Huang, Acute lymphoblastic leukemia relapse after CD19-targeted chimeric antigen receptor T cell therapy. J Leukoc Biol, 2017. 102(6): p. 1347-1356.
29. Campana, D., H. Schwarz, and C. Imai, 4-1BB chimeric antigen receptors. Cancer J, 2014. 20(2): p. 134-40.
30. Wagner, D.L., et al., Immunogenicity of CAR T cells in cancer therapy. Nat Rev Clin Oncol, 2021. 18(6): p. 379-393.
31. Sanjuan-Pla, A., et al., Platelet-biased stem cells reside at the apex of the haematopoietic stem-cell hierarchy. Nature, 2013. 502(7470): p. 232-6.
32. Qiuping, Z., et al., Selectively increased expression and functions of chemokine receptor CCR9 on CD4+ T cells from patients with T-cell lineage acute lymphocytic leukemia. Cancer Res, 2003. 63(19): p. 6469-77.

## Claims

1. A humanized CD1a targeting moiety, wherein the CD1a targeting moiety is an antibody, F(ab')2, Fab, scFab or scFv, comprising a VL domain consisting of SEQ ID NO: 1 and a VH domain consisting of SEQ ID NO: 2.

2. The humanized CD1a targeting moiety according to claim 1, wherein the CD1a targeting moiety is a scFv comprising a VL domain consisting of SEQ ID NO: 1 and a VH domain consisting of SEQ ID NO: 2.

3. A chimeric antigen receptor (CAR) comprising:
a) an extracellular domain comprising a CD1a targeting moiety, wherein the CD1a targeting moiety is a scFv comprising a VL domain consisting of SEQ ID NO: 1 and a VH domain consisting of SEQ ID NO: 2;
b) a transmembrane domain; and
c) an intracellular signaling domain.

4. The CAR according to claim 3, wherein the transmembrane domain comprises the transmembrane domain of CD28, CD3, CD45, CD4, CD8, CD9, CD16, CD22, CD33, CD37, CD64, CD80, CD86, CD134, CD137, or CD154.

5. The CAR according to claim 4, wherein the transmembrane domain comprises the transmembrane domain of CD8.

6. The CAR according to any one of claims 1-5, wherein the intracellular signaling domain comprises the intracellular domain of CD3ζ, FcRy, CD3γ, CD3δ, CD3ε, CD5, CD22, CD79a, CD79b or CD66b.

7. The CAR according to any one of claims 1-6, wherein the CAR further comprises a costimulatory signaling domain, preferably the costimulatory signaling domain comprises the intracellular domain of CD27, CD28, CD137, CD134, CD30, CD40, lymphocyte function-associated antigen-1 (LFA-1), CD2, CD7, LIGHT, NKG2C, or CD276.

8. The CAR according to any one of claims 1-7, consisting of SEQ ID NO: 13.

9. A nucleic acid encoding the CAR according to any one of claims 3-8.

10. A cell comprising the nucleic acid according to claim 9 and/or the CAR according to any one of claims 3-8.

11. The cell according to claim 10, wherein the cell is a T-cell.

12. A pharmaceutical composition comprising a plurality of cells according to claim 11 and a pharmaceutically acceptable carrier or diluent.

13. The cell according to claim 11 or the pharmaceutical composition according to claim 12 for use as a medicament.

14. The cell according to claim 11 or the pharmaceutical composition according to claim 12 for use in a method of treating a CD1a-positive cancer, wherein the method comprises administering the cell or composition to a patient in need thereof.

15. The cell or pharmaceutical composition for use according to claim 14, wherein the CD1a-positive cancer is cortical T-cell acute lymphoblastic leukemia, preferably, relapsed/refractory cortical T-cell acute lymphoblastic leukemia.
